# EUROPEAN PATENT APPLICATION

(11) **EP 1 452 525 A1**
(43) Date of publication of application: **01.09.2004**
(21) Application number: 02779936.0
(22) Date of filing: 29.10.2002
(51) Int. Cl.: C07D 209/18, C07D 209/42, C07D 401/04, C07D 401/06, C07D 401/12, C07D 401/14, C07D 403/12, C07D 403/14, C07D 405/04, C07D 405/12, C07D 405/14, C07D 409/14, C07D 413/14, C07D 417/14, C07D 471/04, A61K 31/404, A61K 31/437, A61K 31/454, A61K 31/4725, A61K 31/496, A61K 31/497

(54) **AMIDE DERIVATIVES AND DRUGS**

(30) Priority: 30.10.2001 JP 2001332942; 30.04.2002 JP 2002127771
(71) Applicant: Nippon Shinyaku Co., Ltd., Kyoto-shi Kyoto 601-8550 (JP)
(72) Inventor: MARUYAMA, Yasufumi, Tsukuba-shi, Ibaraki 305-0051 (JP); HIRABAYASHI, Kazuko, Ukyo-ku, Kyoto-shi, Kyoto 616-8105 (JP); HORI, Katsutoshi, Tsuchiura-shi, Ibaraki 300-0068 (JP)
(74) Representative: VOSSIUS & PARTNER
(86) International application number: PCT/JP2002/011232
(87) International publication number: WO 2003/037862

(57) **Abstract**

The present invention provides an amide derivative represented by the following formula [1]: 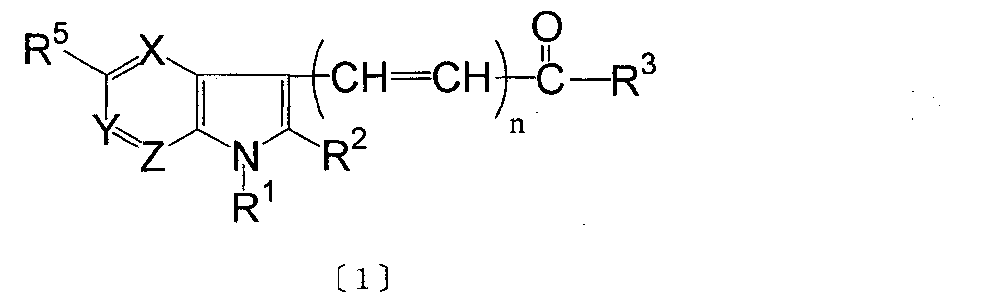 wherein n represents 0 or 1; X represents CR⁴ or N; Y represents CR⁶ or N; Z represents CR⁷ or N; R¹ and R² may be the same or different and each represents hydrogen, optionally substituted alkyl, acyl, optionally substituted aryl, or an optionally substituted aromatic heterocyclic group; R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano, or nitro; and R³ represents optionally substituted alkylamino, optionally substituted arylamino, or optionally substituted cyclic amino, or a pharmaceutically acceptable salt thereof, and a pharmaceutical composition comprising them as an active ingredient.

The compound of the present invention is useful as a TGF-β inhibitor.

## Description

### TECHNICAL FIELD

The present invention relates to a TGF-β (transforming growth factor-β) inhibitor comprising an amide derivative or a pharmaceutically acceptable salt thereof as an active ingredient. The present invention also relates to a novel amide derivative or a pharmaceutically acceptable salt thereof. The TGF-β inhibitor is useful for the prevention or treatment of diseases whose main characteristics are fibroproliferative disorders, cicatricial lesions or sclerosing lesions involving TGF-β.

### BACKGROUND ART

TGF-β is an important cytokine which regulates the proliferation and differentiation of cells, and the repair and regeneration of damaged tissue. TGF-β facilitates the infiltration of leukocytes and vascularization, and promotes the production and deposition of elements of the extracellular matrix (such as laminin B1, fibronectin, collagen, tenascin and proteoglycan) in case of repairing damages tissues.

The production and deposition of the extracellular matrix by TGF-β involves the following three mechanisms. First, TGF-β promotes the expression of genes for extracellular matrix proteins, and increases their synthesis and secretion. Second, TGF-β inhibits the synthesis of proteolytic enzymes which degrade synthesized extracellular matrix proteins, and at the same time enhances the synthesis of inhibitors of proteolytic enzymes. Third, TGF-β increases the levels of integrin, a receptor for extracellular matrix proteins, and enhances the deposition of extracellular matrix.

Thus, TGF-β plays an extremely important role in living organisms. It regulates the proliferation and differentiation of various cells, particularly fibroblasts, which are the main constituent cells of fibrous tissues, and regulates the production and deposition of the extracellular matrix, which is essential for wound healing. However, excessive production of TGF-β or facilitation of its activity promotes diseases characterized by fibroproliferative disorders, cicatricial lesions or sclerosing lesions of tissues, organs or even the whole body. There is also reported an action of serving as a growth factor to cancer cells or promoting the formation of a newborn blood vessel thereby to promote the growth of cancer when cancer cells grow in vivo (Human Cell, 13, 97-101, 2000, Adv. Immunol., 75, 115-157, 2000).

Therefore, the TGF-β inhibitor can be therapeutic drugs for the above diseases involving TGF-β (Border, W. A. et al., J. Clin. Invest., (90), 1-7, 1992).

International Publication WO00/44743 describes that indole-3-carboxamide derivatives have an inhivitory action against TGF-β, while International Publication WO01/43746 describes that indole-3-carboxamide derivatives have an action for the treatment of nephritis.

### DISCLOSURE OF THE INVENTION

The object of the present invention is to provide a novel amide derivative which has higher absorptivity than that of an amide derivative of the prior art upon oral administration, and also has a strong inhibitory action against TGF-β.

As a result of having conducted intensive studies on various compounds, the present inventors have found that an amide derivative of the present invention can achieve the above object, and the present invention has been completed based on this finding.

Thus, the present invention is directed to an amide derivative, which is a compound represented by the following formula [1] in any of the following cases (A), (B) and (C).

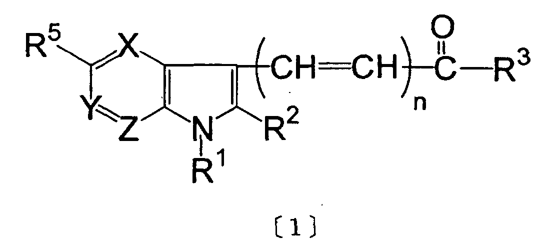

(A)
n represents 0,
X represents CR⁴ or N, Y represents CR⁶ or N, Z represents CR⁷ or N,
R¹ and R² may be the same or different and each represents hydrogen, alkyl, haloalkyl, hydroxyalkyl, alkoxyalkyl, carboxyalkyl, acyl, aryl, an aromatic heterocyclic group or arylalkyl ( the aryl, the aromatic heterocyclic group and the aryl moiety of the arylalkyl may be substituted by R⁸¹, R⁸² or R⁸³, and R⁸¹, R⁸² and R⁸³ may be the same or different and each represents halogen, alkyl, haloalkyl, alkoxy, haloalkoxy, hydroxy, hydroxyalkyl, carboxy, alkoxycarbonyl, cyano or nitro, or the two adjacent groups among R⁸¹, R⁸² and R⁸³ may jointly form methylenedioxy or ethylenedioxy ),
R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano or nitro, or the two adjacent groups among R⁴, R⁵, R⁶ and R⁷ may jointly form methylenedioxy or ethylenedioxy,
R³ represents a group represented by the following formula [2]: 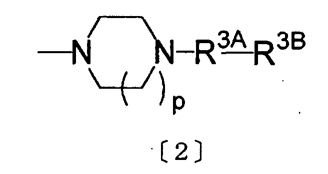
in which p represents 1 or 2,
R^{3A} represents a divalent group of pyridine, pyrazine, pyridazine or pyrimidine,
R^{3B} represents tetrazolyl [ the tetrazolyl may be substituted by alkyl ( the alkyl may be substituted by hydroxy, dialkylamino or cycloalkyl )] or a group represented by the following formula [3], 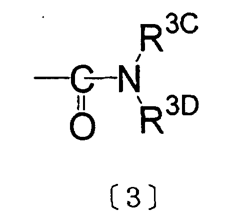
R^{3C} and R^{3D} may be the same or different and each represents any of the following groups (1) to (4):

(1) hydrogen,
(2) cycloalkyl ( the cycloalkyl may be substituted by alkyl, hydroxy, hydroxyalkyl, carboxy, alkoxycarbonyl or aryl ),
(3) alkyl [ the alkyl may be substituted by 1 to 5 same or different members selected from the group consisting of (i) a saturated heterocyclic group ( the saturated heterocyclic group may be substituted by alkyl, hydroxyalkyl or alkoxyalkyl), (ii) an aromatic heterocyclic group ( the aromatic heterocyclic group may be substituted by 1 to 3 same or different members selected from the group consisting of alkyl, hydroxy, amino, hydroxyalkyl and alkoxyalkyl ), (iii) hydroxy, (iv) alkoxy, (v) aryl ( the aryl may be substituted by alkyl, hydroxy, alkoxy or alkoxyalkyl ), (vi) aryloxy ( the aryl moiety of the aryloxy may be substituted by alkyl, hydroxy, alkoxy or alkoxyalkyl ), (vii) alkylamino, (viii) dialkylamino, (ix) carboxy, (x) alkoxycarbonyl and (xi) halogen ], and
(4) an aromatic heterocyclic group ( the aromatic heterocyclic group may be substituted by alkyl, hydroxyalkyl, alkoxyalkyl, alkoxycarbonylalkyl or aryl ), or
   R^{3C}, R^{3D} and adjacent N jointly form cyclic amino ( the cyclic amino may be substituted by alkyl, hydroxyalkyl, alkoxyalkyl, aminoalkyl, hydroxy, aryl or arylalkyl ),
   with the exception of the case that R^{3C} is hydrogen and R^{3D} is hydrogen or non-substituted alkyl;

(B)
n represents 0,
X represents CR⁴ or N, Y represents CR⁶ or N, Z represents CR⁷ or N,
R¹ and R² may be the same or different and each represents alkyl, haloalkyl, hydroxyalkyl, alkoxyalkyl, carboxyalkyl, acyl, aryl, an aromatic heterocyclic group or arylalkyl ( the aryl, the aromatic heterocyclic group and the aryl moiety of the arylalkyl may be substituted by R⁸¹, R⁸² or R⁸³, and R⁸¹, R⁸² and R⁸³ may be the same or different and each represents halogen, alkyl, haloalkyl, alkoxy, haloalkoxy, hydroxy, hydroxyalkyl, carboxy, alkoxycarbonyl, cyano or nitro, or the two adjacent groups among R⁸¹, R⁸² and R⁸³ may jointly form methylenedioxy or ethylenedioxy),
R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano or nitro, or the two adjacent groups among R⁴, R⁵, R⁶ and R⁷ may jointly form methylenedioxy or ethylenedioxy,
R³ represents a group represented by the following formula [4] :

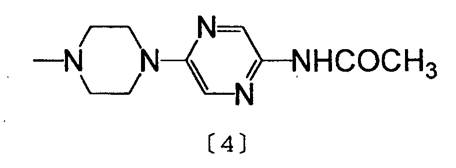

(C)
n represents 1,
X represents CR⁴ or N, Y represents CR⁶ or N, Z represents CR⁷ or N,
R¹ and R² may be the same or different and each represents hydrogen, alkyl, haloalkyl, hydroxyalkyl, alkoxyalkyl, carboxyalkyl, acyl, aryl, an aromatic heterocyclic group or arylalkyl ( the aryl, the aromatic heterocyclic group and the aryl moiety of the arylalkyl may be substituted with R⁸¹, R⁸² or R⁸³, and R⁸¹, R⁸² and R⁸³ may be the same or different and each represents halogen, alkyl, haloalkyl, alkoxy, haloalkoxy, hydroxy, hydroxyalkyl, cyano, alkoxycarbonyl, carboxy or nitro, or the two adjacent groups among R⁸¹, R⁸² and R⁸³ may jointly form methylenedioxy or ethylenedioxy ),
R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano or nitro, or the two adjacent groups among R⁴, R⁵, R⁶ and R⁷ may jointly form methylenedioxy or ethylenedioxy,
R³ represents the following group (1) or (2):

(1) cyclic amino [ the cyclic amino may be substituted by R⁹¹, R⁹² or R⁹³ on the same or different carbon atom and the benzene ring may be condensed, R⁹¹, R⁹² and R⁹³ may be the same or different and each represents alkyl, alkoxy, hydroxy, hydroxyalkyl, alkoxyalkyl, alkoxycarbonylalkyl, carboxy, carboxyalkyl, acyl, 2-hydroxybenzimidazole, aryl ( the aryl may be substituted by alkyl or alkoxy ) or an aromatic heterocyclic group ( the aromatic heterocyclic group may be substituted by alkyl, hydroxy, oxo or alkoxy), or the two groups on the same carbon jointly represent -CONHCH₂N(Ph)- ( Ph represents phenyl ), the benzene ring condensed with the cyclic amino may be substituted with R⁹⁴ or R⁹⁵, and R⁹⁴ and R⁹⁵ may be the same or different and each represents alkyl, alkoxy, halogen, hydroxy, amino, monoalkylamino, dialkylamino, alkylsulfonylamino, acylamino, carbamoyl, monoalkylcarbamoyl, dialkylcarbamoyl, cyano, carboxy, alkoxycarbonyl, sulfamoyl, monoalkylaminosulfonyl, dialkylaminosulfonyl or nitro, or the two adjacent groups among R⁹⁴ and R⁹⁵ may jointly form methylenedioxy or ethylenedioxy ],
(2) a group represented by the following formula [5]: 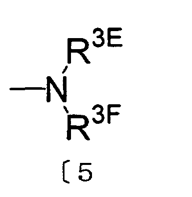 R^{3E} and R^{3F} may be the same or different and each represents any of the following groups (i) to (v):
   (i) hydrogen,
   (ii) alkyl { the alkyl may be substituted by 1 to 3 same or different members selected from the group consisting of (a) hydroxy, (b) aryl ( the aryl may be substituted with 1 to 3 same or different members selected from the group consisting of alkyl, haloalkyl, hydroxy, alkoxy, halogen, cyano and nitro ), (c) carboxy, (d) a saturated heterocyclic group [ the saturated heterocyclic group may be substituted by alkyl or arylalkyl ( the aryl moiety of the arylalkyl may be substituted with alkyl, alkoxy or halogen )], and (e) an aromatic heterocyclic group ( the aromatic heterocyclic group may be substituted by alkyl, haloalkyl, hydroxy, hydroxyalkyl, alkoxy, halogen, cyano or nitro )},
   (iii) aryl ( the aryl may be substituted by alkyl, haloalkyl, hydroxy, hydroxyalkyl, alkoxy, halogen, cyano or nitro ),
   (iv) cycloalkyl ( the cycloalkyl may be substituted by alkoxy, alkoxycarbonyl or hydroxy and the benzene ring may be condensed), and
   (v) a saturated heterocyclic group ( the saturated heterocyclic group may be substituted by alkyl, haloalkyl, hydroxy, hydroxyalkyl, alkoxy, halogen, cyano or nitro ).

The present invention is also directed to a TGF-β inhibitor comprising the amide derivative, which is a compound represented by the above formula [1] in any of the following cases (A), (B) and (C), or a pharmaceutically acceptable salt thereof as an active ingredient.

It has never been known that the compound represented by the above formula [1] has an inhivitory action against TGF-β.

The amide derivative, which is a compound represented by the above formula [1] in either the case (A) or (C), is a novel compound which is not disclosed in any documents.

The amide derivative, which is a compound represented by the above formula [1] in the case (B), is not specifically disclosed in aforementioned International Publication WO00/44743 and International Publication WO01/43746.

Examples of particularly preferred compounds among the compound [1] of the present invention include the following amide derivatives (1) to (36):
(1) 5-{4-[(1,5-dimethyl-2-phenyl-1H-indol-3-yl)carbonyl]piperazin-1-yl}-N-[2-(4-methyl-1,3-thiazol-5-yl)ethyl]pyrazine-2-carboxamide,
(2) 2-[(2E)-3-(1-methyl-2-phenyl-1H-pyrrolo[2,3-b]pyridin-3-yl)prop-2-enoyl]-6,7-dimethoxy-1,2,3,4-tetrahydroisoquinoline,
(3) (2E)-N-methyl-N-(1-methyl-piperidin-4-yl)-3-(1-methyl-2-phenyl-1H-pyrrolo[2,3-b]pyridin-3-yl)acrylamide,
(4) 2-[(2E)-3-(1,5-dimethyl-2-phenyl-1H-pyrrolo[2,3-b]pyridin-3-yl)prop-2-enoyl]-6,7-dimethoxy-1,2,3,4-tetrahydroisoquinoline,
(5) {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetic acid,
(6) ethyl N-benzyl-N-[(5-{4-[(1,5-dimethyl-2-phenyl-1H-indol-3-yl)carbonyl]piperazin-1-yl}pyrazin-2-yl)carbonyl]aminoacetate,
(7) ethyl 2-{N-[(2E)-3-(1-methyl-2-phenyl-1H-pyrrolo[2,3-b]pyridin-3-yl)prop-2-enoyl]amino}-1-cyclopentanecarboxylate,
(8) ethyl 2-{N-[(5-{4-[(1,5-dimethyl-2-phenyl-1H-indol-3-yl)carbonyl]piperazin-1-yl}pyrazin-2-yl)carbonyl]amino}-1-cyclohexanecarboxylate,
(9) 2-[(2E)-3-(5-methyl-6-phenyl-5H-pyrrolo[2,3-b]pyrazin-7-yl)prop-2-enoyl]-6,7-dimethoxy-1,2,3,4-tetrahydroisoquinoline,
(10) ethyl 2-{N-[(5-{4-[(1,5-dimethyl-2-phenyl-1H-indol-3-yl)carbonyl]piperazin-1-yl}pyrazin-2-yl)carbonyl]amino}-1-cyclopentanecarboxylate,
(11) 2-[(2E)-3-(1-methyl-2-phenyl-1H-pyrrolo[2,3-c]pyridin-3-yl)prop-2-enoyl]-6,7-dimethoxy-1,2,3,4-tetrahydroisoquinoline,
(12) 2-{(2E)-3-[1-methyl-2-(3,4-dimethoxyphenyl)-1H-pyrrolo[2,3-b]pyridin-3-yl]prop-2-enoyl}-6,7-dimethoxy-1,2,3,4-tetrahydroisoquinoline,
(13) N-methyl-N-[(5-{4-[(1,5-dimethyl-2-phenyl-1H-indol-3-yl)carbonyl]piperazin-1-yl}pyrazin-2-yl)carbonyl]-(S)-phenylalanine,
(14) methyl {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetate,
(15) {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetic acid,
(16) 2-{(2E)-3-[1-methyl-2-(4-pyridyl)-1H-pyrrolo[2,3-b]pyridin-3-yl]prop-2-enoyl}-6,7-dimethoxy-1,2,3,4-tetrahydroisoquinoline,
(17) 2-[(2E)-3-(1-methyl-2-phenyl-1H-pyrrolo[3,2-b]pyridin-3-yl)prop-2-enoyl]-6,7-dimethoxy-1,2,3,4-tetrahydroisoquinoline,
(18) N-[(2E)-3-(1-methyl-2-phenyl-1H-pyrrolo[2,3-b]pyridin-3-yl)prop-2-enoyl]-N-[2-(2-thienyl)ethyl]aminoacetic acid,
(19) 6,7-dimethoxy-2-[(2E)-3-(2-phenyl-1H-pyrrolo[2,3-b]pyridin-3-yl)prop-2-enoyl]-1,2,3,4-tetrahydroisoquinoline,
(20) 2-[(2E)-3-(1-methoxymethyl-2-phenyl-1H-pyrrolo[2,3-b]pyridin-3-yl)prop-2-enoyl]-6,7-dimethoxy-1,2,3,4-tetrahydroisoquinoline,
(21) 2-{(2E)-3-[1-methyl-2-(4-fluorophenyl)-1H-pyrrolo[2,3-b]pyridin-3-yl]prop-2-enoyl}-6,7-dimethoxy-1,2,3,4-tetrahydroisoquinoline,
(22) 4-acetyl-1-{(2E)-3-[1,5-dimethyl-2-(4-carbomethoxyphenyl)-1H-indol-3-yl]prop-2-enoyl}-4-phenylpiperidine,
(23) 2-{(2E)-3-[1,5-dimethyl-2-(4-carbomethoxyphenyl)-1H-indol-3-yl]prop-2-enoyl}-6,7-dimethoxy-1,2,3,4-tetrahydroisoquinoline,
(24) (2E)-N-methyl-N-(1-methylpiperidin-4-yl)-3-[1,5-dimethyl-2-(4-carbomethoxyphenyl)-1H-indol-3-yl]acrylamide,
(25)5-{4-[(5-bromo-1-methyl-2-phenyl-1H-indol-3-yl)carbonyl]piperazin-1-yl}-N-[2-(4-methyl-1,3-thiazol-5-yl)ethyl]pyrazine-2-carboxamide,
(26) 5-{4-[(1,5-dimethyl-2-phenyl-1H-indol-3-yl)carbonyl]piperazin-1-yl}-N-[2-(4-hydroxyphenyl)ethyl]pyrazine-2-carboxamide,
(27) 4-{3-[3-(4-acetyl-4-phenylpiperidin-1-yl)-3-oxo-1-propenyl]-1,5-dimethyl-1H-indol-2-yl}benzoic acid,
(28) 5-{4-[(5-fluoro-1-methyl-2-phenyl-1H-indol-3-yl)carbonyl]piperazin-1-yl}-N-[2-(4-methyl-1,3-thiazol-5-yl)ethyl]pyrazine-2-carboxamide,
(29) 5-{4-[(1,5-dimethyl-2-phenyl-1H-pyrrolo[2,3-b]pyridin-3-yl)carbonyl]piperazin-1-yl}-N-[2-(4-methyl-1,3-thiazol-5-yl)-ethyl]pyrazine-2-carboxamide,
(30) 2-{(2E)-3-[1-methyl-2-(3,4-methylenedioxyphenyl)-1H-pyrrolo[2,3-b]pyridin-3-yl]prop-2-enoyl}-6,7-dimethoxy-1,2,3,4-tetrahydroisoquinoline hydrochloride,
(31) 5-{4-[(5-chloro-1-methyl-2-phenyl-1H-indol-3-yl)carbonyl]piperazin-1-yl}-N-[2-(4-methyl-1,3-thiazol-5-yl)ethyl]pyrazine-2-carboxamide dihydrochloride,
(32) N-{[5-{4-[(1,5-dimethyl-2-phenyl-1H-indol-3-yl)carbonyl]piperazin-1-yl}pyrazin-2-yl]carbonyl}-(S)-phenylalanine
(33) N-methyl-N-{[5-{4-[(1,5-dimethyl-2-phenyl-1H-indol-3-yl)carbonyl]piperazin-1-yl}pyrazin-2-yl]carbonyl}-(S)-phenylalaninemethyl ester,
(34) 4-{3-[3-(6,7-dimethoxy-1,2,3,4-tetrahydroisoquinolin-2-yl)-3-oxo-propenyl]-1-methyl-1H-pyrrolo[2,3-b]pyridin-2-yl}benzoic acid hydrochloride,
(35) methyl [6,7-dimethoxy-2-{(2E)-3-[1-methyl-2-(4-fluorophenyl)-1H-pyrrolo[2,3-b]pyridin-3-yl]prop-2-enoyl}-1,2,3,4-tetrahydroisoquinolin-1-yl]acetate hydrochloride, and
(36) [6,7-dimethoxy-2-{(2E)-3-[1-methyl-2-(4-fluorophenyl)-1H-pyrrolo[2,3-b]pyridin-3-yl]prop-2-enoyl}-1,2,3,4-tetrahydroisoquinolin-1-yl]acetic acid.

The present invention will now be described in detail.

In the present invention, "alkyl" includes straight or branched alkyl groups containing 1 to 10 carbons, for example, methyl, ethyl, n-propyl, isopropyl, n-butyl, isobutyl, sec-butyl, tert-butyl, n-pentyl, isopentyl, n-hexyl, isohexyl, n-heptyl, and isoheptyl. Straight alkyl groups having 1 to 7 carbon atoms are preferred and straight alkyl groups having 1 to 3 carbon atoms are more preferred, and include, for example, methyl, ethyl and n-propyl.

The alkyl moiety of "haloalkyl", "arylalkyl", "alkoxy", "akoxyalkyl", "haloalkoxy", "monoalkylamino", "dialkylamino", "hydroxyalkyl", "alkylsulfoniumamino", "monoalkylcarbamoyl", "dialkylcarbamoyl", "monoalkylaminosulfonyl", "dialkylaminosulfonyl" and "carboxyalkyl" includes the above-mentioned alkyl.

The cycloalkyl includes cycloalkyl group having 3 to 7 carbon aroms, for example, cyclopropyl, cyclobutyl, cyclopentyl, cyclohexyl, and cycloheptyl.

"Aryl" includes aryl groups having 6 to 10 carbon atoms, for example, phenyl, 1-naphthyl, and 2-naphthyl.

The aryl moiety of "arylalkyl" include the above-mentioned aryl.

"Aromatic heterocyclic group" includes 5- to 6-membered aromatic ring group having 1 to 4 heteroatoms selected from nitrogen, oxygen and sulfur, or benzene-fused ring thereof. When atoms composing the ring of the aromatic heterocyclic group include nitrogen or sulfur atom, such nitrogen or sulfur atom may form oxide. For example, 1-pyrolyl, 2-pyrolyl, 3-pyrolyl, 3-indolyl, 2-furanyl, 3-furanyl, 3-benzofuranyl, 2-thienyl, 3-thienyl, 3-benzothienyl, 2-oxazolyl, 4-isoxazolyl, 2-thiazolyl, 5-thiazolyl, 2-benzothiazolyl, 1-imidazolyl, 2-imidazolyl, 4-imidazolyl, 2-benzimidazolyl, 1H-1,2,4-triazol-1-yl, 1H-tertazol-5-yl, 2H-tertazol-5-yl, 2-pyridyl, 3-pyridyl, 4-pyridyl, 3-pyrazole, 2-pyrimidinyl, 4-pyrimidinyl, 2-pyradinyl and 1,3,5-triadin-2-yl are included.

"Halogen" includes, for example, fluorine, chlorine, bromine and iodine.

The halogen moiety of "haloalkyl" and "haloalkoxy" includes the above-mentioned halogen. "Haloalkyl" includes, for example, trifluoromethyl and 2,2,2-trifluoroethyl. "Haloalkoxy" includes, for example, trifluoromethoxy and 2,2,2-trifluoroethoxy.

"Acyl" includes acyl groups having 1 to 11 carbons, for example, formyl, acetyl, propionyl, butyryl, isobutyryl, benzoyl, 1-naphthoyl, and 2-naphthoyl.

"Cyclic amino" includes 4- to 8-membered ring group having 1 or 2 same or different members selected from the group consisting of nitrogen, oxygen and sulfur atoms as the atoms composing the ring. When the atoms composing the ring of cyclic amino includes nitrogen or sulfur atom, such nitrogen or sulfur atom may form oxide. For example, pyrolidino, piperidino, piperazinyl, 3-methylpiperazin-1-yl, homopiperazinyl, morpholino, thiomorphorino, imidazol-1-yl, thiazolin-3-yl are included.

"Saturated heterocyclic group" includes 5- to 6-membered saturated ring group having 1 to 4 heteroatoms selected from nitrogen, oxygen and sulfur, or benzene-fused ring thereof. For example, 1-pyrolidinyl, 2-pyrolidinyl, 1-morpholinyl, 1-piperidinyl, and 2-tetrahydrofuranyl are included.

The compound of the present invention represented by the above formula [1] can be produced by the method described below, or the method described in International Publication WO00/44743 or International Publication WO01/43746.

In production method described below, it is common that the source materials are used for reaction after protecting with the protecting groups (for example, methoxymethyl, 2-methoxyethoxymethyl, benzyl, 4-methoxybenzyl, triphenylmethyl, 4,4'-dimethoxytrityl, acetyl, tert-butoxycarbonyl, benzyloxycarbonyl, phthaloyl, tetrahydropyranyl or tertbutyldimethylsilyl) by the per se known methods, when the source materials have substituents intended not to be reacted (for example, hydroxy, amino or carboxy). After the reaction, the protecting groups can be removed by per se known methods such as catalytic reduction, alkali treatment and acid treatment. Process 1

The amide derivative [1] can be produced by reacting the indole derivative [6] with amine [7]. 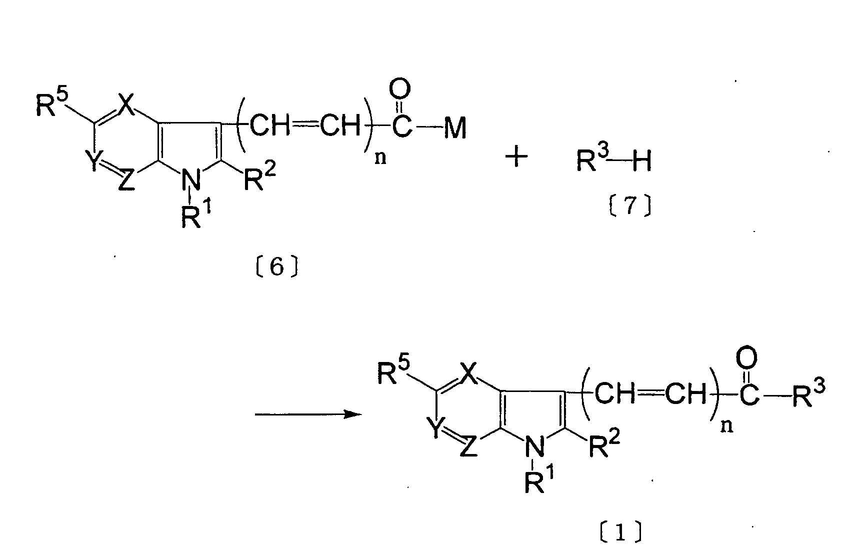 wherein R¹, R², R³, R⁵, X, Y, Z and n are as defined above, and M represents a leaving group such as hydroxy, or halogen (chlorine, bromine, iodine, etc), alkoxy (e.g., methoxy), aryloxy (e.g., p-nitrophenoxy), alkylsulfoxy (e.g., methanesulfoxy), arylsulfoxy (e.g., toluenesulfoxy), imidazolyl, alkylcarboxy or arylcarboxy.

Specifically, the amide derivative [1] can be produced by the method in which the indole derivative [6] (wherein M is the above-mentioned leaving group other than hydroxy), for example, acid halide, alkyl ester, active ester, imidazolide or mixed acid anhydride is appropriately reacted with amine [7], or by the method in which the indole derivative [6] (wherein M is hydroxy) is directly bound to amine [7] using a condensing agent [for example, 1-ethyl-3-(3-dimethylaminopropyl)carbodiimide hydrochloride (hereinafter referred to as WSCD·HCl), dicyclohexylcarbodiimide, diisopropyl-carbodiimide, benzotriazol-1-yl-tris(dimethylamino)phosphonium hexafluorophosphide salt, diphenylphosphorylazide or propane phosphate anhydride] in the presence or absence of an additive (N-hydroxysuccinimide, 1-hydroxybenzotriazole, 3-hydroxy-4-oxo-3,4-dihidro-1,2,3-triazine, and the like).

When acid halide (the indole derivative [6] wherein M is halogen) is used, the amide derivative [1] can be produced by reacting at -20 to 100°C in aprotic solvents (for example, polar solvents such as acetonitrile and; N, N-dimethyl formamide (DMF), ether solvents such as tetrahydrofuran (THF) and diethyl ether, halogenated hydrocarbon solvents such as chloroform and methylene chloride, hydrocarbon solvents such as benzene, toluene and n-hexane, and the mixtures thereof) in the presence of a base (for example, potassium carbonate, sodium carbonate, sodium hydrogen carbonate, potassium hydrogen carbonate, pyridine; 4-dimethylaminopyridine, triethylamine, sodium hydride and the like). The reaction time varies depending on the kinds of acid halide and amine [7] and the temperature of the reaction, and usually the appropriate reaction time is from 30 min to 24 hours. The molar amount of amine [7] for use is preferably 1 to 1.2 molar equivalents based on the acid halide.
Such acid halide can be produced by reacting the indole derivative [6] (wherein M is hydroxy) and thionyl halide (e.g., thionyl chloride, thionyl bromide) at -20 to 100°C in the absence of a solvent or in the same aprotic solvent as the above-mentioned in the presence or absence of the same base as the above-mentioned. The reaction time varies depending on the kind of acid halide and the temperature of the reaction, and usually the appropriate reaction time is 30 min to 24 hours. The amount of thionyl halide needed is equimolar or more equivalents based on the indole derivative [6], and an extremely excessive amount such as 10 molar equivalents or more can be used.

When a condensing agent is used, the amide derivative [1] can be produced by reacting at -20 to 100°C in the same aprotic solvent as the above-mentioned in the presence or absence of the same base as the above-mentioned. The reaction time varies depending on the kind of condensing agents and the temperature of the reaction, and usually the appropriate reaction time is 30 minutes to 24 hours. The amounts of amine [7] and a condensing agent for use are preferably 1 to 1.2 molar equivalents based on the indole derivative [6] (wherein M is hydroxy).

### Process 2

Among the compounds [1] of the present invention, the compound [1b] wherein R³ is the above-mentioned formula [2] and R^{3B} is the above-mentioned formula [3] can also be produced by reacting the indole derivative [8] with amine [9] in the same manner as in the process 1. 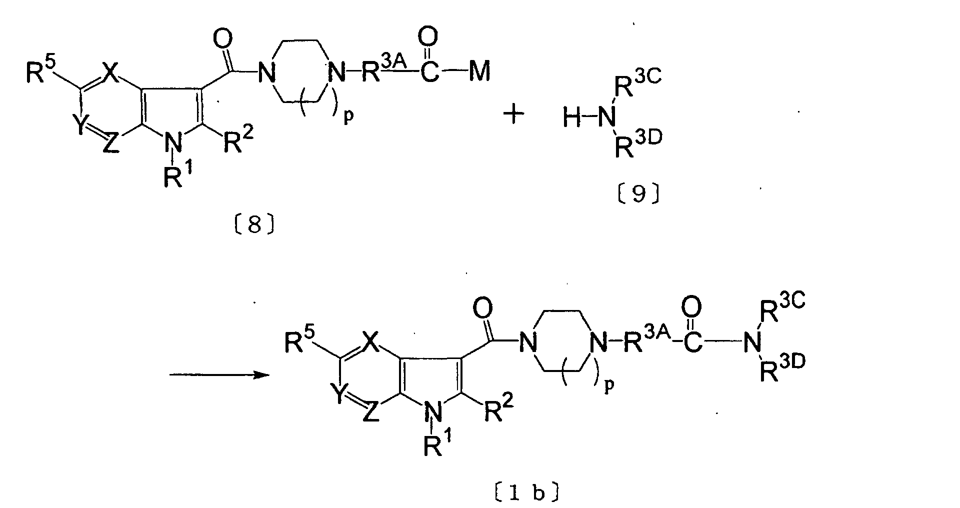 wherein R¹, R², R^{3A}, R^{3C}, R^{3D}, R⁵, X, Y, Z, p and M are as defined above.

Specifically, the amide derivative [1b] can be produced by the method in which the indole derivative [8] (wherein M is the above-mentioned leaving group other than hydroxy), for example, acid halide, alkyl ester, active ester, imidazolide or mixed acid anhydride is appropriately reacted with amine [9], or by the method in which the indole derivative [8] (wherein M is hydroxy) is directly bound to amine [9] using a condensing agent [for example, WSCD·HCl, dicyclohexylcarbodiimide, diisopropyl-carbodiimide, benzotriazol-1-yl-tris(dimethylamino)phosphonium hexafluorophosphide salt, diphenylphosphorylazide or propane phosphate anhydride] in the presence or absence of an additive (N-hydroxysuccinimide, 1-hydroxybenzotriazole, 3-hydroxy-4-oxo-3,4-dihidro-1,2,3-triazine, and the like).

Details of the reaction conditions in these reactions are the same as those in the process 1.

The indole derivative [6], the indole derivative [8], the amine [7] and the amine [9] used as the source materials in these reactions are per se known compounds, or can be produced by the per se known method or the method disclosed in the reference example.

The indole derivative [8] can be produced by version of the method of the process 1, or the method described in WO00/44743.

Among the indole compound [6], the compound [6a] wherein n is 1 and M is alkoxy and the compound [6b] wherein n is 1 and M is hydroxy can be produced from the indole derivative [10] in the following manner. 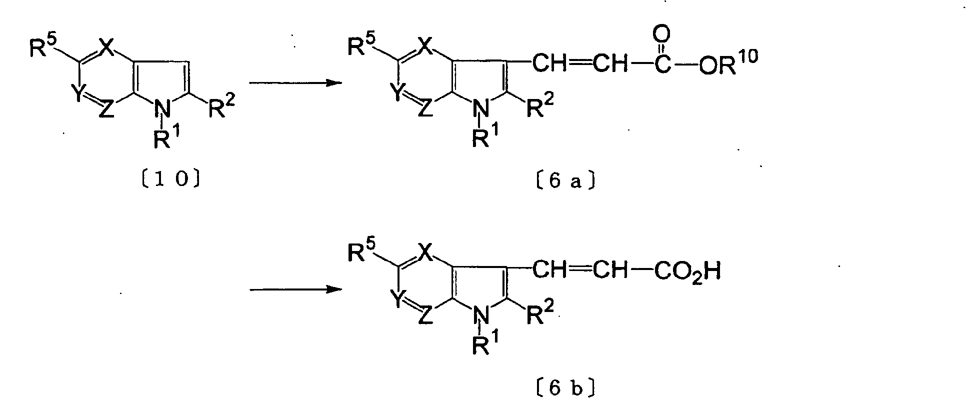 wherein R¹, R², R⁵, X, Y and Z are as defined above, and R¹⁰ represents alkyl.

Specifically, the compound [6a] can be produced by reacting (Vilsmeier reaction) the indole derivative [10] with phosphoryl chloride at -20 to 100°C in N,N-dimethylformamide solvent to obtain a formyl compound and subsequently reacting (Horner-Emmons reaction) the formyl compound with a phosphonic acid ester derivative (e.g., ethyl diethylphosphinoacetate, methyl diethylphosphinoacetate, allyl diethylphosphinoacetate, tert-butyl diethylphosphinoacetate, phenyl diethylphosphinoacetate) at -20 to 100°C in the above aprotic solvent in the presence of the above base.

Alternatively, it can be produced by reacting the indole derivative [10] with acrylic acid derivatives (e.g., ethyl acrylate, n-butyl acrylate, tert-butyl acrylate, methyl acrylate, n-hexyl acrylate, tert-butyl acrylate) at -20 to 150°C in an acetic acid solvent in the presence of a palladium (II) catalyst (e.g., palladium acetate, palladium chloride, palladium trifluoroacetate).

The indole derivative [10] used as the source materials in these reactions are per se known compounds, or can be produced by the per se known method, or the method disclosed in the reference example.

The compound [6b] can be produced from the compound [6a] by the per se known methods such as alkali treatment, acid treatment and treatment using palladium catalysts (e.g., tetrakis(triphenylphosphine)palladium). Alternatively, it can be produced by reacting (Knoevenagel condensation) the formyl compound produced from the above indole derivative [10] with malonic acid at 0 to 100°C in a pyridine solvent in the presence of a catalytic amount of piperidine.

The compound of the present invention can be isolated and purified from the above reaction mixture using the conventional means for isolation and purification such as extraction, concentration, neutralization, filtration, recrystallization, column chromatography or thin layer chromatography.

The compound of the present invention may exist in geometrical isomers (Z and E forms). These geometrical isomers and their mixtures also fall within the scope of the present invention.

The compound of the present invention may exist in keto-enol tautomers. These tautomers and their mixtures also fall within the scope of the present invention.

The compound of the present invention can be used in the form of a free base as a medicine, however, it can be also used as a pharmaceutically acceptable salt made by the per se known methods. These salts include salts of mineral acids such as hydrochloric acid, hydrobromic acid, sulfuric acid and phosphoric acid, and organic acids such as acetic acid, citric acid, tartaric acid, maleic acid, succinic acid, fumaric acid, p-toluene sulfonic acid, benzene sulfonic acid and methane sulfonic acid.

For example, hydrochloride of the amide derivative of the present invention can be obtained by dissolving the amide derivative in an alcohol, ethyl acetate or ether solution of hydrogen chloride.

The compound of the present invention has excellent high absorptivity upon oral administration and/or high inhivitory action against TGF-β as compared with the amide derivative whose inhivitory action against TGF-β is disclosed in aforementioned International Publication WO00/44743, as shown in the test examples given below. Due to its low toxicity, the compound of the present invention is useful for the prevention and treatment of diseases of the liver, gall bladder, heart, lung, skin, pancreas, large intestine, prostate gland, uterus, blood vessels and other organs, and specifically, hepatic fibrosis, biliary fibrosis, cirrhosis of the liver, pulmonary fibrosis, adult respiratory distress syndrome, bone marrow fibrosis, scleroderma, pachyderma, systemic sclerosis, intraocular proliferative disorders, proliferative vitreous retinopathy, cataract, cardiac fibrosis after infarction, restenosis after angioplasty, arterial sclerosis, hyperplasia of connective tissues (abdominal adhesion, scar, keloid) resulting from wounds such as surgical incisions, injury, or burns, fibrosis after radiation therapy, chronic pancreatitis, chronic nephritis, chronic renal insufficiency, diabetic nephropathy, fibrosing tumors, nasal polyp, periodontal disease, chronic ulcerative colitis, sclerosing Hodgkin's disease, prostatic hypertrophy, fibroid uterus, and various tumors. Alternatively, the compound of the present invention is useful as an antitumor agent, a metastasis inhibitor and a hair-restoring agent against various tumors.

The compound of the present invention is useful as an excellent therapeutic agent for nephritis, and is particularly useful as a therapeutic agent for chronic renal insufficiency, diabetic nephropathy and chronic glomerulonephritis, more particularly proliferative glomerulonephritis, among nephritis.

When the compound of the present invention is administered as a medicine, it can be administered to mammals, including humans, either by itself or as a pharmaceutical composition in which the compound is contained in a pharmaceutically acceptable non-toxic and inert carrier in the proportion of, for example, 0.1 to 99.5%, or preferably 0.5 to 90%.

One or more auxiliary agents for formulation such as fillers or a solid, semisolid or liquid diluent are used. It is desirable to administer the pharmaceutical composition is in unit dosage form. The pharmaceutical composition of the present invention can be administered intravenously, orally, directly to the target tissue, topically (e.g., transdermally), or rectally. The formulation suitable for these administration routes is specifically administered.

It is desirable to set the dosage of the compound as a TGF-β inhibitor by considering the condition of the patient, such as age, body weight, and the characteristics and severity of the disease and other factors such as the administration route; but usually for adults, an amount in the range of 0.1 to 1000 mg/person per day, and preferably 1 to 500 mg/ person per day, is generally a dose of the compound of the present invention.

In some cases, amounts below this range are sufficient, and conversely, in other cases larger amounts are required. It can be administered by dividing the total dosage into two or three doses per day.

### BEST MODE FOR CARRYING OUT THE INVENTION

The present invention is described in more detail by presenting Reference Examples, Examples, Test Examples and Formulation Examples of the compound of the present invention, to which, however, the present invention is not limited.

### Reference Example 1

### N-methyl-4-methylformanilide

p-toluidine (51.2 g, 0.48mol) and methyl orthof ormate (76.5 g, 0.72 mol) were stirred with heating at 100°C for one hour in the presence of concentrated sulfuric acid (1.9 g, 0.019 mol) while distilling off by-producing methanol. The mixture was stirred at 175°C for another one hour. Then the reaction solution was distilled under reduced pressure to obtain a colorless oil (21.9 g).
Boiling point: 103-107°C/5 mmHg

### Reference Example 2

### N-methyltoluidine

N-methyl-4-methylformanilide(53.5 g, 0.36 mol) was heated at reflux in 10% hydrochloric acid (163 ml) for one hour. The reaction solution was cooled, alkalified with an aqueous 15% potassium hydroxide solution and then extracted with ether. The organic layer was washed with saturated brine, dried and then concentrated. The resulting residue was distilled under reduced pressure to obtain a colorless oily product (42.6 g)
Boiling point: 100 to 103°C/25mmHg

### Reference Example 3

### N-nitroso-N-methyltoluidine

N-methyltoluidine (42.3 g, 0.35 mol) was added in concentrated hydrochloric acid (52 ml) and ice (142 g) and then aqueous solution (86 ml) of sodium nitrite (24.1 g, 0.35 mol) was gradually added dropwise so that the reaction temperature does not exceed 10°C. After stirring another one hour, the reaction solution was extracted with ethyl acetate, washed with water, dried and then concentrated. The resulting crude crystal was recrystallized from n-hexane to obtain a yellow crystal (47. 9 g).

### Reference Example 4

### 1-methyl-1-(4-methylphenyl)hydrazine

A zinc powder (85.3 g, 1.3 mol) was suspended in water (142 ml) and an acetic acid solution (90 ml) of N-nitroso-N-methyltoluidine (47.8 g, 0.32 mol) was added dropwise over 2.5 hours under ice cooling. After stirring at room temperature for another 2.5 hours, the reaction solution was filtered. The insolubles were sufficiently washed with 10% hydrochloric acid. The mother liquor was alkalified with an aqueous 40% sodium hydroxide solution and then extracted with ether. The organic layer was washed with saturated brine, dried and then concentrated. The residue was purified by silica gel column chromatography (chloroform, and then chloroform:methanol = 50:1) to obtain a brown oily product (33.3 g).

### Reference Example 5

### Another process for producing 1-methyl-1-(4-methylphenyl)hydrazine

Under an argon gas flow, to a suspension of sodium amide (2.14 g, 54.9 mmol) in anhydrous tetrahydrofuran (40 ml), a solution of p-tolylhydrazine (6.38 g, 52.3 mmol) in anhydrous tetrahydrofuran (20 ml) was added dropwise under ice cooling over 80 minutes. After returning to room temperature, the reaction solution was stirred for 30 minutes while bubbling an argon gas, thereby to distill off ammonia. After obtaining a nearly uniform brown solution, the solution was cooled to 10 to 15°C and a solution of methyl iodide (7.79 g, 54.9 mmol) in anhydrous tetrahydrofuran (20 ml) was added dropwise over 80 minutes. After stirring as it is for 30 minutes, the reaction solution was mixed with ice and then concentrated. The residue was mixed with water, extracted with ethyl acetate, dried and then concentrated. The residue was distilled under reduced pressure to obtain the objective product (4.1 g).
Boiling point: 61 to 63°C/2 mmHg

### Reference Example 6

### Ethyl 1,5-dimethyl-2-phenylindole-3-carboxylate

1-methyl-1-(4-methylphenyl)hydrazine (10.3 g, 76 mmol) and benzoylethyl acetate (14.5 g, 76 mmol) were stirred in acetic acid (40 ml) at room temperature overnight. The reaction solution was mixed with ice water and sodium hydrogen carbonate and then extracted with ethyl acetate. The organic layer was washed with saturated brine, dried and then concentrated. The residue was mixed with polyphosphoric acid (100 g) and stirred at 50 to 60°C for 30 minutes. The reaction solution was poured into ice water, neutralized with an aqueous 10% sodium hydroxide solution and then extracted with ethyl acetate. The organic layer was washed with saturated brine, dried and then concentrated. The residue was purified by silica gel column chromatography (chloroform) to obtain a pale yellow needle crystal (6.15 g).

### Reference Example 7

### 1,5-dimethyl-2-phenylindole-3-carboxylic acid

Ethyl 1,5-dimethyl-2-phenylindole-3-carboxylate (6.15 g, 21 mmol), sodium hydroxide (8 g) and water (35 ml) were added to ethanol (80 ml) and the mixture was heated at reflux overnight. The reaction solution was concentrated, mixed with an aqueous 2N-sodium hydroxide solution and then extracted with ethyl acetate. The aqueous layer was acidified with 6N-hydrochloric acid and then extracted with chloroform. The organic layer was washed with saturated brine, dried and then concentrated. The residue was purified by silica gel column chromatography (chloroform) to obtain a pale yellow crystal (5.06 g).

### Reference Example 8

### 2-phenyl-1H-pyrrolo[2,3-b]pyridine

Under an argon gas flow, a solution of 3-methylpyridine (10 g) in anhydrous tetrahydrofuran (100 ml) was added dropwise in a solution of about 1.5 M lithium diisopropylamide (hereinafter referred to as LDA) in anhydrous tetrahydrofuran (200 ml) prepared immediately before the reaction over one hour with stirring at 0°C, followed by stirring at 0°C for another 30 minutes. In a suspension containing a lithium salt deposited therein, a solution of cyanobenzene (10.1 g) in anhydrous tetrahydrofuran (100 ml) was added dropwise over one hour while maintaining at 0°C, followed by stirring at 0°C for 1.5 hours. The above LDA solution (200 ml) was further added, followed by stirring with heating at 40°C for 4 hours. After ice cooling, the reaction mixture was partitioned by adding water and saturated brine. The resulting organic layer was washed twice with saturated brine, dried and then concentrated to obtain a pale orange powder. The resulting powder was washed with diethyl ether and then dried to obtain the objective product (14.3 g) as a white powder.

### Reference Example 9

### 1-methyl-2-phenyl-1H-pyrrolo[2,3-b]pyridine

Under an argon gas flow, sodium hydride(60% dispersion in mineral oil) (3.2 g) was washed three times with n-hexane (20 ml), ice-cooled and then suspended in dimethylformamide (50 ml). Then a solution of 2-phenyl-1H-pyrrolo[2,3-b]pyridine (14 g) in dimethylformamide (200 ml) was added dropwise over one hour and the mixture was stirred at room temperature for 30 minutes. After ice cooling again, a solution of methyl iodide (10.6 g) in dimethylformamide (50 ml) was added dropwise over one hour, followed by stirring under ice cooling for 3 hours. After the reaction solution was mixed with water and extracted with ethyl acetate, the organic layer was washed three times with saturated brine, dried and then concentrated. The resulting residue was purified by silica gel column chromatography (chloroform:methanol = 50:1) to obtain a colorless oily product (11.1 g).

### Reference Example 10

### 1-methyl-2-phenyl-1H-pyrrolo[2,3-b]pyridine-3-carbaldehyde

Under an argon gas flow, 1-methyl-2-phenyl-1H-pyrrolo[2,3-b]pyridine (11 g) was dissolved in dimethylformamide (50 ml) and phosphoryl chloride (12.3 g) was added dropwise under ice cooling over 30 minutes and, after stirring at the same temperature for 3 hours, the reaction solution was poured into ice water (200 ml) and the pH was adjusted to 14 or higher by adding sodium hydroxide. The deposited white powder was collected by suction filtration, washed with water and diethyl ether and then dried under reduced pressure at 40°C for 10 hours to obtain the objective product (11.6 g) as a white powder.

### Reference Example 11

### Ethyl 3-(1-methyl-2-phenyl-1H-pyrrolo[2,3-b]pyridin-3-yl)acrylate

Under an argon gas flow, sodium hydride(60% dispersion in mineral oil) (2.95 g) was washed with three times with n-hexane (20 ml) and suspended in dimethylformamide (10 ml) after ice cooling. Then a solution of diethylphosphinoethyl acetate (16.5 g) in dimethylformamide (50 ml) wad added dropwise over one hour, followed by stirring at room temperature for 30 minutes. To the reaction solution, a solution of 1-methyl-2-phenyl-1H-pyrrolo[2,3-b]pyridine-3-carbaldehyde (11.6 g) in dimethylformamide (150 ml) was added dropwise over one hour, followed by stirring at room temperature for another 3 hours. After the reaction solution was mixed with water under ice cooling and extracted with ethyl acetate, the organic layer was washed three times with saturated brine, dried and then concentrated under reduced pressure. The resulting product was mixed with methanol (150 ml) and, after stirring, the deposited objective product was collected by filtration and then dried to obtain a white powder (15 g).

### Reference Example 12

### 3-(1-methyl-2-phenyl-1H-pyrrolo[2,3-b]pyridin-3-yl)acrylic acid

Under an argon gas flow, ethyl 3-(1-methyl-2-phenyl-1H-pyrrolo[2,3-b]pyridin-3-yl)acrylate (15 g) was dissolved in 2-methyl-2-propanol (60 ml) and an aqueous 10% sodium hydroxide solution (120 ml) was added, followed by stirring with heating at 100°C for 12 hours. After removing 2-methyl-2-propanol under reduced pressure, the reaction solution was poured into water and the pH was adjusted to 4 with 2N-hydrochloric acid. The deposited white powder was collected by filtration and then dried to obtain the objective product (13.2 g).

### Reference Example 13

### (1R,2S)-2-methoxy-1-aminoindane

Under an argon gas flow, sodium hydride(60% dispersion in mineral oil) (0.6 g) was washed three times with n-hexane (20 ml) and suspended in dimethylformamide (10 ml) after ice cooling.

Then a solution of (1R, 2S)-1-amino-2-indanole (2 g) in dimethylformamide (5 ml) was added dropwise over 30 minutes, followed by stirring at room temperature for 30 minutes. After ice cooling again, a solution of methyl iodide (2.1 g) in dimethylformamide (5 ml) was added dropwise over 30 minutes, followed by stirring under ice cooling for another one hour. After water was added and saturated brine was added, the objective product was extracted with ethyl acetate, dried and then concentrated. The residue was purified by silica gel column chromatography (chloroform:methanol = 10:1) to obtain the objective product (1.3 g) as a pale brown oily product.

### Reference Example 14

### (2E)-3-(1-methyl-2-phenyl-1H-indol-3-yl)acrylic acid

Under an argon atmosphere, 1-methyl-2-phenylindole (40 g) was dissolved in acetic acid (600 ml) and ethyl acrylate (62 ml) and palladium (II) acetate (4.34 g) were added, followed by stirring at 100°C overnight. The reaction solution was filtered through celite to remove insolubles and the filtrate was concentrated. After purifying by silica gel column chromatography (n-hexane:ethyl acetate = 10:1), the resulting crude crystal was washed with petroleum ether to obtain a pale yellow powder (34 g). The pale yellow powder was dissolved in tert-butanol (130 ml) and an aqueous 10% sodium hydroxide solution (260 ml), followed by stirring at 100°C overnight. After returning to room temperature, the reaction solution was mixed with water and then washed with ethyl acetate. The aqueous layer was acidified with 10% hydrochloric acid and the deposited crystal was collected by filtration, washed with water and diethyl ether and then dried to obtain the objective product (31.2 g).

### Reference Example 15

### Methyl 6-(piperazin-1-yl)nicotinate

### Step 1

6-chloronicotinic acid (5 g) was dissolved in dimethylformamide (100 ml) and potassium carbonate (13.2 g) was added and, after adding dropwise methyl iodide (9 g) at 0°C, the mixture was stirred at 60°C for 2 hours. After the mixture was mixed with water and extracted with ethyl acetate, the organic layer was washed several times with water, dried and then concentrated to obtain an orange crystal (5.6 g).

### Step 2

Methyl 6-chloronicotinate (5.6 g) was dissolved in toluene (50 ml) and piperazine (8.43 g) was added, followed by stirring at 100°C for 5 hours. After returning to room temperature, the deposit was removed by filtration and water was added to the filtrate. After extracting with ethyl acetate, the organic layer was washed several times with water, dried and then concentrated. The residue was purified by silica gel column chromatography (chloroform:methanol:ammonia water = 20:1:0.1) to obtain a pale yellow crystal (5.1 g).

### Reference Example 16

### 6-{4-[(1,5-dimethyl-2-phenyl-1H-indol-3-yl)carbonyl]piperazin-1-yl}nicotinic acid

### Step 1

1,5-dimethyl-2-phenyl-indole-3-carboxylic acid (2 g) was dissolved in dimethylformamide (50 ml) and WSCD·HCl (1.59 g), 1-hydroxybenzotriazole (1.12 g) and triethylamine (0.83 g) were added, followed by stirring for 30 minutes. Then methyl 6-(piperazin-1-yl)nicotinate (1.83 g) was added, followed by stirring at room temperature overnight. The reaction solution was poured into water, extracted with ethyl acetate, washed several times with water, dried and then concentrated. The resulting crude crystal was crystallized from chloroform-ether to obtain a white powder (3.5 g).

### Step 2

6-{4-[(1,5-dimethyl-2-phenyl-1H-indol-3-yl)carbonyl]piperazin-1-yl}nicotinic acid methylester (3.5 g) was dissolved in an aqueous 1N sodium hydroxide solution (22 ml) and methanol (40 ml), followed by stirring at 60°C for 3 hours. The reaction solution was poured into ice water, neutralized with 10% hydrochloric acid, extracted with chloroform, dried and then concentrated. The resulting crude crystal was crystallized from chloroform-ether to obtain a white powder (2.75 g).

### Reference Example 17

### 1-methyl-2-phenyl-1H-pyrrolo[2,3-b]pyridine-3-carboxylic acid

Under an argon atmosphere, 1-methyl-2-phenyl-1H-pyrrolo[2,3-b]pyridine (5.5 g) was dissolved in dimethylformamide (25 ml) and trifluoroacetic anhydride (6. 66 g) was added dropwise at 0°C, followed by stirring for one hour. The reaction solution was mixed with water (50 ml), extracted with ethyl acetate (100 ml), and then concentrated. To the residue, an aqueous 10% sodium hydroxide solution (80 ml) and methanol (80 ml) were added, followed by stirring at 100°C for 5 hours. After returning to room temperature, the reaction solution was mixed with water and washed with ethyl acetate. The aqueous layer was acidified with 10% hydrochloric acid and the deposited crystal was collected by filtration, washed with water and then dried to obtain the objective product (5.5 g).

### Reference Example 18

### 2-hydroxy-5-carbomethoxy-pyrazine

1,2-diamino-1,2-dicyanoethylene (25 g) and glyoxylic acid monohydrate (21.3 g) were dissolved in 2N-hydrochloric acid (240 ml), and the solution was stirred at room temperature for 4 hours and then concentrated under reduced pressure. To the residue, an aqueous 10% sodium hydroxide solution (600 ml) was added and, after heating at reflux for 13 hours, the reaction solution was ice-cooled and neutralized with concentrated hydrochloric acid. The resulting ocher precipitate was collected by filtration and then washed with water and acetone. The crude product was suspended in acetic acid (800 ml) and heated at reflux at 120°C for 2 hours. The reaction mixture was returned to room temperature and filtered, and then the filtrate was concentrated. The resulting precipitate was collected by filtration and washed with ether. After ice cooling methanol (600 ml), thionyl chloride (60 ml) was added dropwise, followed by stirring at room temperature for one hour, the addition of the crude product and further stirring at 80°C. The reaction solution was concentrated and then purified by silica gel column chromatography (chloroform:methanol = 10:1 to 5:1) to obtain the objective product (8.0 g).

### Reference Example 19

### 2-piperazinyl-5-carbomethoxypyrazine

### Step 1

2-hydroxy-5-carbomethoxy-pyrazine (15.4 g) was dissolved in phosphorus oxychloride (100 ml) and several drops of dimethylformamide were added, followed by stirring with heating at 140°C for 3 hours. After the reaction solution was poured into ice water and extracted with chloroform, the organic layer was washed with an aqueous saturated sodium hydrogen carbonate solution and saturated brine, dried and then concentrated. The resulting crystal was collected by filtration and washed with ether to obtain 2-chloro-5-carbomethoxy-pyrazine (14.0 g).

### Step 2

2-chloro-5-carbomethoxy-pyrazine (14.0 g) and piperazine (21.0 g) were dissolved in toluene (200 ml) , followed by stirring with heating at 130°C for one hour. The reaction solution was mixed with ice water, extracted with chloroform, washed with saturated brine, dried and then concentrated. The resulting crystal was collected by filtration and then washed with ether and a small amount of methanol to obtain the objective product (17.1 g).

### Reference Example 20

### 5-{4-[(1,5-dimethyl-2-phenyl-1H-indol-3-yl)carbonyl]piperazin-1-yl}-2-(carbomethoxy)pyrazine

1,5-dimethyl-2-phenylindole-3-carboxylic acid (9.55 g) and 2-piperazinyl-5-carbomethoxypyrazine (8.0 g) were dissolved in dimethylformamide (360 ml) and triethylamine (10.93 g), WSCD· HCl (10.35 g) and 1-hydroxybenzotriazole (7.30 g) were added, followed by stirring at 50°C for one day. The reaction solution was mixed with water, extracted with ethyl acetate, washed with saturated brine, dried and then concentrated. The residue was purified by silica gel column chromatography (chloroform:methanol = 30:1) to obtain the objective product (16.16 g).

### Reference Example 21

### 5-{4-[(1,5-dimethyl-2-phenyl-1H-indol-3-yl)carbonyl]piperazin-1-yl}pyrazine-2-carboxylic acid

5-{4-[(1,5-dimethyl-2-phenyl-1H-indol-3-yl)carbonyl]piperazin-1-yl}-2-(carbomethoxy)pyrazine (16.16 g) was dissolved in an aqueous 1N sodium hydroxide solution (150 ml) and methanol (300 ml), followed by stirring with heating at 80°C for 5 hours. After the reaction solution was ice-cooled and neutralized with 1N hydrochloric acid (150 ml), the precipitate was collected by filtration, washed with ether and then dried under reduced pressure to obtain the objective product (11.58 g).

### Reference Example 22

### 6-phenyl-5H-pyrrolo[2,3-b]pyrazine

Under an argon gas flow, diisopropylamine (7.70 g) was dissolved in tetrahydrofuran (50 ml), followed by stirring at -78°C. A solution of 1.52 M n-butyl lithium in hexane (50 ml) was slowly added dropwise. After the dropwise addition, the mixture was slowly returned to room temperature over 30 minutes and cooled to -78°C again to prepare lithium diisopropylamide. To this was added 2-methylpyrazine (4.29 g), the solution was stirred at 0°C for 30 minutes and benzonitrile (4.7 g) was slowly added dropwise so that the inner temperature does not exceed 15°C. After stirring at 0°C for 90 minutes, lithium diisopropylamide was added dropwise, followed by stirring at 40°C for 3 hours. The reaction solution was mixed with ice water, extracted with chloroform, washed with saturated brine, dried and then concentrated. The residue was purified by silica gel column chromatography (chloroform:n-hexane = 20:1) to obtain the objective product (2.2 g).

### Reference Example 23

### 5-methyl-6-phenyl-5H-pyrrolo[2,3-b]pyrazine

6-phenyl-5H-pyrrolo[2,3-b]pyrazine (0.8 g) was dissolved in dimethylformamide (40 ml) and sodium hydride (60% dispersion in mineral oil) (246 mg) was added under ice cooling, followed by stirring for 10 minutes. Then, methyl iodide (0.88 g) was added dropwise and the mixture was stirred at room temperature for one day. The reaction solution was mixed with ice water, extracted with ethyl acetate, washed with saturated brine, dried and then concentrated. The residue was purified by silica gel column chromatography (n-hexane:ethyl acetate = 4:1 to 1:1) to obtain the objective product (0.68 g).

### Reference Example 24

### 5-methyl-6-phenyl-5H-pyrrolo[2,3-b]pyrazine-7-carboxylic acid

5-methyl-6-phenyl-5H-pyrrolo[2,3-b]pyrazine (0.68 g) was dissolved in dimethylformamide (4 ml) and trifluoroacetic anhydride (0.82 g) was added under ice cooling, followed by stirring for one hour. After the reaction, the reaction solution was extracted with ethyl acetate (50 ml), washed with saturated brine and then concentrated. The resulting crude product was dissolved in an aqueous 1N sodium hydroxide solution (15 ml) and methanol (30 ml), followed by stirring with heating at 80°C for one hour. After the reaction solution was ice-cooled, neutralized with 1N hydrochloric acid, the precipitate was collected by filtration, washed with ether and then dried to obtain the objective product (227 mg).

### Reference Example 25

### 5-{4-[(5-methyl-6-phenyl-5H-pyrrolo[2,3-b]pyrazin-7-yl)carbonyl]piperazin-1-yl}pyrazine-2-carboxylic acid

### Step 1

5-methyl-6-phenyl-5H-pyrrolo[2,3-b]pyrazine-7-carboxylic acid (227 mg) and 2-piperazinyl-5-carbomethoxypyrazine (200 mg) were dissolved in dimethylformamide (10 ml) and triethylamine (0.28 g), WSCD·HCl (0.26 g) and 1-hydroxybenzotriazole (0.15 g) were added, followed by stirring at 50°C for one day. The reaction solution was mixed with water, extracted with ethyl acetate, washed with saturated brine, dried and then concentrated. The residue was purified by silica gel column chromatography (chloroform:methanol = 20:1) to obtain methyl 5-{4-[(5-methyl-6-phenyl-5H-pyrrolo[2,3-b]pyrazin-7-yl)carbonyl]piperazin-1-yl}pyrazine-2-carboxylate (398 mg).

### Step 2

Methyl 5-{4-[(5-methyl-6-phenyl-5H-pyrrolo[2,3-b]pyrazin-7-yl)carbonyl]piperazin-1-yl}pyrazine-2-carboxylate (398 mg) was dissolved in an aqueous 1N sodium hydroxide solution (10 ml) and methanol (20 ml), followed by stirring with heating at 80°C for 5 hours. After the reaction solution was neutralized with 1N hydrochloric acid under ice cooling, the precipitate was collected by filtration, washed with ether and then dried under reduced pressure to obtain the objective product (300 mg).

### Reference Example 26

### 1-hydroxy-2-phenyl-3-ethoxycarbonyl-1H-pyrrolo[3,2-b]pyridine

### Step 1

In dimethylformamide (15 ml), sodium hydride (60% dispersion in mineral oil) (1.51 g) was suspended and benzoylethyl acetate (7.27 g) was added dropwise so that the temperature does not exceed 50°C. After stirring at room temperature for 20 minutes, 2-chloro-3-nitropyridine (2.0 g) was added by several portions, followed by stirring at room temperature for 80 hours. The reaction solution was mixed with water,extracted with ethyl acetate, dried and then concentrated. The residue was purified by silica gel column chromatography (n-hexane:ethyl acetate = 3:1) to obtain 2-(3-nitropyridin-2-yl)benzoylethyl acetate (1.4 g) as a yellow oily product.

### Step 2

2-(3-nitropyridin-2-yl)benzoylethyl acetate (1.4 g) was dissolved in ethanol (120 ml) and 5% palladium-carbon (1.0 g) was added and the mixture was subjected to catalytic hydrogenation for 4 hours. After filtration, the filtrate was concentrated and the residue was purified by silica gel column chromatography (chloroform:methanol = 19:1 to 9:1) to obtain a yellow powder (0. 83 g).

### Reference Example 27

### 2-phenyl-3-ethoxycarbonyl-1H-pyrrolo[3,2-b]pyridine

1-hydroxy-2-phenyl-3-ethoxycarbonyl-1H-pyrrolo[3,2-b] pyridine (0.50 g) was dissolved in acetic acid (30 ml) and zinc powder (1.26 g) was added, and then the mixture was heated at reflux for one hour. After filtration, the filtrate was concentrated and the residue was mixed with an aqueous saturated sodium hydrogen carbonate solution, extracted with chloroform, dried and then concentrated to obtain a brown powder (0.4 g).

### Reference Example 28

### 1-methyl-2-phenyl-3-ethoxycarbonyl-1H-pyrrolo[3,2-b]pyridine

2-phenyl-3-ethoxycarbonyl-1H-pyrrolo[3,2-b]pyridine (0.4 g) was dissolved in dimethylformamide (20 ml) and sodium hydride (60% dispersion in mineral oil) (90 mg) was added, followed by stirring at room temperature for 15 minutes. Then, methyl iodide (0.32 g) was added and the mixture was stirred at room temperature for 3.5 hours. The reaction solution was mixed with water, extracted with ethyl acetate, dried and then concentrated. The residue was purified by silica gel column chromatography (chloroform:methanol = 19:1) to obtain a brown powder (0.32 g).

### Reference Example 29

### 1-methyl-2-phenyl-1H-pyrrolo[3,2-b]pyridine-3-carboxylic acid

1-methyl-2-phenyl-3-ethoxycarbonyl-1H-pyrrolo[3,2-b]pyridine (0.32 g) was dissolved in ethanol (3 ml) and an aqueous solution (1 ml) of potassium hydroxide (0.19 g) was added and the mixture was heated at reflux for 6 hours. After concentration, water was added and the pH was adjusted to 5 with 1N-hydrochloric acid. The solution was extracted with chloroform, dried and then concentrated to obtain a brown powder (0.27 g).

### Reference Example 30

### 5-{4-[(1-methyl-2-phenyl-1H-pyrrolo[3,2-b]pyridin-3-yl)carbonyl]piperazin-1-yl}-2-pyrazinecarboxylic acid

### Step 1

1-methyl-2-phenyl-1H-pyrrolo[3,2-b]pyridine-3-carboxylic acid (0.15 g) and 2-piperazinyl-5-carbomethoxypyrazine (0.19 g) were dissolved in dimethylformamide (5 ml) and triethylamine (0.18 g), WSCD·HCl (0.17 g) and 1-hydroxybenzotriazole (0.12 g) were added, followed by stirring at room temperature overnight. The reaction solution was mixed with water, extracted with ethyl acetate, dried and then concentrated. The residue was purified by silica gel column chromatography (chloroform:methanol = 19:1) to obtain a pale brown powder (0.37 g).

### Step 2

Methyl 5-{4-[(1-methyl-2-phenyl-1H-pyrrolo[3,2-b]pyridin-3-yl)carbonyl]piperazin-1-yl}pyrazine-2-carboxylate (0.37 g) was dissolved in methanol and an aqueous 10% sodium hydroxide solution (3 ml) was added, followed by stirring at 60 °C for one hour. After concentration, the reaction solution was mixed with water, neutralized with 1N-hydrochloric acid, extracted with chloroform, dried and then concentrated to obtain a pale brown powder (0.32 g).

In the same manner as in Reference Examples 26 to 30, 5-{4-[(1-methyl-2-phenyl-1H-pyrrolo[2,3-c]pyridin-3-yl)carbonyl]piperazin-1-yl}-2-pyrazinecarboxylic acid was synthesized.

### Reference Example 31

### 1-benzyloxycarbonyl-4-(5-bromopyridin-2-yl)piperazine

Under an argon atmosphere, 1-benzyloxycarbonyl-4-(pyridin-2-yl)piperazine (16 g) was dissolved in dihloromethane (300 ml) and N-bromosuccinimide (10.5 g) was added by several portions at 0°C, followed by stirring for one hour. The reaction solution was mixed with water, extracted with chloroform, dried and then concentrated. The residue was purified by silica gel column chromatography (n-hexane:ethyl acetate = 5:1). The resulting crude crystal was washed with petroleum ether to obtain the objective product (18.4 g).

### Reference Example 32

### 2-[(1-benzyloxycarbonyl)piperazin-4-yl]pyridine-5-boric acid

1-benzyloxycarbonyl-4-(5-bromopyridin-2-yl)piperazine (9 g) was dissolved in tetrahydrofuran (90 ml) and boric acid triisopropyl ester (6.52 g) was added. Under an argon atmosphere, 1.52M n-butyl lithium (22 ml) was added dropwise (inner temperature: -70°C or lower) at -78°C over 1.5 hours, followed by stirring for 3 hours. The reaction solution was heated to 15°C and an aqueous 1M potassium hydroxide solution (78 ml) was slowly added (inner temperature: 20°C). After stirring for 10 minutes, the reaction solution was concentrated and the residue was dissolved in isopropanol (26 ml) and an aqueous 1M sulfuric acid solution (72 ml) was added at 55-60°C (pH3-4). After stirring at 55-60°C for 2 hours and returning to room temperature, the crystal was collected by filtration, washed with water and then dried to obtain the objective product (5.4 g).

### Reference Example 33

### 2-benzyloxymethyl-5-bromo-2H-tetrazole

2-benzyloxymethyl-2H-tetrazole (6.2 g) was dissolved in tetrahydrofuran (90 ml) and tetramethylethylenediamine (7.69 g) was added. Under an argon atmosphere, 1.52M n-butyl lithium (22 ml) was added dropwise (inner temperature: -70°C) at -78°C over 30 minutes, followed by stirring for 10 minutes. A solution of bromine (5.29 g) in tetrahydrofuran (48 ml) was added dropwise over 30 minutes, followed by stirring for 2.5 hours. The reaction solution was mixed with water, extracted with diethyl ether, dried and then concentrated. The residue was purified by silica gel column chromatography (n-hexane:ethyl acetate = 6:1) to obtain the objective product (4.5 g).

### Reference Example 34

### 1-{5-[2-(2-hydroxyethyl)-2H-tetrazol-5-yl]pyridin-2-yl}piperazine

### Step 1

2-[(1-benzyloxycarbonyl)piperazin-4-yl]pyridine-5-boric acid (5.4 g) and 2-benzyloxymethyl-5-bromo-2H-tetrazole (3.88 g) were dissolved in a solvent (toluene:water:ethanol = 8:1:1) (310 ml) and tetrakistriphenylphosophine palladium (0.72 g) and sodium carbonate (3.36 g) were added, followed by stirring at 90°C for 5 hours. After returning the reaction solution to room temperature, the solvent was distilled off and the residue was mixed with water, extracted with chloroform, dried and then concentrated. The residue was purified by silica gel column chromatography (chloroform) to obtain 1-benzyloxycarbonyl-4-{5-[2-(2-benzyloxymethyl)-2H-tetrazol-5-yl]pyridin-2-yl}piperazine as a white powder (4.8 g).

### Step 2

1-benzyloxycarbonyl-4-{5-[2-(2-benzyloxymethyl)-2H-tetrazol-5-yl]pyridin-2-yl}piperazine (4.8 g) was dissolved in methanol (150 ml) and 10% hydrochloric acid (150 ml) was added, followed by stirring at 65°C for 9 hours. After the reaction solution was ice-cooled, the precipitate was collected by filtration, washed with water and then dried to obtain 1-benzyloxycarbonyl-4-[5-(2H-tetrazol-5-yl)pyridin-2-yl]piperazine as a white powder (3 g).

### Step 3

1-benzyloxycarbonyl-4-[5-(2H-tetrazol-5-yl)pyridin-2-yl]piperazine (3 g) was dissolved in dimethylformamide (40 ml) and potassium carbonate (6.2 g) and 2-bromoethanol (2.8 g) were added, followed by stirring at 100°C for 3 hours. After returning to room temperature, the reaction solution was mixed with water, extracted with chloroform, dried and then concentrated. The resulting crude crystal was crystallized from chloroform/ether to obtain 1-benzyloxycarbonyl-4-{5-[2-(2-hydroxyethyl)-2H-tetrazol-5-yl]pyridin-2-yl}piperazine as a pale yellow powder (2.44 g).

### Step 4

1-benzyloxycarbonyl-4-{5-[2-(2-hydroxyethyl)-2H-tetrazol-5-yl]pyridin-2-yl}piperazine (2.44 g) was dissolved in a solvent (methanol:chloroform = 2:1) and 10% palladium-carbon (3.5 g) was added, and then the mixture was subjected to catalytic hydrogenation (5 kgf/cm²) at 50°C for 4 days. The reaction solution was filtered and then concentrated to obtain the objective product (1.5 g).

### Reference Example 35

### 1-methyl-1H-pyrrolo[2,3-b]pyridine

To dimethylformamide (50 ml), sodium hydride (60% dispersion in mineral oil) (5.1 g) was added under ice cooling, followed by stirring. To this was gradually added dropwise a solution of 7-azwindole (10 g) in dimethylformamide (50 ml). After stirring at the same temperature for 30 minutes, methyl iodide (6.3 ml) was added dropwise, followed by stirring for another 20 minutes. After the reaction solution was mixed with ice water, extracted with ethyl acetate, the organic layer was washed with water and saturated brine, dried and then concentrated. The resulting residue was purified by silica gel column chromatography (n-hexane: ethyl acetate = 2:1) to obtain a yellow oily product (10.26 g).

### Reference Example 36

### 1-methyl-1H-pyrrolo[2,3-b]pyridine-3-boric acid

To a solution of 1-methyl-1H-pyrrolo[2,3-b] pyridine (5.1 g) in anhydrous tetrahydrofuran (50 ml), t-butyl lithium (1.6M, n-hexane solution) (36 ml) was added dropwise at -78°C for 15 minutes. After stirring for 45 minutes, a solution of boric acid triisopropyl (17.8 ml)in tetrahydrofuran (36 ml) was gradually added dropwise. After reacting at the same temperature for 30 minutes and at room temperature for one hour, water (100 ml) was added, followed by stirring for 40 minutes. After tetrahydrofuran was distilled off, the resulting aqueous layer was washed with diethyl ether. After the pH of the aqueous layer was adjusted to 6 to 7, the deposited crystal was collected by filtration and dried. The objective product (5.43 g) was obtained as a white crystal.

### Reference Example 37

### 2-(4-fluorophenyl)-1-methyl-1H-pyrrolo[2,3-b]pyridine

1-methyl-1H-pyrrolo[2,3-b]pyridine-3-boric acid (3 g) was dissolved in toluene-ethanol (2:1) (60 ml) and 1-bromo-4-fluorobenzene (4.5 g), an aqueous 2M sodium hydrogen carbonate solution (26ml) and lithium chloride (2.2 g) were added, followed by deaeration and further replacement by argon. To the solution, tetrakis(triphenylphosphine)palladium (0) (591 mg) was added, followed by replacement by argon and stirring with heating at 80°C for 17 hours. The reaction solution was cooled and filtered through celite, and then the filtrate was concentrated. After adding water to the residue, the solution was extracted with ethyl acetate and the organic layer was washed with water and saturated brine, dried and then concentrated. The resulting residue was purified by silica gel column chromatography (n-hexane:ethyl acetate = 5:1) to obtain the objective product (2.34 g).
In the same manner as in Reference Examples 35 to 37, 2-(3,4-dimethoxyphenyl)-1-methyl-1H-pyrrolo[2,3-b]pyridine, 2-(4-pyridyl)-1-methyl-1H-pyrrolo[2,3-b]pyridine, 2-(3,4-methylenedioxyphenyl)-1-methyl-1H-pyrrolo[2,3-b]pyridine, and 2-(4-carbomethoxyphenyl)-1-methyl-1H-pyrrolo[2,3-b]pyridine were synthesized.
In the same manner as in Reference Example 10, 2-(4-carbomethoxyphenyl)-1-methyl-1H-pyrrolo[2,3-b]pyridine-carbaldehyde was synthesized.
In the same manner as in Reference Examples 10 to 12, 3-[2-(4-fluorophenyl)-1-methyl-1H-pyrrolo[2,3-b]pyridin-3-yl]acrylic acid, 3-[2-(3,4-dimethoxyphenyl)-1-methyl-1H-pyrrolo[2,3-b]pyridin-3-yl]acrylic acid, 3-[2-(4-pyridyl)-1-methyl-1H-pyrrolo[2,3-b]pyridin-3-yl]acrylic acid, and 3-[2-(3,4-methylenedioxyphenyl)-1-methyl-1H-pyrrolo[2,3-b]pyridin-3-yl]acrylic acid were synthesized.

### Reference Example 38

### 2-phenyl-1H-pyrrolo[2,3-c]pyridine

### Step 1

Under an argon atmosphere, 3-aminopyridine (4.32 g) was dissolved in tetrahydrofuran (50 ml) and a solution of 1.0M sodium hexamethyldisilazidein tetrahydrofuran (100 ml) was added dropwise at room temperature with stirring over 15 minutes. After stirring for 10 minutes, a solution of di-t-butoxy dicarbonate (12.0 g) in tetrahydrofuran (20 ml) was added dropwise over 10 minutes. After stirring for 3 hours, the reaction solution was extracted with ethyl acetate, washed with saturated brine, dried over anhydrous magnesium sulfate, and then concentrated under reduced pressure. The residue was purified by silica gel column chromatography (chloroform:methanol = 20:1) to obtain 3-(t-butoxycarbonylamino)pyridine (5.47 g)

### Step 2

Under an argon atmosphere, 3-(t-butoxycarbonylamino)pyridine (2.40 g) was dissolved in tetrahydrofuran (60 ml) and a solution of 1.58M n-butyl lithium in tetrahydrofuran (20 ml) was added dropwise with stirring at -78°C. The mixture was stirred at the same temperature for one hour and then stirred at 0°C for 2 hours. After cooling to - 78°C again, methyl iodide (2.1 g) was added dropwise, followed by stirring for one hour. After returning to room temperature and stirring for one hour, the reaction solution was mixed with water, extracted with ethyl acetate, washed with saturated brine, dried over anhydrous magnesium sulfate and then concentrated under reduced pressure. The residue was purified by silica gel column chromatography (n-hexane:ethyl acetate = 1:1) to obtain 3-(t-butoxycarbonylamino)-4-methylpyridine (0.85 g).

### Step 3

Under an argon atmosphere, 3-(t-butoxycarbonylamino)-4-methylpyridine (1.50 g) was dissolved in tetrahydrofuran (40 ml) and a solution of 1. 58M n-butyl lithiumin tetrahydrofuran (10 ml) was added dropwise with stirring at -78°C. The mixture was stirred at the same temperature for 30 minutes and then stirred at -20°C for 30 minutes. After cooling to -78°C again, a solution of N-methoxy-N-methylbenzamide (1.78 g) in tetrahydrofuran (10 ml) was added dropwise and the mixture was stirred until the inner temperature reaches room temperature. The reaction solution was mixed with water, extracted with ethyl acetate, washed with saturated brine, dried over anhydrous magnesium sulfate and then concentrated under reduced pressure. The residue was purified by silica gel column chromatography (n-hexane:ethyl acetate = 1:1). The residue was dissolved in tetrahydrofuran (25 ml) and 5.5M hydrochloric acid (6 ml) was added, followed by stirring at 50°C for 2 hours. The reaction solution was alkalified with aqueous 10% sodium hydroxide solution, extracted with ethyl acetate, washed with saturated brine, dried over anhydrous magnesium sulfate and then concentrated under reduced pressure. 2-phenyl-1H-pyrrolo[2,3-c]pyridine (0.8 g) was obtained.

### Reference Example 39

### 2-phenyl-1H-pyrrolo[2,3-c]pyridine-3-carbaldehyde

Under an argon atmosphere, 2-phenyl-1H-pyrrolo[2,3-c]pyridine (0.4 g) was added to a mixed solvent of nitromethane (15 ml) and 1,2-dichloroethane (15 ml), followed by stirring at 0°C. To the suspension, dichloromethyl methyl ether (1.18 g) and aluminum chloride (0. 96 g) were added, followed by stirring. The suspension was converted into a uniform solution, followed by stirring for 20 minutes. The same amount of dichloromethyl methyl ether and aluminum chloride were added, followed by stirring for one hour. The reaction solution was alkalified by adding an aqueous 10% sodium hydroxide solution and then extracted with chloroform. The extract was washed with saturated brine, dried over anhydrous magnesium sulfate and then concentrated under reduced pressure. The residue was purified by silica gel column chromatography (chloroform:methanol = 20:1) to obtain the objective product (0.35 g).

### Reference Example 40

### 1-methyl-2-phenyl-1H-pyrrolo[2,3-c]pyridine-3-carbaldehyde

2-phenyl-1H-pyrrolo[2,3-c]pyridine-3-carbaldehyde (0.35 g) was dissolved in dimethylformamide (10 ml) and sodium hydride(60% dispersion in mineral oil) (100 mg) was added under ice cooling, followed by stirring for 2 hours. Then, methyl iodide (0.35 g) was added dropwise, followed by stirring at room temperature for 20 minutes. After adding ice water, the reaction solution was extracted with ethyl acetate, washed with saturated brine, died over anhydrous magnesium sulfate and then concentrated under reduced pressure. The resulting crude product was purified by NH silica gel column chromatography (hexane: ethyl acetate = 2:1) to obtain the objective product (300 mg).

In the same manner as in Reference Examples 11 to 12, 3-(1-methyl-2-phenyl-1H-pyrrolo[2,3-c]pyridin-3-yl)acrylic acid was synthesized.

### Reference Example 41

### 2-phenyl-1H-pyrrolo[3,2-b]pyridine

### Step 1

2-chloro-3-aminopyridine (5 g) was dissolved in triethylamine (50 ml), followed by deaeration-replacement by argon, addition of bis(triphenylphosphine)palladium (II) chloride (55 mg) and copper iodide (I) (150 mg) and further deaeration-replacement by argon. Under ice cooling, ethynylbenzene (6.3 ml) was slowly added dropwise over 30 minutes. After the completion of dropwise addition, the mixture was stirred with heating in an oil bath heated previously to 80°C for 12.5 hours. After cooling to room temperature, the reaction solution was filtered through celite and then concentrated. The resulting residue was purified by silica gel column chromatography (n-hexane:ethyl acetate = 4:1) to obtain 2-(2-phenylethynyl)-3-aminopyridine (1.54 g) as a brown powder.

### Step 2

2-(2-phenylethynyl)-3-aminopyridine (1.54 g) was dissolved in dehydrated dimethylformamide (140 ml), followed by deaeration-replacement by argon, addition of copper iodide (I) (0.76 g) and further deaeration-replacement by argon. The mixture was stirred with heating in an oil bath heated previously to 110°C for 20 hours. After cooling to room temperature, 2/3 of the solvent was removed and the mixture was partitioned with ethyl acetate□saturated brine, and then the organic layer was dried over anhydrous magnesium sulfate and concentrated. The resulting residue was purified by silica gel column chromatography (n-hexane:ethyl acetate = 1:1) to obtain 2-phenyl-1H-pyrrolo[3,2-b]pyridine (1.12 g) as a white powder.

In the same manner as in Reference Examples 9 to 12, 3-(1-methyl-2-phenyl-1H-pyrrolo[3,2-b]pyridin-3-yl)acrylic acid was synthesized.

### Reference Example 42

### 5-methyl-2-phenyl-1H-pyrrolo[2,3-b]pyridine

### Step 1

Under an argon atmosphere, 2-amino-3-bromo-5-methylpyridine (3.0 g) was dissolved in triethylamine (50 ml) and ethynyl benzene (3.27 g) was added. Then, bis(triphenylphosphine)palladium (II) chloride (1.12 g) and copper iodide (I) (0.31 g) were added, followed by stirring with heating at 70°C for 3 hours. The reaction solution was mixed with chloroform and then filtered through celite to remove insolubles. The organic layer was washed with water, dried over anhydrous magnesium sulfate and then concentrated under reduced pressure. The residue was purified by silica gel columnchromatography (n-hexane:ethyl acetate = 4:1 to 1:1) to obtain 2-amino-3-phenylethynyl-5-methylpyridine (3.0 g).

### Step 2

Under an argon atmosphere, potassium hydride (4.5 g) were suspended in N-methylpiperidone (100 ml) and a solution of 2-amino-3-phenylethynyl-5-methylpyridine (3.4 g) in N-methylpiperidone (60 ml) was added with stirring at room temperature, followed by stirring for 12 hours. After ice cooling, a small amount of ice was added to the reaction mixture and the reaction was completed by adding ice water, and then the reaction mixture was extracted with ethyl acetate. Since a precipitate was produced during a partitioning operation, the precipitate was collected and dissolved in chloroform to obtain a filtrate. The organic layer was washed with water and then washed with saturated brine. The entire organic layers were combined, dried over anhydrous magnesium sulfate and then concentrated under reduced pressure. Ether was added to the resulting residue and the resulting deposit was collected by filtration. 5-methyl-2-phenyl-1H-pyrrolo[2,3-b]pyridine (2.30 g) was obtained.

In the same manner as in Reference Examples 9, 24, 16 and 17, 6-{4-[(1,5-dimethyl-2-phenyl-1H-pyrrolo[2,3-b]pyridin-3-yl)carbonyl]piperazin-1-yl}nicotinic acid was synthesized. In the same manner as in Reference Examples 9 to 12, 3-(1,5-dimethyl-2-phenyl-1H-pyrrolo[2,3-b]pyridin-3-yl)acrylic acid was synthesized.

### Reference Example 43

### 5-fluoro-2-phenyl-1H-indole

### Step 1

To a solution of 5-fluoro-2-nitrotoluene (10.0 g) in anhydrous methanol (50 ml), 10% palladium-carbon (1.0 g) was added, followed by stirring under normal pressure for 7 hours. Furthermore, 10% palladium-carbon (1.5 g) was added and the mixture was stirred under pressure of 2.5 atom overnight. The palladium-carbon was removed by filtration and the solution was concentrated under reduced pressure. 4-fluoro-2-methylaniline was obtained as a reddish violet oily product (7.24 g).

### Step 2

### N-(4-fluoro-2-methylphenyl)benzamide

To a solution of 4-fluoro-2-methylaniline (5.0 g) in anhydrous methylene chloride (50 ml), benzoyl chloride (4.6 ml) was added dropwise under ice cooling, over 10 minutes, followed by stirring at room temperature for 3.5 hours. The reaction solution was mixed with an aqueous 1N sodium hydroxide solution under ice cooling and then diluted with ethyl acetate (200 ml). The aqueous layer was extracted with ethyl acetate (200 ml) and the combined organic layer was washed in turn with 1N hydrochloric acid (100 ml), water (100 ml) and saturated brine (100 ml), dried and then concentrated. The resulting crude product was recrystallized from ethyl acetate to obtain N-(4-fluoro-2-methylphenyl)benzamide as a white needle crystal (7. 1g).

### Step 3

To a suspension of N-(4-fluoro-2-methylphenyl)benzamide (5.0 g) in anhydrous tetrahydrofuran (40 ml), n-butyl lithium (1.58M hexane solution) (27.5 ml) was added dropwise at -25°C over 20 minutes, followed by stirring at -78°C for one hour and further stirring at room temperature overnight. The reaction solution was mixed with 2N hydrochloric acid (200 ml) under ice cooling and then diluted with ethyl acetate (100 ml). The aqueous layer was extracted with ethyl acetate (150 ml × 2) and the combined organic layer was washed in turn with saturated sodium bicarbonate water (50 ml), water (50 ml) and saturated brine (50 ml), dried and then concentrated. The resulting residue was purified by silica gel column chromatography (n-hexane:ethyl acetate = 10:1 → 5:1 → 2:1) to obtain 5-fluoro-2-phenyl-1H-indole as a white crystal (814 mg).

In the same manner as in Reference Examples 9, 24, 16 and 17, 6-{4-[(5-fluoro-1-methyl-2-phenyl-1H-indol-3-yl)carbonyl]piperazin-1-yl}nicotinic acid was synthesized.

### Reference Example 44

### 5-bromo-2-phenyl-1H-indole

### Step 1

To a solution of 4-bromo-2-iodoaniline (3.0 g) in anhydrous pyridine (100 ml), ethyl chlorocarbonate (1.02 ml) was added dropwise under ice cooling, followed by stirring for 40 minutes. To the reaction solution, water (100 ml) was added and the resulting white crystal was collected by filtration to obtain ethyl (4-bromo-2-iodophenyl)carbamate (3.01 g). The resulting objective product was used for the following reaction as it is.

### Step 2

To a solution of ethyl (4-bromo-2-iodophenyl) carbamate (2.5 g) in triethylamine (20 ml), ethynylbenzene (1.1 ml), bis(triphenylphosphine)palladium (II) chloride (475 mg) and copper iodide (I) (128.9 mg) were added, followed by stirring under an argon atmosphere at room temperature for one hour and further stirring at 50°C overnight. The reaction solution was diluted with ethyl acetate (30 ml), filtered through celite and then mixed with water (50 ml) and saturated brine (50 ml). The aqueous layer was extracted with ethyl acetate (200 ml × 2) and the combined the organic layer was washed with saturated brine (50 ml), dried and then concentrated. The resulting residue was purified by silica gel column chromatography (n-hexane: ethyl acetate = 30: 1) to obtain ethyl (4-bromo-2-phenylethynyl-phenyl)carbamate as a pale yellow crystal (775 mg).

### Step 3

To a solution of ethyl (4-bromo-2-phenylethynyl-phenyl) carbamate (765 mg) in tetrahydrofuran (15 ml), tetrabutylammonium fluoride (5.5 ml) (1.0M tetrahydrofuran solution) was added and the mixture was heated at reflux for 17 hours. After the reaction solution was concentrated under reduced pressure and diluted with ethyl acetate (30 ml) and water (20 ml), the aqueous layer was extracted with ethyl acetate (30 ml × 2) and the combined organic layer was washed with saturated brine (10 ml), dried and then concentrated. The resulting residue was purified by silica gel column chromatography (n-hexane: ethyl acetate = 5:1) to obtain 5-bromo-2-phenyl-1H-indole as a yellow crystal (590 mg).

In the same manner as in Reference Examples 9, 24, 16 and 17, 6-{4-[(5-bromo-1-methyl-2-phenyl-1H-indol-3-yl)carbonyl]piperazin-1-yl}nicotinic acid was synthesized.

### Reference Example 45

### 1-methoxymethyl-2-phenyl-1H-pyrrolo[2,3-b]pyridine

sodium hydride (60% dispersion in mineral oil) (148 mg) was washed three times with n-hexane under an argon atmosphere and then dehydrated dimethylformamide (5.0 ml) was added under ice-cooling. To the resulting suspension, a solution of 2-phenyl-1H-pyrrolo[2,3-b]pyridine (550 mg) in dehydrated dimethylformamide (20 ml) was added dropwise over 30 minutes, followed by stirring at the same temperature for one hour. A solution of chloromethyl methyl ether (148 ml) in dimethylformamide (5.0 ml) was added dropwise over 30 minutes and the mixture was stirred under ice-cooling for one hour and then stirred at room temperature for 30 minutes. The reaction solution was mixed with water under ice-cooling and then extracted with ethyl acetate. The organic layer was washed with saturated brine, dried over anhydrous sodium sulfate and then concentrated. The residue was purified by silica gel column chromatography (n-hexane: ethyl acetate = 2:1) to obtain the objective product (529 mg) as a white amorphous compound.

In the same manner as in Reference Examples 10 to 12, 3-(1-methoxymethyl-2-phenyl-1H-pyrrolo[2,3-b]pyridin-3-yl)acrylic acid was synthesized.

### Reference Example 46

### 2-(4-bromophenyl)-1,5-dimethyl-1H-indole

p-tolylhydrazine hydrochloride (4.0 g) and 4-bromoacetophenone (5.0 g) were added to acetic acid (100 ml), followed by stirring at 100°C for 8 hours. The mixture was concentrated under reduced pressure and the deposited crystal was collected by filtration. The crystal was added to 116% polyphosphoric acid (30 g) and then stirred at 120°C for 2 hours. After the reaction solution was poured into ice water and extracted with ethyl acetate (200 ml), the organic layer was washed with saturated brine (100 ml), dried over anhydrous sodium sulfate and then concentrated under reduced pressure. The deposited crystal was collected by filtration. Under an argon gas flow, sodium hydride (60% dispersion in mineral oil) (460 mg) was suspended in anhydrous dimethylformamide (30 ml). A solution of the above crystal in anhydrous dimethylformamide (10ml) was added dropwise over 10 minutes, followed by stirring for another one hour. To the mixture was added methyl iodide (0. 90 ml) , followed by stirring for 15 hours. After water (10 ml) was added and saturated brine (50 ml) was added and extracted with ethyl acetate (200 ml) , the organic layer was dried over anhydrous sodium sulfate. After concentration under reduced pressure, the residue was purified by silica gel column chromatography (n-hexane:ethyl acetate = 10:1) to obtain the objective product (2.2 g) as a yellow powder.

### Reference Example 47

### Methyl 4-(1,5-dimethyl-1H-indol-2-yl)benzoate

Under an argon atmosphere, 2-(4-bromophenyl)-1,5-dimethyl-1H-indole (2.2 g) was dissolved in dimethylformamide (40 ml) and 1,1'-bis(diphenylphosphino) ferrocene (960 mg), triethylamine (2.8 ml), methanol (5.3 ml) and palladium(II)acetate(300 mg) were added. The argon atmosphere in the reaction vessel was replaced by carbon monoxide, followed by stirring at 60°C for 15 hours. After cooling to room temperature, water (40 ml) was added and the objective product was extracted with ethyl acetate (200 ml). The organic layer was washed with saturated brine (30 ml), dried over anhydrous sodium sulfate and then concentrated under reduced pressure. The residue was purified by silica gel column chromatography (n-hexane: ethyl acetate = 10:1 → 5:1) to obtain the objective product (1.7 g) as a pale yellow powder.

### Reference Example 48

### Allyl (2E)-3-[1,5-dimethyl-2-(4-carbomethoxyphenyl)-1H-indol-3-yl]acrylate

Under an argon atmosphere, methyl 4-(1,5-dimethyl-1H-indol-2-yl)benzoate (780 mg) was dissolved in anhydrous dimethylformamide (10 ml) and phosphoryl chloride (0.27 ml) was added under ice cooling. After returning to room temperature and stirring for 4 hours, the reaction solution was poured into ice-cooled distilled water (20ml) and extracted with chloroform (100 ml). The organic layer was washed with saturated brine (30 ml), dried over anhydrous sodium sulfate and then concentrated under reduced pressure. Under an argon atmosphere, sodium hydride (60% dispersion in mineral oil) (230 mg) was suspended in anhydrous dimethylformamide (10 ml) and allyl diethylphosphinoacetate (1.2 ml) was added, followed by stirring at room temperature for 30 minutes. To the mixture, a solution prepared by dissolving the residue obtained after concentration in anhydrous dimethylformamide (20 ml) was added dropwise for 15 minutes, followed by stirring for 2 hours, heating to 100°C and further stirring for another 15 hours. After water (20 ml) was added and the solution was extracted with chloroform (100 ml), the resulting organic layer was dried over anhydrous sodium sulfate and then concentrated under reduced pressure. The residue was purified by silica gel column chromatography (n-hexane: ethyl acetate = 10:1) to obtain the objective product (580 mg) as a pale yellow powder.

### Reference Example 49

### (2E)-3-[1,5-dimethyl-2-(4-carbomethoxyphenyl)-1H-indol-3-yl]acrylic acid

Under an argon atmosphere, allyl (2E)-3-[1,5-dimethyl-2-(4-carbomethoxyphenyl)-1H-indol-3-yl]acrylate (580 mg) was dissolved in acetonitrile (10 ml) and morpholine (0.65 ml) and tetrakis(triphenylphosphine)palladium (0) (50 mg) were added, followed by stirring for 15 hours. The residue obtained after concentration under reduced pressure was dissolved in chloroform (100 ml) and the solution was washed with 10% hydrochloric acid (10 ml) and saturated brine (10 ml). The organic layer was dried over anhydrous sodium sulfate and then concentrated under reduced pressure. The deposited crystal was collected by filtration to obtain the objective product (430 mg) as a pale yellow powder.

### Reference Example 50

### 1-[2-(trimethylsilyl)ethoxy]methyl-2-phenyl-1H-pyrrolo[2,3-b]pyridine

To a solution of 2-phenyl-1H-pyrrolo[2, 3-b]pyridine (2. 0 g) in dimethylformamide (25 ml), sodium hydride(60% dispersion in mineral oil) (600 mg) was added under ice cooling. After stirring for one hour under ice cooling, [2-(trimethylsilyl)ethoxy]methyl chloride (1.84 g) was added dropwise, followed by stirring under ice cooling for 20 minutes. After the reaction solution was mixed with water and extracted with ethyl acetate, the organic layer was washed twice with water, washed once with saturated brine, dried and then concentrated. The resulting residue was purified by silica gel column chromatography (hexane:ethyl acetate = 1:1) to obtain a colorless oily product (2.84 g).

In the same manner as in Examples 10 to 12, 3-{1-[2-(trimethylsilyl)ethoxy]methyl-2-phenyl-1H-pyrrolo[2,3-b]pyridin-3-yl}acrylic acid was synthesized.

### Reference Example 51

### Methyl 6,7-dimethoxy-1,2,3,4-tetrahydro-1-isoquinolineacetate hydrochloride

Under ice cooling, thionyl chloride (10 ml) was added dropwise to methanol (25 ml), followed by stirring for 30 minutes. To the solution, 6,7-dimethoxy-1,2,3,4-tetrahydro-1-isoquinolineacetic acid monohydrate (1.42 g) was added and the mixture was heated at reflux for 3 hours. The reaction solution was concentrated under reduced pressure and diethyl ether and methanol were added to the residue. The resulting crystal was collected by filtration, washed with diethyl ether and then dried. A white crystal (1.55 g) was obtained.

### Reference Example 52

### 3-[2-(4-carbomethoxyphenyl)-1-methyl-1H-pyrrolo[2,3-b]pyridin-3-yl]acrylic acid

A solution of 2-(4-carbomethoxyphenyl)-1-methyl-1H-pyrrolo[2,3-b]pyridine-3-carbaldehyde (170 mg), malonic acid (120 mg) and piperidine (0.69 ml) in pyridine (4 ml) were stirred at 80 °C for 45 minutes and then stirred at 120 °C for another one hour. The reaction solution was concentrated under reduced pressure and chloroform was added to the residue. The mixture was washed with 10% hydrochloric acid, water and saturated brine, dried and then concentrated to obtain the objective product (120 mg)

### Example 1

### 6-{4-[(1,5-dimethyl-2-phenyl-1H-indol-3-yl)carbonyl]piperazin-1-yl}-N-(2-hydroxyethyl)nicotinamide

6-{4-[(1,5-dimethyl-2-phenyl-1H-indol-3-yl)carbonyl]piperazin-1-yl}nicotinic acid (20 g) was dissolved in dimethylformamide (100 ml) and WSCD·HCl (12.7 g), 1-hydroxybenzotriazole (8.9 g) and triethylamine (13.4 g) were added, followed by stirring for 30 minutes. Then, 2-hydroxyethylamine 5.4 g was added and the mixture was stirred at room temperature overnight. The reaction solution was poured into water, extracted with ethyl acetate, washed several times with water, dried and then concentrated. The resulting residue was washed with chloroform-ether to obtain the objective product (20.1 g).

| Elemental analysis for (C₂₉H₃₁N₅O₃) | | | |
|---|---|---|---|
| Calcd.(%) | C,70.00; | H,6.28; | N,14.07 |
| Found (%) | C,69.87; | H,6.37; | N,13.96 |

### Example 2

### (2E)-N-(4-methoxybenzyl)-3-(1-methyl-2-phenyl-1H-indol-3-yl)acrylamide

(2E)-3-(1-methyl-2-phenyl-1H-indol-3-yl)acrylic acid (17.5 g) was dissolved in dimethylformamide (200 ml) and WSCD· HCl (13.3 g), 1-hydroxybenzotriazole (9.4 g) and triethylamine (7.0 g) were added, followed by stirring for 30 minutes. Then, 4-methoxybenzylamine (8.66 g) was added and the mixture was stirred at room temperature overnight. The reaction solution was poured into water, extracted with ethyl acetate, washed several times with water, dried and then concentrated.. The residue was purified by silica gel column chromatography (chloroform) and the resulting concentrate was washed with chloroform-ether to obtain a white powder (20.2 g).

| Elemental analysis for (C₂₆H₂₄N₂O₂) | | | |
|---|---|---|---|
| Calcd.(%) | C,78.76; | H,6.10; | N,7.07 |
| Found (%) | C,78.92; | H,6.22; | N,7.04 |

### Example 3

### 6,7-dimethoxy-1-methyl-2-[(2E)-3-(1-methyl-2-phenyl-1H-pyrrolo[2,3-b]pyridin-3-yl)prop-2-enoyl]-1,2,3,4-tetrahydroisoquinoline hydrochloride

Under an argon atmosphere, to 3-(1-methyl-2-phenyl-1H-pyrrolo[2,3-b]pyridin-3-yl)-acrylic acid (9.74 g), WSCD·HCl (0.95 g) and 1-hydroxybenzotriazole (7.1 g), dimethylformamide (20 ml) was added, followed by stirring until a uniform mixture is obtained. Then, triethylamine (7.1 g) was added and the mixture was stirred for 30 minutes. Furthermore, salsolidine hydrochloride (9.75 g) was added, followed by stirring at room temperature for 15 hours. After the reaction solution was poured into water, the deposited white powder was collected by filtration, washed with water, dissolved in chloroform, washed with saturated brine, dried and then concentrated. The residue was purified by silica gel column chromatography (n-hexane: ethyl acetate = 2:1) and the resulting product was dissolved in dichloroethane (100 ml). Under an argon atmosphere, a 4.0M HCl/ethyl acetate solution (10 ml) was added during stirring with ice cooling. After ether was added, the deposit was collected by filtration and then dried under reduced pressure at 40°C for 10 hours to obtain the objective product (15.7 g).

| Elemental analysis for (C₂₉H₂₉N₃O₃·HCl·1.5H₂O) | | | |
|---|---|---|---|
| Calcd.(%) | C,65.59; | H,6.26; | N,7.91 |
| Found (%) | C,65.96; | H,6.00; | N,7.92 |

Positive ion ESI-MS m/z : 468[M+H]⁺

In the same manner as in Example 3, the following compound of Example 4 was synthesized.

### Example 4

### (2E)-N-[(1R,2S)-2-methoxy-2,3-dihydro-1H-inden-1-yl]-3-(1-methyl-2-phenyl-1H-pyrrolo[2,3-b]pyridin-3-yl)acrylamide hydrochloride

| Elemental analysis for (C₂₇H₂₅N₃O₂·HCl·H₂O) | | | |
|---|---|---|---|
| Calcd.(%) | C,67.98; | H,6.07; | N,8.81 |
| Found (%) | C,67.84; | H,5.90; | N,8.79 |

Positive ion FAB-MS m/z: 424[M+H]⁺

### Example 5

### 5-{4-[(1,5-dimethyl-2-phenyl-1H-indol-3-yl)carbonyl]piperazin-1-yl}-N-[2-(4-methyl-1,3-thiazol-5-yl)ethyl]pyrazine-2-carboxamide trihydrochloride

Under an argon atmosphere, to 5-{4-[(1,5-dimethyl-2-phenyl-1H-indol-3-yl)carbonyl]piperazin-1-yl}pyrazine-2-carboxylic acid (1.5 g), WSCD·HCl (0.95 g) and hydroxybenzotriazole (0.67 g), dimethylformamide (10 ml) was added, followed by stirring until a uniform mixture is obtained. Then, triethylamine (0.50 g) was added and the mixture was stirred for 30 minutes. Then, 2-(4-methylthiazol-5-yl)ethylamine (0.56 g) was added, followed by stirring at room temperature for 15 hours. After the reaction solution was poured into water, the deposited white powder was collected by filtration, washed with water, dissolved in chloroform, washed with saturated brine, dried and then concentrated. The resulting product was dissolved in dichloroethane (100 ml). Under an argon atmosphere, a 1.0M HCl/diethyl ether solution (10 ml) was added during stirring with ice cooling and the deposited powder was collected by filtration and then dried under reduced pressure at 40°C for 10 hours to obtain the objective product (2.0 g).

| Elemental analysis for (C₃₂H₃₃N₇O₂S·3HCl·5H₂O) | | | |
|---|---|---|---|
| Calcd.(%) | C,49.33; | H,5.95; | N,12.58 |
| Found (%) | C,49.88; | H,6.12; | N,12.61 |

Positive ion ESI-MS m/z: 580[M+H]⁺

In the same manner as in Example 5, the following compound of Example 6 was synthesized.

### Example 6

### 5-{4-[(1-methyl-2-phenyl-1H-pyrrolo[2,3-c]pyridin-3-yl)carbonyl]piperazin-1-yl}-N-[2-(4-methyl-1,3-thiazol-5-yl)ethyl]pyrazine-2-carboxamide dihydrochloride

| Elemental analysis for (C₃₀H₃₀N₈O₂S·2HCl·3H₂O) | | | |
|---|---|---|---|
| Calcd.(%) | C,51.95; | H,5.52; | N,16.15 |
| Found (%) | C,52.18; | H,5.52; | N,16.21 |

Positive ion ESI-MS m/z: 567[M+H]⁺

### Example 7

### 5-{4-[(1-methyl-2-phenyl-1H-pyrrolo[3,2-b]pyridin-3-yl)carbonyl]piperazin-1-yl}-N-[2-(4-methyl-1,3-thiazol-5-yl)ethyl]pyrazine-2-carboxamide

5-{4-[(1-methyl-2-phenyl-1H-pyrrolo[3,2-b]pyridin-3-yl)carbonyl]piperazin-1-yl}-2-pyrazinecarboxylic acid (0.15 g) and 2-(4-methyl-1,3-thiazol-5-yl)ethylamine (53 mg) were dissolved in dimethylformamide (5 ml) and triethylamine (75 mg), WSCD·HCl (71 mg) and 1-hydroxybenzotriazole (50 mg) were added, followed by stirring at room temperature for 3 days. The reaction solution was mixed with water, extracted with ethyl acetate, dried and then concentrated. The residue was washed with ethanol to obtain a colorless powder (0.11 g).
Melting point: 224.6°C
Positive ion ESI-MS m/z: 567[M+H]⁺

In the same manner as in Example 7, the following compound of Example 8 was synthesized.

### Example 8

### 5-{4-[(5-methyl-6-phenyl-5H-pyrrolo[2,3-b]pyrazin-7-yl)carbonyl]piperazin-1-yl}-N-[2-(4-methyl-1,3-thiazol-5-yl)ethyl]pyrazine-2-carboxamide

Positive ion FABMS m/z: 568[M+H]⁺

### Example 9

### 1,5-dimethyl-2-phenyl-3-[(4-{5-[2-(2-hydroxyethyl)-2H-tetrazol-5-yl]pyridin-2-yl}piperazin-1-yl)carbonyl]-1H-indole hydrochloride

### Step 1

Under an argon atmosphere, 1,5-dimethyl-2-phenylindole-3-carboxylic acid (1.4 g), 1-(5-[2-(2-hydroxyethyl)-2H-tetrazol-5-yl]pyridin-2-yl}piperazine (1.5 g), WSCD·HCl (1.1 g) and hydroxybenzotriazole (0.78 g) were dissolved in dimethylformamide (20 ml) and triethylamine (1.17 g) was added, followed by stirring at 60°C for 2 days. After the reaction solution was poured into water, the deposited white powder was collected by filtration, washed with water, dissolved in chloroform, washed with saturated brine, dried and then concentrated. The residue was purified by silica gel column chromatography (chloroform:methanol = 50:1) and then washed with' ether.

### Step 2

The resulting 1,5-dimethyl-2-phenyl-3-[(4-{5-[2-(2-hydroxyethyl)-2H-tetrazol-5-yl]pyridin-2-yl}piperazin-1-yl)carbonyl]-1H-indole was dissolved in chloroform (10 ml) and a 1.0M HCl in diethyl ether solution was added under ice cooling, and then the deposited powder was collected by filtration to obtain the objective product (1.29 g).
Positive ion FAB-MS m/z: 523[M+H]⁺

### Example 10

### 5-{4-[(1,5-dimethyl-2-phenyl-1H-indol-3-yl)carbonyl]piperazin-1-yl}-N-(4-pyridylmethyl)pyrazine-2-carboxamide

Under an argon atmosphere, 5-{4-[(1,5-dimethyl-2-phenyl-1H-indol-3-yl)carbonyl]piperazin-1-yl}pyrazine-2-carboxylic acid (0.2 g), 4-aminomethylpyridine (52 mg), WSCD·HCl (93 mg) and hydroxybenzotriazole (65 mg) were dissolved in dimethylformamide (5 ml) and triethylamine (98 mg) was added, followed by stirring for 15 hours. After water was added to the reaction solution, the deposited white powder was collected by filtration, washed with water, dissolved in chloroform, washed with saturated brine, dried and then concentrated. The residue was purified by silica gel column chromatography (chloroform:methanol = 19:1), dissolved in methanol and, after adding ether, the deposited powder was collected by filtration and dried to obtain the objective product (0.13 g).

| Elemental analysis for (C₃₂H₃₁N₇O₂·0.5H₂O) | | | |
|---|---|---|---|
| Calcd.(%) | C,69.30; | H,5.82; | N,17.68 |
| Found (%) | C,69.70; | H,5.87; | N,17.43 |

In the same manner as in Example 10, the following compounds of Examples 11 to 19 were synthesized.

### Example 11

### 5-{4-[(1,5-dimethyl-2-phenyl-1H-indol-3-yl)carbonyl]piperazin-1-yl}-N-[2-(4-pyridyl)ethyl]pyrazine-2-carboxamide

| Elemental analysis for (C₃₃H₃₃N₇O₂·2H₂O) | | | |
|---|---|---|---|
| Calcd.(%) | C,66.54; | H,6.26; | N,16.46 |
| Found (%) | C,66.68; | H,5.81; | N,16.48 |

### Example 12

### 5-{4-[(1,5-dimethyl-2-phenyl-1H-indol-3-yl)carbonyl]piperazin-1-yl}-N-(2-thienylmethyl)pyrazine-2-carboxamide

| Elemental analysis for (C₃₁H₃₀N₆O₂S) | | | |
|---|---|---|---|
| Calcd.(%) | C,67.37; | H,5.84; | N,15.21 |
| Found (%) | C,67.19; | H,5.55; | N,14.94 |

### Example 13

### 5-{4-[(1,5-dimethyl-2-phenyl-1H-indol-3-yl)carbonyl]piperazin-1-yl}-N-[2-(2-thienyl)ethyl]pyrazine-2-carboxamide

| Elemental analysis for (C₃₂H₃₂N₆O₂S) | | | |
|---|---|---|---|
| Calcd.(%) | C,68.06; | H,5.71; | N,14.88 |
| Found (%) | C,67.95; | H,5.88; | N,14.48 |

### Example 14

### 5-{4-[(1,5-dimethyl-2-phenyl-1H-indol-3-yl)carbonyl]piperazin-1-yl}-N-[2-(3-thienyl)ethyl]pyrazine-2-carboxamide

| Elemental analysis for (C₃₂H₃₂N₆O₂S) | | | |
|---|---|---|---|
| Calcd.(%) | C,68.06; | H,5.71; | N,14.88 |
| Found (%) | C,67.86; | H,5.78; | N,14.44 |

### Example 15

### 5-{4-[(1,5-dimethyl-2-phenyl-1H-indol-3-yl)carbonyl]piperazin-1-yl}-N-(2-furylmethyl)pyrazine-2-carboxamide

| Elemental analysis for (C₃₁H₃₀N₆O₃) | | | |
|---|---|---|---|
| Calcd.(%) | C,69.65; | H,5.66; | N,15.72 |
| Found (%) | C,69.48; | H,5.74; | N,15.42 |

### Example 16

### 5-{4-[(1,5-dimethyl-2-phenyl-1H-indol-3-yl)carbonyl]piperazin-1-yl}-N-[2-(piperidin-1-yl)ethyl]pyrazine-2-carboxamide

Positive ion ESI-MS m/z: 566[M+H]⁺

### Example 17

### 5-{4-[(1,5-dimethyl-2-phenyl-1H-indol-3-yl)carbonyl]piperazin-1-yl}-N-[2-(pyrrolidin-1-yl)ethyl]pyrazine-2-carboxamide

Positive ion ESI-MS m/z: 552[M+H]⁺

### Example 18

### 5-{4-[(1,5-dimethyl-2-phenyl-1H-indol-3-yl)carbonyl]piperazin-1-yl}-N-[2-(morpholin-4-yl)ethyl]pyrazine-2-carboxamide

| Elemental analysis for (C₃₂H₃₇N₇O₃·H₂O) : | | | |
|---|---|---|---|
| Calcd.(%) | C,65.62; | H, 6.71; | N,16.74 |
| Found (%) | C,65.45; | H,6.48; | N,16.52 |

### Example 19

### 5-{4-[(1,5-dimethyl-2-phenyl-1H-indol-3-yl)carbonyl]piperazin-1-yl}-N-[3-(morpholin-4-yl)propyl]pyrazine-2-carboxamide

| Elemental analysis for (C₃₃H₃₉N₇O₃·0.25H₂O) | | | |
|---|---|---|---|
| Calcd.(%) | C,67.61; | H,6.79; | N,16.73 |
| Found (%) | C,67.66; | H,6.79; | N,16.54 |

In the same manner as in Example 5, the following compounds of Examples 20 to 25 were synthesized.

### Example 20

### 4-[(1,5-dimethyl-2-phenyl-1H-indol-3-yl)carbonyl]-1-(5-[(thiomorpholin-4-yl)carbonyl]pyridin-2-yl}piperazine hydrochloride

| Elemental analysis for (C₃₁H₃₃N₅O₂S·HCl·1.5H₂O) | | | |
|---|---|---|---|
| Calcd.(%) | C,61.73; | H,6.18; | N,11.61 |
| Found (%) | C,62.01; | H,6.07; | N,11.64 |

Positive ion ESI-MS m/z: 540[M+H]⁺

### Example 21

### 6-{4-[(1,5-dimethyl-2-phenyl-1H-indol-3-yl)carbonyl]piperazin-1-yl}-N-(2-thienylmethyl)nicotinamide hydrochloride

| Elemental analysis for (C₃₂H₃₁N₅O₂S·HCl·1H₂O) | | | |
|---|---|---|---|
| Calcd.(%) | C,63.62; | H,5.67; | N,11.59 |
| Found (%) | C,63.63; | H,5.77; | N,11.33 |

Positive ion ESI-MS m/z: 550[M+H]⁺

### Example 22

### 6-{4-[(1,5-dimethyl-2-phenyl-1H-indol-3-yl)carbonyl]piperazin-1-yl}-N-(2-methoxy-1-methylethyl)nicotinamide hydrochloride

| Elemental analysis for (C₃₁H₃₅N₅O₃·HCl·1H₂O) | | | |
|---|---|---|---|
| Calcd.(%) | C,64.18; | H,6.60; | N,12.07 |
| Found (%) | C,63.96; | H,6.48; | N,12.28 |

Positive ion ESI-MS m/z: 526[M+H]⁺

### Example 23

### 6-{4-[(1,5-dimethyl-2-phenyl-1H-indol-3-yl)carbonyl]piperazin-1-yl}-N-ethyl-N-(4-pyridylmethyl)nicotinamide dihydrochloride

| Elemental analysis for (C₃₅H₃₆N₆O₂·2HCl·2.5H₂O) | | | |
|---|---|---|---|
| Calcd.(%) | C,60.87; | H,6.28; | N,12.17 |
| Found (%) | C,61.14; | H,6.44; | N,12.38 |

Positive ion ESI-MS m/z: 573[M+H]⁺

### Example 24

### 6-{4-[(1,5-dimethyl-2-phenyl-1H-indol-3-yl)carbonyl]piperazin-1-yl}-N-[2-(4-methyl-1,3-thiazol-5-yl)ethyl]nicotinamide dihydrochloride

| Elemental analysis for (C₃₃H₃₄N₆O₂S·2HCl·2.5H₂O) | | | |
|---|---|---|---|
| Calcd.(%) | C,56.89; | H,5.93; | N,12.06 |
| Found (%) | C,56.90; | H,5.85; | N,11.90 |

Positive ion ESI-MS m/z: 579[M+H]⁺

### Example 25

### 6-{4-[(1,5-dimethyl-2-phenyl-1H-indol-3-yl)carbonyl]piperazin-1-yl}-N-[(5-methylpyrazin-2-yl)methyl]nicotinamide dihydrochloride

| Elemental analysis for (C₃₃H₃₃N₇O₂·2HCl·2H₂O) | | | |
|---|---|---|---|
| Calcd.(%) | C,59.28; | H,5.88; | N,14.66 |
| Found (%) | C,59.58; | H,5.94; | N,14.78 |

Positive ion ESI-MS m/z: 560[M+H]⁺

In the same manner as in Example 9, the following compound of Example 26 was synthesized.

### Example 26

### 1,5-dimethyl-2-phenyl-3-[{4-[5-(2-methyl-2H-tetrazol-5-yl)pyridin-2-yl]piperazin-1-yl}carbonyl]-1H-indole

| Elemental analysis for (C₂₈H₂₈N₈O·0.5H₂O) | | | |
|---|---|---|---|
| Calcd.(%) | C,67.66; | H,5.78; | N,22.54 |
| Found (%) | C,67.72; | H,5.81; | N,22.09 |

Positive ion FAB-MS m/z: 492[M]⁺

In the same manner as in Example 5, the following compound of Example 27 was synthesized.

### Example 27

### 5-{4-[(1-methyl-2-phenyl-1H-pyrrolo[2,3-b]pyridin-3-yl)carbonyl]piperazin-1-yl}-N-[2-(4-methyl-1,3-thiazol-5-yl)ethyl]pyrazine-2-carboxamide dihydrochloride

| Elemental analysis for (C₃₀H₃₀N₈O₂S·2HCl·2H₂O) | | | |
|---|---|---|---|
| Calcd.(%) | C,53.33; | H,5.37; | N,16.57 |
| Found (%) | C,53.14; | H,5.56; | N,16.20 |

In the same manner as in Example 3, the following compounds of Examples 28 to 30 were synthesized.

### Example 28

### 5-methyl-6-[(2E)-3-(1-methyl-2-phenyl-1H-pyrrolo[2,3-b]pyridin-3-yl)prop-2-enoyl]-5,6,7,8-tetrahydro[1,3]dioxolo[4,5-g]isoquinoline hydrochloride

| Elemental analysis for (C₂₈H₂₅N₃O₃·HCl·H₂O) | | | |
|---|---|---|---|
| Calcd.(%) | C,66.46; | H,5.58; | N,8.30 |
| Found (%) | C,66.32; | H,5.69; | N,8.82 |

Positive ion ESI-MS m/z: 452[M+H]⁺

### Example 29

### 1-{1-[(2E)-3-(1-methyl-2-phenyl-1H-pyrrolo[2,3-b]pyridin-3-yl)prop-2-enoyl]piperidin-4-yl}-1,3-dihydro-2H-benzimidazol-2-one hydrochloride

| Elemental analysis for (C₂₉H₂₇N₅O₂·HCl·2H₂O) | | | |
|---|---|---|---|
| Calcd.(%) | C,63.32; | H,5.86; | N,12.73 |
| Found (%) | C,63.93; | H,5.58; | N,12.30 |

Positive ion ESI-MS m/z: 478[M+H]⁺

### Example 30

### (2E)-N-{[4-(4-fluorobenzyl)morpholin-2-yl]methyl}-3-(1-methyl-2-phenyl-1H-indol-3-yl)acrylamide hydrochloride

| Elemental analysis for (C₃₀H₃₀N₃O₂F·HCl·2H₂O) | | | |
|---|---|---|---|
| Calcd.(%) | C,64.80; | H,6.34; | N,7.56 |
| Found (%) | C,65.18; | H,6.24; | N,7.61 |

In the same manner as in Example 10, the following compounds of Examples 31 and 32 were synthesized.

### Example 31

### 5-{4-[(1,5-dimethyl-2-phenyl-1H-indol-3-yl)carbonyl]piperazin-1-yl}-N-[3-(1H-imidazol-1-yl)propyl]pyrazine-2-carboxamide

| Elemental analysis for (C₃₂H₃₄N₈O₂) | | | |
|---|---|---|---|
| Calcd.(%) | C,68.00; | H,6.15; | N,20.25 |
| Found (%) | C,68.31; | H,6.09; | N,19.91 |

Positive ion ESI-MS m/z: 563[M+H]⁺

### Example 32

### 5-{4-[(1,5-dimethyl-2-phenyl-1H-indol-3-yl)carbonyl]piperazin-1-yl}-N-[(3,5-dimethylisoxazol-4-yl)methyl]pyrazine-2-carboxamide

| Elemental analysis for (C₃₂H₃₃N₇O₃) | | | |
|---|---|---|---|
| Calcd.(%) | C,67.90; | H,6.01; | N,17.29 |
| Found (%) | C,68.19; | H,5.90; | N,17.40 |

Positive ion MS m/z: 564[M+H]⁺

In the same manner as in the step 1 of Example 9, the following compounds of Examples 33 to 35 were synthesized.

### Example 33

### 1,5-dimethyl-2-phenyl-3-[{4-[5-(1H-tetrazol-5-yl)pyridin-2-yl]piperazin-1-yl}carbonyl]-1H-indole

| Elemental analysis for (C₂₇H₂₆N₈O·0.4H₂O) | | | |
|---|---|---|---|
| Calcd.(%) | C,66.76; | H,5.56; | N,23.07 |
| Found (%) | C,67.27; | H,5.59; | N,22.68 |

Positive ion FAB-MS m/z: 478[M]⁺

### Example 34

### 1,5-dimethyl-2-phenyl-3-[{4-[5-(1-methyl-1H-tetrazol-5-yl)pyridin-2-yl]piperazin-1-yl}carbonyl]-1H-indole

Positive ion FAB-MS m/z: 492[M]⁺

### Example 35

### 1,5-dimethyl-2-phenyl-3-[{4-[5-(2-isobutyl-2H-tetrazol-5-yl)pyridin-2-yl]piperazin-1-yl}carbonyl]-1H-indole

Positive ion FAB-MS m/z: 535[M+H]⁺

In the same manner as in Example 9, the following compounds of Examples 36 to 37 were synthesized.

### Example 36

### 1,5-dimethyl-2-phenyl-3-[{4-[5-(2-cyclohexylmethyl-2H-tetrazol-5-yl)pyridin-2-yl]piperazin-1-yl}carbonyl]-1H-indole hydrochloride

Positive ion FAB-MS m/z: 575[M+H]⁺

### Example 37

### 1,5-dimethyl-2-phenyl-3-[{4-[5-(2-diethylaminoethyl-2H-tetrazol-5-yl)pyridin-2-yl]piperazin-1-yl}carbonyl]-1H-indole hydrochloride

Positive ion FAB-MS m/z: 578[M+H]⁺

In the same manner as in Example 5, the following compounds of Examples 38 to 61 were synthesized.

### Example 38

### 6-{4-[(1,5-dimethyl-2-phenyl-1H-indol-3-yl)carbonyl]piperazin-1-yl}-N-cyclohexyl-N-methylnicotinamide hydrochloride

| Elemental analysis for (C₃₄H₃₉N₅O₂·HCl·H₂O) | | | |
|---|---|---|---|
| Calcd.(%): | C,67.59; | H,7.01; | N,11.59 |
| Found (%): | C,67.34; | H,6.98; | N,11.22 |

Positive ion FAB-MS m/z: 550[M+H]⁺

### Example 39

### 6-{4-[(1,5-dimethyl-2-phenyl-1H-indol-3-yl)carbonyl]piperazin-1-yl}-N-(2-hydroxyethyl)-N-methylnicotinamide hydrochloride

Positive ion FAB-MS m/z: 512[M+H]⁺

### Example 40

### 6-{4-[(1,5-dimethyl-2-phenyl-1H-indol-3-yl)carbonyl]piperazin-1-yl}-N,N-bis(2-hydroxyethyl)nicotinamide hydrochloride

Positive ion FAB-MS m/z: 542[M+H]⁺

### Example 41

### [1-{[6-{4-[(1,5-dimethyl-2-phenyl-1H-indol-3-yl)carbonyl]piperazin-1-yl}pyridin-3-yl]carbonyl}piperidin-2-yl]methanol hydrochloride

Positive ion FAB-MS m/z: 552[M+H]⁺

### Example 42

### 1-{[6-{4-[(1,5-dimethyl-2-phenyl-1H-indol-3-yl)carbonyl]piperazin-1-yl}pyridin-3-yl]carbonyl}piperidin-3-ol hydrochloride

Positive ion FAB-MS m/z: 538[M+H]⁺

### Example 43

### 1-{[6-{4-[(1,5-dimethyl-2-phenyl-1H-indol-3-yl)carbonyl]piperazin-1-yl}pyridin-3-yl]carbonyl}piperidin-4-ol hydrochloride

Positive ion FAB-MS m/z: 538[M+H]⁺

### Example 44

### 6-{4-[(1,5-dimethyl-2-phenyl-1H-indol-3-yl)carbonyl]piperazin-1-yl}-N-[(2S)-pyrrolidin-2-ylmethyl]nicotinamide hydrochloride

Positive ion FAB-MS m/z: 537[M+H]⁺

### Example 45

### 6-{4-[(1,5-dimethyl-2-phenyl-1H-indol-3-yl)carbonyl]piperazin-1-yl}-N-(3-hydroxypropyl)nicotinamide hydrochloride

Positive ion FAB-MS m/z: 512[M+H]⁺

### Example 46

### 6-{4-[(1,5-dimethyl-2-phenyl-1H-indol-3-yl)carbonyl]piperazin-1-yl}-N-[2-hydroxy-2-(hydroxymethyl)ethyl]nicotinamide hydrochloride

Positive ion FAB-MS m/z: 528[M+H]⁺

### Example 47

### {(2S)-1-[(6-{4-[(1,5-dimethyl-2-phenyl-1H-indol-3-yl)carbonyl]piperazin-1-yl}pyridin-3-yl)carbonyl]pyrrolidin-2-yl}methylamine hydrochloride

Positive ion FAB-MS m/z: 537[M+H]⁺

### Example 48

### 5-{4-[(1,5-dimethyl-2-phenyl-1H-indol-3-yl)carbonyl]piperazin-1-yl}-N-[(5-methylpyrazin-2-yl)methyl]pyrazine-2-carboxamide hydrochloride

Positive ion ESI-MS m/z: 561[M]⁺

### Example 49

### 5-{4-[(5-methyl-6-phenyl-5H-pyrrolo[2,3-b]pyrazin-7-yl)carbonyl]piperazin-1-yl}-N[2-(4-methyl-1,3-thiazol-5-yl)ethyl]pyrazine-2-carboxamide hydrochloride

Positive ion ESI-MS m/z: 568[M]⁺

### Example 50

### 6-{4-[(1,5-dimethyl-2-phenyl-1H-indol-3-yl)carbonyl]piperazin-1-yl}-N-[2-(dimethylamino)ethyl]nicotinamide hydrochloride

Positive ion FAB-MS m/z: 525[M+H]⁺

### Example 51

### 6-{4-[(1,5-dimethyl-2-phenyl-1H-indol-3-yl)carbonyl]piperazin-1-yl}-N-[3-(dimethylamino)propyl]nicotinamide dihydrochloride

| Elemental analysis for (C₃₂H₃₈N₆O₂·2HCl·2.5H₂O) | | | |
|---|---|---|---|
| Calcd.(%) | C,58.53; | H,6.91; | N,12.80 |
| Found (%) | C,58.56; | H,7.06; | N,12.49 |

Positive ion FAB-MS m/z: 539[M+H]⁺

### Example 52

### 4-{[6-{4-[(1,5-dimethyl-2-phenyl-1H-indol-3-yl)carbonyl]piperazin-1-yl}pyridin-3-yl]carbonyl}morpholine hydrochloride

| Elemental analysis for (C₃₁H₃₃N₅O₃·HCl·2H₂O) | | | |
|---|---|---|---|
| Calcd.(%) | C,62.46; | H,6.43; | N,11.75 |
| Found (%) | C,62.98; | H,6.37; | N,11.26 |

Positive ion FAB-MS m/z: 524[M+H]⁺

### Example 53

### 6-{4-[(1,5-dimethyl-2-phenyl-1H-indol-3-yl)carbonyl]piperazin-1-yl}-N-[2-(1-methylpyrrolidin-2-yl)ethyl]nicotinamide hydrochloride

Positive ion FAB-MS m/z: 565[M+H]⁺

### Example 54

### 6-{4-[(1,5-dimethyl-2-phenyl-1H-indol-3-yl)carbonyl]piperazin-1-yl}-N-(2-furylmethyl)nicotinamide hydrochloride

Positive ion FAB-MS m/z: 533[M]⁺

### Example 55

### 6-{4-[(1,5-dimethyl-2-phenyl-1H-indol-3-yl)carbonyl]piperazin-1-yl}-N-(2-methoxyethyl)nicotinamide hydrochloride

| Elemental analysis for (C₃₀H₃₃N₅O₃·HCl·1.5H₂O) | | | |
|---|---|---|---|
| Calcd.(%) | C,62.65; | H,6.48; | N,12.18 |
| Found (%) | C,62.37; | H,6.37; | N,11.98 |

Positive ion FAB-MS m/z: 512[M+H]⁺

### Example 56

### 6-{4-[(1,5-dimethyl-2-phenyl-1H-indol-3-yl)carbonyl]piperazin-1-yl}-N-butylnicotinamide hydrochloride

| Elemental analysis for (C₃₁H₃₅N₅O₂·HCl·1.5H₂O) | | | |
|---|---|---|---|
| Calcd.(%) | C,64.96; | H,6.86; | N,12.22 |
| Found (%) | C,65.18; | H,6.70; | N,12.26 |

Positive ion FAB-MS m/z: 510[M+H]⁺

### Example 57

### 6-{4-[(1,5-dimethyl-2-phenyl-1H-indol-3-yl)carbonyl]piperazin-1-yl}-N-(2-phenoxyethyl)nicotinamide hydrochloride

| Elemental analysis for (C₃₅H₃₅N₅O₃·HCl·1.5H₂O) | | | |
|---|---|---|---|
| Calcd.(%) | C,65.98; | H,6.17; | N,10.99 |
| Found (%) | C,65.91; | H,6.07; | N,10.88 |

Positive ion FAB-MS m/z: 574[M+H]⁺

### Example 58

### 6-{4-[(1,5-dimethyl-2-phenyl-1H-indol-3-yl)carbonyl]piperazin-1-yl}-N-(trans-4-hydroxycyclohexyl)nicotinamide hydrochloride

| Elemental analysis for (C₃₃H₃₇N₅O₃·HCl·2.5H₂O) | | | |
|---|---|---|---|
| Calcd.(%) | C,62.60; | H, 6.84; | N,11.06 |
| Found (%) | C,62.81; | H,6.56; | N,10.75 |

Positive ion FAB-MS m/z: 551[M]⁺

### Example 59

### 6-{4-[(1,5-dimethyl-2-phenyl-1H-indol-3-yl)carbonyl]piperazin-1-yl}-N-(2-methoxyethyl)-N-methylnicotinamide hydrochloride

| Elemental analysis for (C₃₁H₃₅N₅O₃·HCl·H₂O) | | | |
|---|---|---|---|
| Calcd.(%) | C,64.18; | H,6.60; | N,12.07 |
| Found (%) | C,64.20; | H,6.41; | N,11.99 |

Positive ion FAB-MS m/z: 526[M+H]⁺

### Example 60

### 6-{4-[(1,5-dimethyl-2-phenyl-1H-indol-3-yl)carbonyl]piperazin-1-yl}-N-(2-hydroxyethyl)-N-benzylnicotinamide hydrochloride

Positive ion FAB-MS m/z: 588[M+H]⁺

### Example 61

### 6-{4-[(1,5-dimethyl-2-phenyl-1H-indol-3-yl)carbonyl]piperazin-1-yl}-N,N-bis(2-methoxyethyl)nicotinamide hydrochloride

| Elemental analysis for (C₃₃H₃₉N₅O₄·HCl·0.5H₂O) | | | |
|---|---|---|---|
| Calcd.(%) | C,64.43; | H,6.72; | N,11.38 |
| Found (%) | C,64.06; | H,6.61; | N,11.30 |

Positive ion FAB-MS m/z: 569[M]⁺

In the same manner as in Example 10, the following compounds of Examples 62 to 90 were synthesized.

### Example 62

### 6-{4-[(1,5-dimethyl-2-phenyl-1H-indol-3-yl)carbonyl]piperazin-1-yl}-N-cyclohexylnicotinamide

| Elemental analysis for (C₃₃H₃₇N₅O₂·0.6H₂O) | | | |
|---|---|---|---|
| Calcd.(%) | C,72.53; | H,7.05; | N,12.81 |
| Found (%) | C,72.39; | H,6.91; | N,12.71 |

Positive ion FAB-MS m/z: 536[M+H]⁺

### Example 63

### 5-{4-[(1,5-dimethyl-2-phenyl-1H-indol-3-yl)carbonyl]piperazin-1-yl}-N,N-dimethyl-pyrazine-2-carboxamide

| Elemental analysis for (C₂₈H₃₀N₆O₂) | | | |
|---|---|---|---|
| Calcd.(%) | C,69.68; | H,6.27; | N,17.41 |
| Found (%) | C,69.41; | H,6.34; | N,17.15 |

Positive ion FAB-MS m/z: 483[M+H]⁺

### Example 64

### 5-{4-[(1,5-dimethyl-2-phenyl-1H-indol-3-yl)carbonyl]piperazin-1-yl}-N-(2-hydroxyethyl)pyrazine-2-carboxamide

| Elemental analysis for (C₂₈H₃₀N₆O₃·0.7H₂O) | | | |
|---|---|---|---|
| Calcd.(%) | C,65.79; | H,6.19; | N,16.44 |
| Found (%) | C,65.89; | H,6.30; | N,16.01 |

Positive ion FAB-MS m/z: 499[M+H]⁺

### Example 65

### 5-{4-[(1,5-dimethyl-2-phenyl-1H-indol-3-yl)carbonyl]piperazin-1-yl}-N-(2-methoxyethyl)pyrazine-2-carboxamide

| Elemental analysis for (C₂₉H₃₂N₆O₃·0.6H₂O) | | | |
|---|---|---|---|
| Calcd.(%) | C,66.55; | H,6.39; | N,16.06 |
| Found (%) | C,66.94; | H,6.43; | N,15.56 |

Positive ion FAB-MS m/z: 513[M+H]⁺

### Example 66

### 5-{4-[(1,5-dimethyl-2-phenyl-1H-indol-3-yl)carbonyl]piperazin-1-yl}-N-(tetrahydrofuran-2-ylmethyl)pyrazine-2-carboxamide

| Elemental analysis for (C₃₁H₃₄N₆O₃·0.5H₂O) | | | |
|---|---|---|---|
| Calcd.(%) | C,67.99; | H,6.44; | N,15.35 |
| Found (%) | C,68.22; | H,6.56; | N,14.87 |

Positive ion FAB-MS m/z: 539[M+H]⁺

### Example 67

### 5-{4-[(1,5-dimethyl-2-phenyl-1H-indol-3-yl)carbonyl]piperazin-1-yl}-N-(2-hydroxy-1,1-dimethylethyl)pyrazine-2-carboxamide

Positive ion FAB-MS m/z: 527[M+H]⁺

### Example 68

### 2-[1-{[5-{4-[(1,5-dimethyl-2-phenyl-1H-indol-3-yl)carbonyl]piperazin-1-yl}pyrazin-2-yl]carbonyl}piperidin-2-yl]ethanol

Positive ion FAB-MS m/z: 567[M+H]⁺

### Example 69

### 5-{4-[(1,5-dimethyl-2-phenyl-1H-indol-3-yl)carbonyl]piperazin-1-yl}-N-(2,2,2-trifluoroethyl)pyrazine-2-carboxamide

| Elemental analysis for (C₂₈H₂₇F₃N₆O₂) | | | |
|---|---|---|---|
| Calcd.(%) | C,62.68; | H,5.07; | N,15.66 |
| Found (%) | C,62.59; | H,5.16; | N,15.33 |

Positive ion FAB-MS m/z: 537[M+H]⁺

### Example 70

### 5-{4-[(1,5-dimethyl-2-phenyl-1H-indol-3-yl)carbonyl]piperazin-1-yl}-N-[3-(1H-imidazol-1-yl)propyl]nicotinamide

Positive ion ESI-MS m/z: 562[M+H]⁺

### Example 71

### 5-{4-[(1,5-dimethyl-2-phenyl-1H-indol-3-yl)carbonyl]piperazin-1-yl}-N-(3-pyridylmethyl)pyrazine-2-carboxamide

Positive ion ESI-MS m/z: 546[M+H]⁺

### Example 72

### 5-{4-[(1,5-dimethyl-2-phenyl-1H-indol-3-yl)carbonyl]piperazin-1-yl}-N-[2-(3-pyridyl)ethyl]pyrazine-2-carboxamide

| Elemental analysis for (C₃₃H₃₃N₇O₂·0.5H₂O) | | | |
|---|---|---|---|
| Calcd.(%) | C,69.70; | H,6.03; | N,17.24 |
| Found (%) | C,69.89; | H,6.00; | N,17.21 |

Positive ion ESI-MS m/z: 560[M+H]⁺

### Example 73

### 5-{4-[(1,5-dimethyl-2-phenyl-1H-indol-3-yl)carbonyl]piperazin-1-yl}-N-[2-(1H-pyrrol-1-yl)ethyl]pyrazine-2-carboxamide

| Elemental analysis for (C₃₂H₃₃N₇O₂·0.25H₂O) | | | |
|---|---|---|---|
| Calcd.(%) | C,69.61; | H,6.12; | N,17.76 |
| Found (%) | C,69.50; | H,6.14; | N,17.58 |

Positive ion ESI-MS m/z: 548[M+H]⁺

### Example 74

### 5-{4-[(1,5-dimethyl-2-phenyl-1H-indol-3-yl)carbonyl]piperazin-1-yl}-N-[3-(1H-pyrrol-1-yl)propyl]pyrazine-2-Carboxamide

| Elemental analysis for (C₃₃H₃₅N₇O₂·0.2H₂O) | | | |
|---|---|---|---|
| Calcd.(%) | C,70.12; | H,6.31; | N,17.34 |
| Found (%) | C,70.10; | H,6.34; | N,17.23 |

Positive ion ESI-MS m/z: 562[M+H]⁺

### Example 75

### 5-{4-[(1,5-dimethyl-2-phenyl-1H-indol-3-yl)carbonyl]piperazin-1-yl}-N-(2-pyridylmethyl)pyrazine-2-carboxamide

| Elemental analysis for (C₃₂H₃₁N₇O₂) | | | |
|---|---|---|---|
| Calcd.(%) | C,70.44; | H,5.73; | N,17.97 |
| Found (%) | C,70.18; | H,5.89; | N,17.65 |

Positive ion FAB-MS m/z: 546[M+H]⁺

### Example 76

### 5-{4-[(1,5-dimethyl-2-phenyl-1H-indol-3-yl)carbonyl]piperazin-1-yl}-N-[2-(2-pyridyl)ethyl]pyrazine-2-carboxamide

| Elemental analysis for (C₃₃H₃₃N₇O₂·0.3H₂O) | | | |
|---|---|---|---|
| Calcd.(%) | C,70.14; | H,5.99; | N,17.35 |
| Found (%) | C,70.25; | H,5.98; | N,17.28 |

Positive ion FAB-MS m/z: 560[M+H]⁺

### Example 77

### 5-{4-[(1,5-dimethyl-2-phenyl-1H-indol-3-yl)carbonyl]piperazin-1-yl}-N-(2-phenylethyl)pyrazine-2-carboxamide

| Elemental analysis for (C₃₄H₃₄N₆O₂·0.3H₂O) | | | |
|---|---|---|---|
| Calcd.(%) | C,72.40; | H,6.18; | N,14.90 |
| Found (%) | C,72.69; | H,6.14; | N,14.87 |

Positive ion FAB-MS m/z: 559[M+H]⁺

### Example 78

### 5-{4-[(1,5-dimethyl-2-phenyl-1H-indol-3-yl)carbonyl]piperazin-1-yl}-N-[2-(4-methoxyphenyl)ethyl]pyrazine-2-carboxamide

| Elemental analysis for (C₃₅H₃₆N₆O₃·0.3H₂O) | | | |
|---|---|---|---|
| Calcd.(%) | C,70.76; | H,6.21; | N,14.15 |
| Found (%) | C,70.94; | H,6.17; | N,14.06 |

Positive ion FAB-MS m/z: 589[M+H]⁺

### Example 79

### 5-{4-[(1,5-dimethyl-2-phenyl-1H-indol-3-yl)carbonyl]piperazin-1-yl}-N-[3-(3-pyridyl)propyl]pyrazine-2-carboxamide

| Elemental analysis for (C₃₄H₃₅N₇O₂) | | | |
|---|---|---|---|
| Calcd.(%) | C,71.18; | H,6.15; | N,17.09 |
| Found (%) | C,71.14; | H,6.17; | N,17.12 |

### Example 80

### 5-{4-[(1,5-dimethyl-2-phenyl-1H-indol-3-yl)carbonyl]piperazin-1-yl}-N-[2-(4-methyl-1,3-thiazol-5-yl)ethyl]pyrazine-2-carboxamide

| Elemental analysis for (C₃₂H₃₃N₇O₂S·0.25H₂O) | | | |
|---|---|---|---|
| Calcd.(%) | C,65.79; | H,5.78; | N,16.78 |
| Found (%) | C,65.77; | H,5.70; | N,16.66 |

### Example 81

### 5-{4-[(1,5-dimethyl-2-phenyl-1H-indol-3-yl)carbonyl]piperazin-1-yl}-N-[(5-methylpyrazin-2-yl)methyl]pyrazine-2-carboxamide

| Elemental analysis for (C₃₂H₃₂N₈O₂·0.25H₂O) | | | |
|---|---|---|---|
| Calcd.(%) | C,68.01; | H,5.80; | N,19.83 |
| Found (%) | C,67.98; | H,5.90; | N,19.53 |

### Example 82

### 5-{4-[(1,5-dimethyl-2-phenyl-1H-indol-3-yl)carbonyl]piperazin-1-yl}-N-[2-(1-methyl-1H-pyrrol-2-yl)ethyl]pyrazine-2-carboxamide

Positive ion ESI-MS m/z: 562[M+H]⁺

### Example 83

### 5-{4-[(1,5-dimethyl-2-phenyl-1H-indol-3-yl)carbonyl]piperazin-1-yl}-N-[2-(4-hydroxyphenyl)ethyl]pyrazine-2-carboxamide

| Elemental analysis for (C₃₄H₃₄N₆O₃) | | | |
|---|---|---|---|
| Calcd.(%) | C,71.06; | H,5.96; | N,14.62 |
| Found (%) | C,70.95; | H,6.03; | N,14.73 |

Positive ion FAB-MS m/z: 575[M+H]⁺

### Example 84

### 5-{4-[(1,5-dimethyl-2-phenyl-1H-indol-3-yl)carbonyl]piperazin-1-yl}-N-(pyrazin-2-ylmethyl)pyrazine-2-carboxamide

| Elemental analysis for (C₃₁H₃₀N₈O₂) | | | |
|---|---|---|---|
| Calcd.(%) | C,68.11; | H,5.53; | N,20.50 |
| Found (%) | C,68.52; | H,5.76; | N,20.10 |

Positive ion FAB-MS m/z: 547[M+H]⁺

### Example 85

### 5-{4-[(1,5-dimethyl-2-phenyl-1H-indol-3-yl)carbonyl]piperazin-1-yl}-N-(pyrimidine-2-ylmethyl)pyrazine-2-carboxamide

| Elemental analysis for (C₃₁H₃₀N₈O₂·0.2H₂O) | | | |
|---|---|---|---|
| Calcd.(%) | C,67.67; | H,5.57; | N,20.36 |
| Found (%) | C,67.84; | H,5.59; | N,19.99 |

Positive ion FAB-MS m/z: 547[M+H]⁺

### Example 86

### 5-{4-[(1-methyl-2-phenyl-1H-pyrrolo[2,3-b]pyridin-3-yl)carbonyl]piperazin-1-yl}-N-[3-(1H-imidazol-1-yl)propyl]pyrazine-2-carboxamide

| Elemental analysis for (C₃₀H₃₁N₉O₂·0.5H₂O) | | | |
|---|---|---|---|
| Calcd.(%) | C,64.50; | H,5.77; | N,22.57 |
| Found (%) | C,64.60; | H,5.63; | N,22.49 |

### Example 87

### 5-{4-[(1-methyl-2-phenyl-1H-pyrrolo[2,3-b]pyridin-3-yl)carbonyl]piperazin-1-yl}-N-[2-(4-methyl-1,3-thiazol-5-yl)ethyl]pyrazine-2-carboxamide

| Elemental analysis for (C₃₀H₃₀N₈O₂S·0.3H₂O) | | | |
|---|---|---|---|
| Calcd.(%) | C,62.98; | H,5.39; | N,19.59 |
| Found (%) | C,63.26; | H,5.38; | N,19.36 |

### Example 88

### 5-{4-[(1,5-dimethyl-2-phenyl-1H-indol-3-yl)carbonyl]piperazin-1-yl}-N-[2-(2-amino-1,3-thiazol-4-yl)ethyl]pyrazine-2-carboxamide

Positive ion ESI-MS m/z:581 [M+H]⁺

### Example 89

### 5-{4-[(1,5-dimethyl-2-phenyl-1H-indol-3-yl)carbonyl]piperazin-1-yl}-N-[2-(3,5-dimethylisoxazol-4-yl)ethyl]pyrazine-2-carboxamide

Positive ion ESI-MS m/z: 578[M+H]+

### Example 90

### 5-{4-[(1,5-dimethyl-2-phenyl-1H-indol-3-yl)carbonyl]piperazin-1-yl}-N-[(5-ethoxycarbonylmethyl)1,3-thiazol-2-yl]pyrazine-2-carboxamide

Positive ion ESI-MS m/z: 634[M+H]⁺

In the same manner as in Example 2, the following compounds of Examples 91 to 100 were synthesized.

### Example 91

### (2E)-N-(2-fluorobenzyl)-3-(1-methyl-2-phenyl-1H-indol-3-yl)acrylamide

| Elemental analysis for (C₂₅H₂₁FN₂O) | | | |
|---|---|---|---|
| Calcd.(%) | C,78.10; | H,5.51; | N,7.29 |
| Found (%) | C,77.91; | H,5.57; | N,7.24 |

### Example 92

### (2E)-N-(3-fluorobenzyl)-3-(1-methyl-2-phenyl-1H-indol-3-yl)acrylamide

| Elemental analysis for (C₂₅H₂₁FN₂O) | | | |
|---|---|---|---|
| Calcd.(%) | C,78.10; | H,5.51; | N,7.29 |
| Found (%) | C,78.14; | H,5.60; | N,7.16 |

### Example 93

### (2E)-N-(4-fluorobenzyl)-3-(1-methyl-2-phenyl-1H-indol-3-yl)acrylamide

| Elemental analysis for (C₂₅H₂₁FN₂O) | | | |
|---|---|---|---|
| Calcd.(%) | C,78.10; | H,5.51; | N,7.29 |
| Found (%) | C,78.11; | H,5.68; | N,7.29 |

### Example 94

### (2E)-N-(2-methylbenzyl)-3-(1-methyl-2-phenyl-1H-indol-3-yl)acrylamide

| Elemental analysis for (C₂₆H₂₄N₂O) | | | |
|---|---|---|---|
| Calcd.(%) | C,82.07; | H,6.36; | N,7.36 |
| Found (%) | C,82.11; | H,6.44; | N,7.35 |

### Example 95

### (2E)-N-(3-methylbenzyl)-3-(1-methyl-2-phenyl-1H-indol-3-yl)acrylamide

| Elemental analysis for (C₂₆H₂₄N₂O) | | | |
|---|---|---|---|
| Calcd.(%) | C,82.07; | H,6.36; | N,7.36 |
| Found (%) | C,82.19; | H,6.43; | N,7.35 |

### Example 96

### (2E)-N-(4-methylbenzyl)-3-(1-methyl-2-phenyl-1H-indol-3-yl)acrylamide

| Elemental analysis for (C₂₆H₂₄N₂O) | | | |
|---|---|---|---|
| Calcd.(%) | C,82.07; | H,6.36; | N,7.36 |
| Found (%) | C,81.66; | H,6.40; | N,7.33 |

### Example 97

### (2E)-N-[(1R, 2S)-2-hydroxy-2,3-dihydro-1H-inden-1-yl]-3-(1-methyl-2-phenyl-5,6-methylenedioxy-1H-indol-3-yl)acrylamide

Positive ion FAB-MS m/z: 452[M]⁺

### Example 98

### 1-[(2E)-3-(1-methyl-2-phenyl-1H-pyrrolo[2,3-b]pyridin-3-yl)prop-2-enoyl]-4-(4-methoxyphenyl)piperazine

| Elemental analysis for (C₂₈H₂₈N₄O₂·0.5H₂O) | | | |
|---|---|---|---|
| Calcd.(%) | C,72.86; | H,6.33; | N,12.14 |
| Found (%) | C,72.47; | H,6.07; | N,12.38 |

Positive ion ESI-MS m/z: 453[M+H]⁺

### Example 99

### (2E)-N-[(1R, 2S)-2-methoxy-2,3-dihydro-1H-inden-1-yl]-3-(1-methyl-2-phenyl-5,6-methylenedioxy-1H-indol-3-yl)acrylamide

| Elemental analysis for (C₂₉H₂₆N₂O₄·0.5H₂O) | | | |
|---|---|---|---|
| Calcd.(%) | C,73.25; | H,5.72; | N,5.89 |
| Found (%) | C,73.20; | H,5.84; | N,5.59 |

Positive ion ESI-MS m/z: 467[M+H]⁺

### Example 100

### (2E)-N-(4-methoxyphenyl)-3-(1-methyl-2-phenyl-1H-pyrrolo[2,3-b]pyridin-3-yl)acrylamide

| Elemental analysis for (C₂₄H₂₁N₃O₂) | | | |
|---|---|---|---|
| Calcd.(%) | C,75.18; | H,5.52; | N,10.96 |
| Found (%) | C,74.69; | H,5.66; | N,10.84 |

Positive ion ESI-MS m/z: 384[M+H]⁺

In the same manner as in Example 3, the following compounds of Examples 101 to 109 were synthesized.

### Example 101

### (2E)-N-{[4-(4-fluorobenzyl)morpholin-2-yl]methyl}-3-(1-methyl-2-phenyl-5-methoxy-1H-indol-3-yl)acrylamide hydrochloride

| Elemental analysis for (C₃₁H₃₂FN₃O₃·HCl·1.5H₂O) | | | |
|---|---|---|---|
| Calcd.(%) | C,64.52; | H,6.29; | N,7.28 |
| Found (%) | C,64.70; | H,6.41; | N,7.31 |

### Example 102

### (2E)-N-{[4-(4-fluorobenzyl)morpholin-2-yl]methyl}-3-(1,5-dimethyl-2-phenyl-1H-indol-3-yl)acrylamide hydrochloride

| Elemental analysis for (C₃₁H₃₂FN₃O₂·HCl·2.5H₂O) | | | |
|---|---|---|---|
| Calcd.(%) | C,64.30; | H,6.61; | N,7.26 |
| Found (%) | C,64.36; | H, 6.21; | N,7.34 |

### Example 103

### (2E)-N-{[4-(4-fluorobenzyl)morpholin-2-yl]methyl}-3-(1-methyl-2-phenyl-5-fluoro-1H-indol-3-yl)acrylamide hydrochloride

| Elemental analysis for (C₃₀H₂₉F₂N₃O₂·HCl·0.75H₂O) | | | |
|---|---|---|---|
| Calcd.(%) | C,65.33; | H,5.75; | N,7.62 |
| Found (%) | C,65.05; | H,5.48; | N,8.16 |

### Example 104

### 2-[(2E)-3-(1-methyl-2-phenyl-1H-pyrrolo[2,3-b]pyridin-3-yl)prop-2-enoyl]-1,2,3,4-tetrahydroisoquinoline hydrochloride

| Elemental analysis for (C₂₆H₂₃N₃O·HCl) | | | |
|---|---|---|---|
| Calcd.(%) | C,72.63; | H,5.63; | N,9.77 |
| Found (%) | C,72.13; | H,5.71; | N,9.19 |

Positive ion ESI-MS m/z: 394[M+H]⁺

### Example 105

### 1-[(2E)-3-(1-methyl-2-phenyl-1H-pyrrolo[2,3-b]pyridin-3-yl)prop-2-enoyl]-4-(4-pyrazin-2-yl)piperazine trihydrochloride

| Elemental analysis for (C₂₅H₂₄N₆O·3HCl) | | | |
|---|---|---|---|
| Calcd.(%) | C,56.24; | H,5.10; | N,15.74 |
| Found (%) | C,56.28; | H,5.58; | N,15.26 |

Positive ion ESI-MS m/z: 425[M+H]⁺

### Example 106

### 8-[(2E)-3-(1-methyl-2-phenyl-1H-pyrrolo[2,3-b]pyridin-3-yl)prop-2-enoyl]-2-phenyl-1,3,8-triazabicyclo[4.5]decan-4-one hydrochloride

| Elemental analysis for (C₃₀H₂₉N₅O₂·HCl·H₂O) | | | |
|---|---|---|---|
| Calcd.(%) | C,65.99; | H,5.91; | N,12.83 |
| Found (%) | C,66.22; | H,5.66; | N,12.18 |

Positive ion ESI-MS m/z: 492[M+H]⁺

### Example 107

### (2E)-N-(3-hydroxy-3-phenylpropyl)-3-(1-methyl-2-phenyl-1H-pyrrolo[2,3-b]pyridin-3-yl)acrylamide hydrochloride

| Elemental analysis for (C₂₆H₂₅N₃O₂·HCl·2H₂O) | | | |
|---|---|---|---|
| Calcd.(%) | C,64.52; | H,6.25; | N,8.68 |
| Found (%) | C,65.96; | H,6.66; | N,8.08 |

Positive ion ESI-MS m/z: 412[M+H]⁺

### Example 108

### 1-[(2E)-3-(1-methyl-2-phenyl-1H-pyrrolo[2,3-b]pyridin-3-yl)prop-2-enoyl]-4-phenylpiperidin-4-ol hydrochloride

| Elemental analysis for (C₂₈H₂₇N₃O₂·HCl·H₂O) | | | |
|---|---|---|---|
| Calcd.(%) | C,68.35; | H,6.15; | N,8.54 |
| Found (%) | C,68.71; | H,6.20; | N,8.06 |

Positive ion ESI-MS m/z: 438[M+H]⁺

### Example 109

### 2-[(2E)-3-(1-methyl-2-phenyl-1H-pyrrolo[2,3-b]pyridin-3-yl)prop-2-enoyl]-6,7-dimethoxy-1,2,3,4-tetrahydroisoquinoline hydrochloride

| Elemental analysis for (C₂₈H₂₇N₃O₃·HCl·0.8H₂O) | | | |
|---|---|---|---|
| Calcd.(%) | C,66.67; | H,5.91; | N,8.33 |
| Found (%) | C,66.63; | H,5.91; | N,8.37 |

Positive ion ESI-MS m/z: 454[M+H]⁺

### Example 110

### (2E)-N-methyl-N-(3-hydroxy-3-phenylpropyl)-3-[1-methyl-2-(4-fluorophenyl)-1H-indol-3-yl]acrylamide

(2E)-3-[1-methyl-2-(4-fluorophenyl)-1H-indol-3-yl]acrylic acid (30 mg) was dissolved in dimethylformamide (3 ml) and PS(polystyrene)-carbodiimide (0.88 mmol/g, 174 mg) and 1-hydroxybenzotriazole (21 mg) were added, followed by stirring for 30 minutes. Then, α-[2-(methylamino)ethyl]benzyl alcohol (19 mg) was added and the mixture was stirred overnight. To the reaction solution was added PS-trisamine (4.50 mmol/g, 100 mg), followed by stirring for another one hour. The reaction solution was filtered and the filtrate was concentrated. The resulting residue was purified by LC-MS to obtain the objective product (29 mg).
Positive ion ESI-MS m/z: 443[M+H]⁺

In the same manner as in Example 110, the following compounds of Examples 111 to 119 were synthesized.

### Example 111

### (2E)-N-methyl-N-(3-hydroxy-3-phenylpropyl)-3-(1-methyl-2-phenyl-7-methoxy-1H-indol-3-yl)acrylamide

Positive ion ESI-MS m/z: 455[M+H]⁺

### Example 112

### (2E)-N-methyl-N-(3-hydroxy-3-phenylpropyl)-3-[1-methyl-2-(3,4-methylenedioxyphenyl)-1H-indol-3-yl]acrylamide

Positive ion ESI-MS m/z: 469[M+H]⁺

### Example 113

### (2E)-N-[(1R,2S)-2-hydroxy-2,3-dihydro-1H-inden-1-yl]-3-[1-methyl-2-(3,4-methylenedioxyphenyl)-1H-indol-3-yl]acrylamide

Positive ion ESI-MS m/z: 453[M+H]⁺

### Example 114

### 2-{(2E)-3-[1-methyl-2-(3,4-methylenedioxyphenyl)-1H-indol-3-yl]prop-2-enoyl}-1-methyl-6,7-dihydroxy-1,2,3,4-tetrahydroisoquinoline

Positive ion ESI-MS m/z: 483[M+H]⁺

### Example 115

### (2E)-N-methyl-N-[2-(3,4-dimethoxyphenyl)ethyl]-3-[1-methyl-2-(3,4-methylenedioxyphenyl)-1H-indol-3-yl]acrylamide

Positive ion ESI-MS m/z: 499[M+H]⁺

### Example 116

### (2E)-N-(4-hydroxybutyl)-N-benzyl-3-[1-methyl-2-(3,4-methylenedioxyphenyl)-1H-indol-3-yl]acrylamide

Positive ion ESI-MS m/z: 483[M+H]⁺

### Example 117

### 2-{(2E)-3-[1-methyl-2-phenyl-(5,6-methylenedioxy)-1H-indol-3-yl]prop-2-enoyl}-1-methyl-6,7-dihydroxy-1,2,3,4-tetrahydroisoquinoline

Positive ion ESI-MS m/z: 483[M+H]⁺

### Example 118

### (2E)-N-methyl-N-(3-hydroxy-3-phenylpropyl)-3-[1-methyl-2-(3-methoxyphenyl)-1H-indol-3-yl]acrylamide

Positive ion ESI-MS m/z: 455[M+H]⁺

### Example 119

### (2E)-N-methyl-N-(3-hydroxy-3-phenylpropyl)-3-[1-methyl-2-(4-methoxyphenyl)-1H-indol-3-yl]acrylamide

Positive ion ESI-MS m/z: 455[M+H]⁺

In the same manner as in Example 5, the following compound of Example 120 was synthesized.

### Example 120

### 6-{4-[(1,5-dimethyl-2-phenyl-1H-indol-3-yl)carbonyl]piperazin-1-yl}-N,N-dimethylnicotinamide hydrochloride

| Elemental analysis for (C₂₉H₃₁N₅O₂·HCl·0.5H₂O) | | | |
|---|---|---|---|
| Calcd.(%) | C,66.09; | H,6.31; | N,13.29 |
| Found (%) | C,66.29; | H,6.42; | N,12.95 |

Positive ion ESI-MS m/z: 482[M+H]⁺

In the same manner as in the step 1 of Example 9, the following compound of Example 121 was synthesized.

### Example 121

### N-[5-{4-[(1,5-dimethyl-2-phenyl-1H-indol-3-yl)carbonyl]piperazin-1-yl}pyrazin-2-yl]acetamide

| Elemental analysis for (C₂₇H₂₈N₆O₂) | | | |
|---|---|---|---|
| Calcd.(%) | C,64.80; | H,6.34; | N,7.56 |
| Found (%) | C,65.18; | H,6.24; | N,7.61 |

Positive ion FABMS m/z: 469[M+H]⁺

In the same manner as in Example 110, the following compound of Example 122 was synthesized.

### Example 122

### (2E)-N-methyl-N-(1-methyl-piperidin-4-yl)-3-(1-methyl-2-phenyl-1H-pyrrolo[2,3-b]pyridin-3-yl)acrylamide

Positive ion ESI-MS m/z: 389[M+H]⁺

In the same manner as in Example 3, the following compound of Example 123 was synthesized.

### Example 123

### 2-[(2E)-3-(1,5-dimethyl-2-phenyl-1H-pyrrolo[2,3-b]pyridin-3-yl)prop-2-enoyl]-6,7-dimethoxy-1,2,3,4-tetrahydroisoquinoline hydrochloride

| Elemental analysis for (C₂₉H₂₉N₃O₃·HCl·0.5H₂O) | | | |
|---|---|---|---|
| Calcd.(%) | C,67.89; | H,6.09; | N,8.19 |
| Found (%) | C,67.73; | H,6.02; | N,8.18 |

Positive ion FAB-MS m/z: 468[M+H]⁺

### Example 124

### {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetic acid

1-carboxymethyl-6,7-dimethoxy-3,4-dihydro-1H-isoquinoline-2-carboxylic acid9H-fluoren-9-ylmethyl ester (100 mg) was dissolved in dimethylformamide (3 ml) and a Wang resin (2.53 mmol/g, 100 mg), diisopropyl carbodiimide (0.15 ml) and dimethylaminopyridine (12 mg) were added, followed by stirring for 5 hours. After suction filtration, the resin was washed five times with dimethylformamide (4 ml). Subsequently, a 20% piperidine in dimethylformamide solution (4 ml) was added, followed by stirring for 20 minutes. After suction filtration, the resin was washed four times with dimethylformamide (4 ml). Dimethylformamide (3 ml) was added, and diisopropyl carbodiimide (0.15 ml), hydroxybenztriazole (135 mg) and (2E)-3-[1-methyl-2-phenyl-1H-pyrrolo[2,3-b]pyridin-3-yl]acrylic acid (30 mg) were added, followed by stirring for 5 hours, After suction filtration, the resin was washed three times with dimethylformamide (4 ml). Trifluoroacetic acid (2 ml) was added, followed by stirring for 2 hours. The reaction solution was subjected to suction filtration and the filtrate was concentrated under reduced pressure. The resulting residue was purified by LC-MS to obtain the objective product (32 mg).

Positive ion ESI-MS m/z: 512 [M+H]⁺

In the same manner as in Example 110, the following compounds of Examples 125 to 127 were synthesized.

### Example 125

### N-benzyl-N-[(5-{4-[(1,5-dimethyl-2-phenyl-1H-indol-3-yl)carbonyl]piperazin-1-yl}pyrazin-2-yl)carbonyl]aminoethyl acetate

Positive ion ESI-MS m/z: 631[M+H]⁺

### Example 126

### Ethyl 2-{N-[(2E)-3-(1-methyl-2-phenyl-1H-pyrrolo[2,3-b]pyridin-3-yl)prop-2-enoyl]amino}-1-cyclopentanecarboxylate

Positive ion ESI-MS m/z: 418[M+H]⁺

### Example 127

### Ethyl 2-{N-[(5-{4-[(1,5-dimethyl-2-phenyl-1H-indol-3-yl)carbonyl]piperazin-1-yl}pyrazin-2-yl)carbonyl]amino}-1-cyclohexanecarboxylate

Positive ion ESI-MS m/z: 609[M+H]⁺

In the same manner as in Example 3, the following compound of Example 128 was synthesized.

### Example 128

### 2-[(2E)-3-(5-methyl-6-phenyl-5H-pyrrolo[2,3-b]pyrazin-7-yl)prop-2-enoyl]-6,7-dimethoxy-1,2,3,4-tetrahydroisoquinoline hydrochloride

| Elemental analysis for (C₂₇H₂₆N₄O₃·HCl·0.25H₂O) | | | |
|---|---|---|---|
| Calcd.(%) | C,65.45; | H,5.59; | N,11.31 |
| Found (%) | C,65.48; | H,5.61; | N,11.34 |

Positive ion ESI-MS m/z: 454[M]⁺

In the same manner as in Example 110, the following compound of Example 129 was synthesized.

### Example 129

### Ethyl 2-{N-[(5-{4-[(1,5-dimethyl-2-phenyl-1H-indol-3-yl)carbonyl]piperazin-1-yl}pyrazin-2-yl)carbonyl]amino}-1-cyclopentanecarboxylate

Positive ion ESI-MS m/z: 595[M+H]⁺

In the same manner as in Example 3, the following compounds 130 and 131 were synthesized.

### Example 130

### 2-[(2E)-3-(1-methyl-2-phenyl-1H-pyrrolo[2,3-c]pyridin-3-yl)prop-2-enoyl]-6,7-dimethoxy-1,2,3,4-tetrahydroisoquinoline hydrochloride

| Elemental analysis for (C₂₈H₂₇N₃O₃·HCl·2H₂O) | | | |
|---|---|---|---|
| Calcd.(%) | C,63.93; | H,6.13; | N,7.99 |
| Found (%) | C,63.82; | H,6.65; | N,8.04 |

Positive ion ESI-MS m/z: 453[M]⁺

### Example 131

### 2-{(2E)-3-[1-methyl-2-(3,4-dimethoxyphenyl)-1H-pyrrolo[2,3-b]pyridin-3-yl]prop-2-enoyl}-6,7-dimethoxy-1,2,3,4-tetrahydroisoquinoline hydrochloride

| Elemental analysis for (C₃₀H₃₁N₃O₅·HCl·2H₂O) | | | |
|---|---|---|---|
| Calcd.(%) | C,61.48; | H,6.19; | N,7.17 |
| Found (%) | C,61.43; | H,6.12; | N,7.08 |

Positive ion ESI-MS m/z: 513[M]⁺

In the same manner as in Example 125, the following compound of Example 132 was synthesized.

### Example 132

### N-methyl-N-[(5-{4-[(1,5-dimethyl-2-phenyl-1H-indol-3-yl)carbonyl]piperazin-1-yl}pyrazin-2-yl)carbonyl]-(S)-phenylalanine

Positive ion ESI-MS m/z: 617[M+H]⁺

In the same manner as in Example 3, the following compound of Example 133 was synthesized.

### Example 133

### Methyl {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetate hydrochloride

| Elemental analysis for (C₃₁H₃₁N₃O₅·HCl·0.75H₂O) | | | |
|---|---|---|---|
| Calcd.(%) | C,64.69; | H,5.87; | N,7.30 |
| Found (%) | C,64.93; | H,6.18; | N,7.26 |

Positive ion ESI-MS m/z: 525[M]⁺

The following compound of Example 134 was synthesized by hydrolyzing the compound of Example 133 with an alkali and converting the product into a hydrochloride using 4N hydrochloric acid in ethyl acetate.

### Example 134

### {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetic acid hydrochloride

| Elemental analysis for (C₃₀H₂₉N₃O₅·HCl·H₂O) | | | |
|---|---|---|---|
| Calcd.(%) | C,63.66; | H,5.70; | N,7.42 |
| Found (%) | C,63.98; | H,5.94; | N,7.14 |

Positive ion ESI-MS m/z: 511[M]⁺

In the same manner as in Example 3, the following compounds of Examples 135 and 136 were synthesized.

### Example 135

### 2-{(2E)-3-[1-methyl-2-(4-pyridyl)-1H-pyrrolo[2,3-b]pyridin-3-yl]prop-2-enoyl}-6,7-dimethoxy-1,2,3,4-tetrahydroisoquinoline dihydrochloride

| Elemental analysis for (C₂₇H₂₆N₄O₃·2HCl·0.8H₂O) | | | |
|---|---|---|---|
| Calcd.(%) | C,59.85; | H,5.51; | N,10.33 |
| Found (%) | C,59.94; | H,5.66; | N,10.03 |

### Example 136

### 2-[(2E)-3-(1-methyl-2-phenyl-1H-pyrrolo[3,2-b]pyridin-3-yl)prop-2-enoyl]-6,7-dimethoxy-1,2,3,4-tetrahydroisoquinoline hydrochloride

| Elemental analysis for (C₂₈H₂₇N₃O₃·HCl·0.5H₂O) | | | |
|---|---|---|---|
| Calcd.(%) | C,67.39; | H,5.86; | N,8.42 |
| Found (%) | C,67.11; | H,5.87; | N,8.33 |

Positive ion ESI-MS m/z: 453[M]⁺

### Example 137

### N-[(2E)-3-(1-methyl-2-phenyl-1H-pyrrolo[2,3-b]pyridin-3-yl)prop-2-enoyl]-N-[2-(2-thienyl)ethyl]aminoacetic acid

2-(2-thienyl)ethylamine (20 mg) was dissolved in acetonitrile (2 ml) and bromoacetic acid tert-butyl ester (63 mg) and triethylamine (50 mg) were added, followed by stirring for 14 hours. To the reaction solution was added a 4-benzyloxybenzaldehyde polystyrene resin (2.81 mmol/g, 100 mg), followed by stirring for another 8 hours. The reaction solution was subjected to suction filtration and the filtrate was concentrated under reduced pressure. The concentrate was dissolved in dimethylformamide (2 ml) and 1-ethyl-3-(3'-dimethylaminopropyl)carbodiimide hydrochloride (25 mg), hydroxybenztriazole (17 mg) and (2E)-3-[1-methyl-2-phenyl-1H-pyrrolo[2,3-b]pyridin-3-yl]acrylic acid (30 mg) were added, followed by stirring for 14 hours. The reaction solution was concentrated under reduced pressure and trifluoroacetic acid (2 ml) was added, followed by stirring for 12 hours. The solution was concentrated under reduced pressure again and the resulting residue was purified by LC-MS to obtain the objective product (18 mg).
Positive ion ESI-MS m/z: 446 [M+H]⁺

### Example 138

### 6,7-dimethoxy-2-[(2E)-3-(2-phenyl-1H-pyrrolo[2,3-b]pyridin-3-yl)prop-2-enoyl]-1,2,3,4-tetrahydroisoquinoline dihydrochloride

To a solution of 6,7-dimethoxy-2-[(2E)-3-{1-[2-(trimethylsilyl)ethoxy]methyl-2-phenyl-1H-pyrrolo[2,3-b]pyridin-3-yl)prop-2-enoyl]-1,2,3,4-tetrahydroisoquinoline (150 mg) obtained in the same manner as in Example 3 in tetrahydrofuran (1 ml), 1.0M tetrabutylammonium fluoride (2.1 ml) was added at room temperature. After refluxing for 3 hours, a 1.0M tetrabutylammonium chloride tetrahydrofuran solution (1.5 ml) was further added and the mixture was heated at reflux for 13.5 hours. The reaction solution was mixed with water, extracted with ethyl acetate, washed twice with water, washed once with saturated brine, dried and then concentrated. The resulting product was washed with diethyl ether and methanol to obtain a white powder (52 mg). The white powder was dissolved in chloroform (2 ml) and a 4.0M HCl in ethyl acetate solution (0.031 ml) was added during stirring at room temperature. The reaction solution was concentrated and the resulting product was washed with diethyl ether and ethyl acetate to obtain a white powder (45 mg).

| Elemental analysis for (C₂₇H₂₅N₃O₃·2HCl·2.2H₂O) | | | |
|---|---|---|---|
| Calcd.(%) | C,58.74; | H,5.73; | N,7.61 |
| Found (%) | C,58.67; | H,5.58; | N,7.50 |

In the same manner as in Example 3, the following compounds of Examples 139 and 140 were synthesized.

### Example 139

### 2-[(2E)-3-(1-methoxymethyl-2-phenyl-1H-pyrrolo[2,3-b]pyridin-3-yl)prop-2-enoyl]-6,7-dimethoxy-1,2,3,4-tetrahydroisoquinoline hydrochloride

| Elemental analysis for (C₂₉H₂₉N₃O₄·HCl·) | | | |
|---|---|---|---|
| Calcd.(%) | C,66.98; | H,5.81; | N,8.08 |
| Found (%) | C,67.09; | H,5.91; | N,8.05 |

### Example 140

### 2-{(2E)-3-[1-methyl-2-(4-fluorophenyl)-1H-pyrrolo[2,3-b]pyridin-3-yl]prop-2-enoyl}-6,7-dimethoxy-1,2,3,4-tetrahydroisoquinoline hydrochloride

| Elemental analysis for (C₂₈H₂₆FN₃O₃·HCl·H₂O) | | | |
|---|---|---|---|
| Calcd.(%) | C,63.94; | H,5.56; | N,7.99 |
| Found (%) | C,64.00; | H,5.55; | N,8.04 |

In the same manner as in Example 110, the following compounds of Examples 141 to 143 were synthesized.

### Example 141

### 4-acetyl-1-{(2E)-3-[1,5-dimethyl-2-(4-carbomethoxyphenyl)-1H-indol-3-yl]prop-2-enoyl}-4-phenylpiperidine

Positive ion ESI-MS m/z: 535[M+H]⁺

### Example 142

### 2-{(2E)-3-[1,5-dimethyl-2-(4-carbomethoxyphenyl)-1H-indol-3-yl]prop-2-enoyl}-6,7-dimethoxy-1,2,3,4-tetrahydroisoquinoline

Positive ion ESI-MS m/z: 525[M+H]⁺

### Example 143

### (2E)-N-methyl-N-(1-methylpiperidin-4-yl)-3-[1,5-dimethyl-2-(4-carbomethoxyphenyl)-1H-indol-3-yl]acrylamide

Positive ion ESI-MS m/z: 460[M+H]⁺

In the same manner as in Example 5, the following compounds of Examples 144 and 145 were synthesized.

### Example 144

### 5-{4-[(5-bromo-1-methyl-2-phenyl-1H-indol-3-yl)carbonyl]piperazin-1-yl}-N-[2-(4-methyl-1,3-thiazol-5-yl)ethyl]pyrazine-2-carboxamide hydrochloride

Positive ion FAB-MS m/z: 644[M+H]⁺

### Example 145

### 5-{4-[(1,5-dimethyl-2-phenyl-1H-indol-3-yl)carbonyl]piperazin-1-yl}-N-[2-(4-hydroxyphenyl)ethyl]pyrazine-2-carboxamide hydrochloride Positive ion FAB-MS m/z: 575[M+H]⁺

The following compound of Example 146 was synthesized by hydrolyzing the compound obtained in the same manner as in Example 110 with an alkali.

### Example 146

### 4-{3-[3-(4-acetyl-4-phenylpiperidin-1-yl)-3-oxo-1-propenyl]-1,5-dimethyl-1H-indol-2-yl}benzoic acid

Positive ion ESI-MS m/z: 521[M+H]⁺

In the same manner as in Example 5, the following compound of Example 147 was synthesized.

### Example 147

### 5-{4-[(5-fluoro-1-methyl-2-phenyl-1H-indol-3-yl)carbonyl]piperazin-1-yl}-N-[2-(4-methyl-1,3-thiazol-5-yl)ethyl]pyrazine-2-carboxamide dihydrochloride

| Elemental analysis for (C₃₁H₃₀FN₇O₂S·2HCl·3.5H₂O) | | | |
|---|---|---|---|
| Calcd.(%) | C,51.74; | H,5.46; | N,13.62 |
| Found (%) | C,51.75; | H,5.81; | N,13.59 |

In the same manner as in Example 5, the following compound of Example 148 was synthesized.

### Example 148

### 5-{4-[(1,5-dimethyl-2-phenyl-1H-pyrrolo[2,3-b]pyridin-3-yl)carbonyl]piperazin-1-yl}-N-[2-(4-methyl-1,3-thiazol-5-yl)ethyl]pyrazine-2-carboxamide trihydrochloride

| Elemental analysis for (C₃₁H₃₂N₈O₂S·3HCl·4H₂O) | | | |
|---|---|---|---|
| Calcd.(%) | C,46.65; | H,5.93; | N,14.04 |
| Found (%) | C,46.73; | H,5.85; | N,13.98 |

Positive ion ESI-MS m/z: 580[M]⁺

In the same manner as in Example 3, the following compound of Example 149 was synthesized.

### Example 149

### 2-{(2E)-3-[1-methyl-2-(3,4-methylenedioxyphenyl)-1H-pyrrolo[2,3-b]pyridin-3-yl]prop-2-enoyl}-6,7-dimethoxy-1,2,3,4-tetrahydroisoquinoline hydrochloride

| Elemental analysis for (C₂₉H₂₇N₃O₅·HCl·H₂O) | | | |
|---|---|---|---|
| Calcd.(%) | C,63.10; | H,5.48; | N,7.62 |
| Found (%) | C,63.11; | H,5.39; | N,7.69 |

In the same manner as in Example 5, the following compound of Example 150 was synthesized.

### Example 150

### 5-{4-[(5-chloro-1-methyl-2-phenyl-1H-indol-3-yl)carbonyl]piperazin-1-yl}-N-[2-(4-methyl-1,3-thiazol-5-yl)ethyl]pyrazine-2-carboxamide dihydrochloride

Positive ion ESI-MS m/z:600[M+H]⁺

The following compound of Example 151 was synthesized by hydrolyzing the compound obtained in the same manner as in Example 5 with an alkali.

### Example 151

### N-{[5-{4-[(1,5-dimethyl-2-phenyl-1H-indol-3-yl)carbonyl]piperazin-1-yl}pyrazin-2-yl]carbonyl}-(S)-phenylalanine

| Elemental analysis for (C₃₅H₃₄N₆O₄·H₂O) | | | |
|---|---|---|---|
| Calcd.(%) | C,67.73; | H,5.85; | N,13.54 |
| Found (%) | C,67.47; | H,5.66; | N,13.48 |

In the same manner as in Example 5, the following compound of Example 152 was synthesized.

### Example 152

### N-methyl-N-{[5-{4-[(1,5-dimethyl-2-phenyl-1H-indol-3-yl)carbonyl]piperazin-1-yl}pyrazin-2-yl]carbonyl}-(S)-phenylalaninemethyl ester

| Elemental analysis for (C₃₈H₄₀N₆O₄·0.5H₂O) | | | |
|---|---|---|---|
| Calcd.(%) | C,69.81; | H,6.32; | N,12.85 |
| Found (%) | C,69.64; | H,6.18; | N,12.87 |

The following compound of Example 153 was synthesized by hydrolyzing the compound obtained in the same manner as in Example 3 with an alkali.

### Example 153

### 4-{3-[3-(6,7-dimethoxy-1,2,3,4-tetrahydroisoquinolin-2-yl)-3-oxo-propenyl]-1-methyl-1H-pyrrolo[2,3-b]pyridin-2-yl}benzoic acid hydrochloride

| Elemental analysis for (C₂₉H₂₇N₃O₅·HCl·0.5H₂O) | | | |
|---|---|---|---|
| Calcd.(%) | C,64.14; | H,5.38; | N,7.74 |
| Found (%) | C,64.37; | H,5.11; | N,7.76 |

In the same manner as in Example 3, the following compound of Example 154 was synthesized.

### Example 154

### Methyl [6,7-dimethoxy-2-{(2E)-3-[1-methyl-2-(4-fluorophenyl)-1H-pyrrolo[2,3-b]pyridin-3-yl]prop-2-enoyl}-1,2,3,4-tetrahydroisoquinolin-1-yl]acetate hydrochloride

| Elemental analysis for (C₃₂H₃₂FN₃O₅·HCl·1.3H₂O) | | | |
|---|---|---|---|
| Calcd.(%) | C,62.24; | H,5.81; | N,6.80 |
| Found (%) | C,62.33; | H,5.91; | N,6.99 |

The following compound of Example 155 was synthesized by hydrolyzing the compound obtained in the same manner as in Example 3 with an alkali.

### Example 155

### [6,7-dimethoxy-2-{(2E)-3-[1-methyl-2-(4-fluorophenyl)-1H-pyrrolo[2,3-b]pyridin-3-yl]prop-2-enoyl}-1,2,3,4-tetrahydroisoquinolin-1-yl]acetic acid

| Elemental analysis for (C₃₀H₂₈FN₃O₅·0.7H₂O) | | | |
|---|---|---|---|
| Calcd.(%) | C,66.46; | H,5.47; | N,7.75 |
| Found (%) | C,66.53; | H,5.58; | N,7.53 |

In the following Test Examples 1 and 2, 1-(1,5-dimethyl-2-phenylindol-3-ylcarbonyl)-4-(2-pyridyl)piperazine hydrochloride (hereinafter referred to as a compound A) whose inhivitory action against TGF-β is described in International Publication WO00/44743 was used as a control compound for comparison.

### Test Example 1

### Inhibitory effect on collagen production induced by TGF-β

As an index of collagen production, the uptake of [³H] proline was measured.

Using a 96-well microplate, normal human skin derived fibroblast (NHDF, 10⁴/well) or human embryonic lung fibroblast (TIG-3, 10⁴/well) were cultured in a 10% fetal calf serum (FCS)-containing Dulbecco's modified Eagle medium (DMEM) for one day. After removing the culture supernatant, the cells were washed once with a 0.1% bovine serum albumin (BSA) -containing DMEM medium and then cultured in the 0.1% bovine serum albumin (BSA)-containing DMEM medium for one day. After adding each of the test compounds in each concentration (1, 3, 10 µM), the cells were cultured and, after adding TGF-β1 (3ng/ml) and [³H] proline (18.5 kBq/well), the cells were further cultured for additional one day.

After removing the culture supernatant, the cells were washed twice with a phosphate buffer solution (PBS) and dissolved with 0.3M NaOH and 1% sodium dodecylsulfate (SDS) , and then each protein fraction was precipitated with trichloroacetic acid (TCA). After the precipitate was filtered through a glass filter (GF/B) and washed, the quantity of [³H] proline on the glass filter was measured by a liquid scintillation counter. The number of cells was measured by a cell growth measuring kit (XTT, Roche) and the uptake of [³H] proline was corrected by the number of cells. Inhibitory ratio was calculated as compared with the case where a difference between the uptake of [³H] proline with or without TGF-β1.

The results of NHDF are shown in Table 1, and the results of TIG-3 are shown in Table 2.

**Table 1:**

| Inhibitory effect on collagen production induced by TGF-β (NHDF cells) | | | |
|---|---|---|---|
| Inhibitory effect on collagen production induced Test drugs by TGF-β (% inhibition) | | | |
| | 1 µM | 3 µM | 10 µM |
| Compound A | 8 | 9 | 30 |
| Example 2 | 23 | 61 | 79 |
| Example 30 | 41 | 94 | 148 |
| Example 121 | - | 39 | 54 |
| Example 31 | - | - | 74 |
| Example 5 | 40 | 43 | 88 |
| Example 27 | 25 | 35 | 42 |
| Example 97 | 37 | 69 | - |
| Example 98 | 47 | 92 | - |
| Example 3 | 65 | 105 | 143 |
| Example 28 | 31 | 40 | 92 |
| Example 4 | 41 | 70 | 138 |
| Example 29 | 64 | 72 | 123 |
| Example 109 | 50 | 76 | 135 |
| Example 110 | - | 66 | 143 |
| Example 111 | - | 82 | 168 |
| Example 112 | - | 73 | 143 |
| Example 113 | - | 88 | 148 |
| Example 114 | - | 94 | 165 |
| Example 115 | - | 84 | 155 |
| Example 116 | - | 76 | 168 |
| Example 117 | - | 82 | 140 |
| Example 118 | - | 81 | 147 |
| Example 119 | - | 75 | 153 |
| Example 122 | 20 | 57 | - |
| Example 123 | 55 | 125 | - |
| Example 124 | 21 | 108 | - |
| Example 125 | 52 | 91 | - |
| Example 126 | 29 | 86 | - |
| Example 127 | 72 | 78 | - |
| Example 128 | 61 | 76 | - |
| Example 129 | 75 | 73 | - |
| Example 130 | 92 | 157 | - |
| Example 131 | 96 | 141 | - |
| Example 132 | 67 | 122 | - |
| Example 133 | 57 | 104 | - |
| Example 134 | 90 | 111 | - |
| Example 135 | 78 | 110 | - |
| Example 136 | 66 | 111 | - |
| Example 137 | 38 | 72 | - |
| Example 138 | 65 | 53 | - |
| Example 139 | 37 | 54 | - |
| Example 140 | 140 | 171 | - |
| Example 141 | 111 | 114 | - |
| Example 142 | 65 | 130 | - |
| Example 143 | 42 | 104 | - |
| Example 144 | 78 | 85 | - |
| Example 145 | 55 | 64 | - |
| Example 146 | 43 | 60 | - |
| Example 147 | 29 | 59 | - |
| Example 148 | 29 | 53 | - |

**Table 2:**

| Inhibitory effect on collagen production induced by TGF-β (TIG-3 cells) | | |
|---|---|---|
| Test drugs | Inhibitory effect on collagen production induced by TGF-β (% inhibition) | |
| | 3 µM | 10 µM |
| Compound A | 28 | 63 |
| Example 2 | 74 | 117 |
| Example 9 | 42 | 71 |
| Example 31 | 36 | 90 |
| Example 1 | 21 | 56 |
| Example 76 | 78 | 101 |
| Example 80 | 105 | 149 |
| Example 15 | 82 | 140 |
| Example 32 | 62 | 117 |
| Example 60 | 70 | 133 |

As is apparent from the results shown in Table 1 and Table 2, the compound of the present invention has an excellent inhivitory action against TGF-β.

### Test Example 2

### Measurement of concentration of compound in rat plasma

Each of the test compounds was dissolved or suspended in methyl cellulose and the resulting solution or suspension was orally administered to rats at a dosage of 10 mg/kg and blood was collected after 0.25 hours, 1 hour and 4 hours. The concentration in plasma in each of the compounds was measured by HPLC.

The results are shown in Table 3.

**Table 3:**

| Concentration of compound in rat plasma | | | |
|---|---|---|---|
| Test drugs | Concentration (ng/ml) | | |
| | After 0.25 hours | After 1 hour | After 4 hours |
| Compound A | 0 to 69 | 55 to 120 | 5 to 18 |
| Example 121 | 3900 to 5800 | 2100 to 5800 | 310 to 580 |
| Example 26 | 230 to 800 | 130 to 300 | 0 to 92 |
| Example 9 | 1300 to 3900 | 720 to 2200 | 350 to 530 |
| Example 120 | 6400 to 10000 | 3200 to 5700 | 2500 to 3100 |
| Example 31 | 320 to 370 | 320 to 640 | 420 to 650 |
| Example 1 | 2300 to 29000 | 1300 to 7300 | 750 to 940 |
| Example 5 | 1400 to 2300 | 520 to 720 | 87 to 160 |
| Example 27 | 2400 to 4100 | 1500 to 1900 | N.D. |
| Example 3 | 120 to 660 | 370 to 640 | 260 to 310 |
| Example 28 | 71 to 170 | 400 to 550 | 320 to 460 |
| Example 4 | 390 to 500 | 490 to 590 | 180 to 290 |
| Example 104 | 14 to 19 | 44 to 71 | 25 to 32 |
| Example 29 | 330 to 940 | 280 to 1100 | 170 to 710 |

| | | | |
|---|---|---|---|
| N.D.: not determined | | | |

As is apparent from the results shown in Table 3, the concentration in the compound of the present invention in blood is by far higher than the concentration of the compound A in blood.

### Test Example 3

### Acute toxicity

A suspension of the test drug was orally administered to mice at a dosage of 1000 mg/kg, and their general condition was observed for a week after administration. Each of the compounds in Examples 1, 9, 31, 120, and 121 was administered.

As a result, no deaths were observed in any of the administration groups.

### Formulation Example 1

**Tablet (oral tablet)**

| Formulation/tablet (in 80 mg) | |
|---|---|
| Compound of Example 3 | 5.0 mg |
| Corn Starch | 46.6 mg |
| Crystalline cellulose | 24.0 mg |
| Methyl cellulose | 4.0 mg |
| Magnesium stearate | 0.4 mg |

The mixed powder of this composition is compressed and molded to make oral tablets.

### Formulation Example 2

**Tablet (oral tablet)**

| Formulation/tablet (in 80 mg) | |
|---|---|
| Compound of Example 5 | 5.0 mg |
| Corn Starch | 46.6 mg |
| Crystalline cellulose | 24.0 mg |
| Methyl cellulose | 4.0 mg |
| Magnesium stearate | 0.4 mg |

The mixed powder of this composition is compressed and molded to make oral tablets.

### INDUSTRIAL APPLICABILITY

As described above, since the compound of the present invention has an excellent inhivitory action against TGF-β and is a safe compound with low toxicity, a pharmaceutical composition containing the compound of the invention as an active ingredient is useful as an inhibitor of TGF-β in mammals, including humans.

## Claims

1. An amide derivative, which is a compound represented by the following formula [1] in any of the following cases (A), (B) and (C), or a pharmaceutically acceptable salt thereof. 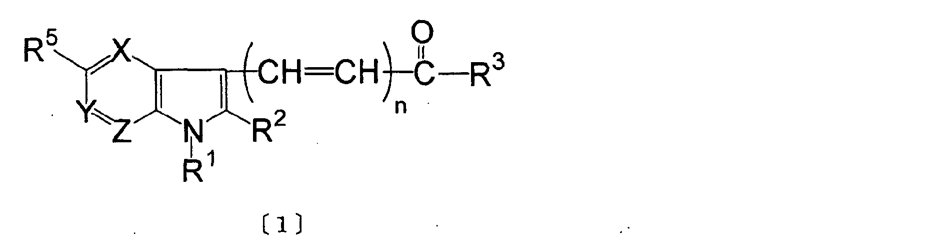
(A)
n represents 0,
X represents CR⁴ or N, Y represents CR⁶ or N, Z represents CR⁷ or N,
R¹ and R² may be the same or different and each represents hydrogen, alkyl, haloalkyl, hydroxyalkyl, alkoxyalkyl, carboxyalkyl, acyl, aryl, an aromatic heterocyclic group or arylalkyl ( the aryl, the aromatic heterocyclic group and the aryl moiety of the arylalkyl may be substituted by R⁸¹, R⁸² or R⁸³, and R⁸¹, R⁸² and R⁸³ may be the same or different and each represents halogen, alkyl, haloalkyl, alkoxy, haloalkoxy, hydroxy, hydroxyalkyl, carboxy, alkoxycarbonyl, cyano or nitro, or the two adjacent groups among R⁸¹, R⁸² and R⁸³ may jointly form methylenedioxy or ethylenedioxy ),
R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano or nitro, or the two adjacent groups among R⁴, R⁵, R⁶ and R⁷ may jointly form methylenedioxy or ethylenedioxy,
R³ represents a group represented by the following formula [2]: 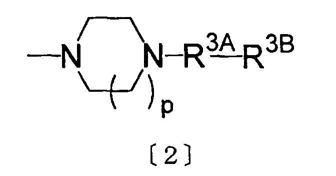 in which p represents 1 or 2,
R^{3A} represents a divalent group of pyridine, pyrazine, pyridazine or pyrimidine,
R^{3B} represents tetrazolyl [ the tetrazolyl may be substituted by alkyl ( the alkyl may be substituted by hydroxy, dialkylamino or cycloalkyl ] or a group represented by the following formula [3], 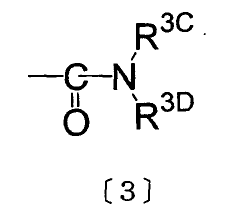
R^{3C} and R^{3D} may be the same or different and each represents any of the following groups (1) to (4):
(1) hydrogen,
(2) cycloalkyl ( the cycloalkyl may be substituted by alkyl, hydroxy, hydroxyalkyl, carboxy, alkoxycarbonyl or aryl ),
(3) alkyl [ the alkyl may be substituted by 1 to 5 same or different members selected from the group consisting of (i) a saturated heterocyclic group ( the saturated heterocyclic group may be substituted by alkyl, hydroxyalkyl or alkoxyalkyl ) , (ii) an aromatic heterocyclic group ( the aromatic heterocyclic group may be substituted by 1 to 3 same or different members selected from the group consisting of alkyl, hydroxy, amino, hydroxyalkyl and alkoxyalkyl ), (iii) hydroxy, (iv) alkoxy, (v) aryl ( the aryl may be substituted by alkyl, hydroxy, alkoxy or alkoxyalkyl ), (vi) aryloxy ( the aryl moiety of the aryloxy may be substituted by alkyl, hydroxy, alkoxy or alkoxyalkyl ), (vii) alkylamino, (viii) dialkylamino, (ix) carboxy, (x) alkoxycarbonyl and (xi) halogen ], and
(4) an aromatic heterocyclic group ( the aromatic heterocyclic group may be substituted by alkyl, hydroxyalkyl, alkoxyalkyl, alkoxycarbonylalkyl or aryl ), or
R^{3C}, R^{3D} and adjacent N jointly form cyclic amino ( the cyclic amino may be substituted by alkyl, hydroxyalkyl, alkoxyalkyl, aminoalkyl, hydroxy, aryl or arylalkyl ),
with the exception of the case that R^{3C} is hydrogen and R^{3D} is hydrogen or non-substituted alkyl;
(B)
n represents 0,
X represents CR⁴ or N, Y represents CR⁶ or N, Z represents CR⁷ or N,
R¹ and R² may be the same or different and each represents alkyl, haloalkyl, hydroxyalkyl, alkoxyalkyl, carboxyalkyl, acyl, aryl, an aromatic heterocyclic group or arylalkyl ( the aryl, the aromatic heterocyclic group and the aryl moiety of the arylalkyl may be substituted by R⁸¹, R⁸² or R⁸³, and R⁸¹, R⁸² and R⁸³ may be the same or different and each represents halogen, alkyl, haloalkyl, alkoxy, haloalkoxy, hydroxy, hydroxyalkyl, carboxy, alkoxycarbonyl, cyano or nitro, or the two adjacent groups among R⁸¹, R⁸² and R⁸³ may jointly form methylenedioxy or ethylenedioxy ),
R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano or nitro, or the two adjacent groups among R⁴, R⁵, R⁶ and R⁷ may jointly form methylenedioxy or ethylenedioxy,
R³ represents a group represented by the following formula [4]: 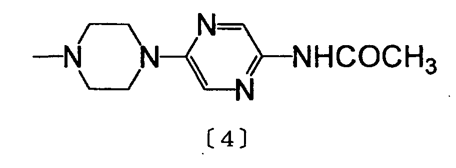
(C)
n represents 1,
X represents CR⁴ or N, Y represents CR⁶ or N, Z represents CR⁷ or N,
R¹ and R² may be the same or different and each represents hydrogen, alkyl, haloalkyl, hydroxyalkyl, alkoxyalkyl, carboxyalkyl, acyl, aryl, an aromatic heterocyclic group or arylalkyl ( the aryl, the aromatic heterocyclic group and the aryl moiety of the arylalkyl may be substituted with R⁸¹, R⁸² or R⁸³, and R⁸¹, R⁸² and R⁸³ may be the same or different and each represents halogen, alkyl, haloalkyl, alkoxy, haloalkoxy, hydroxy, hydroxyalkyl, cyano, alkoxycarbonyl, carboxy or nitro, or the two adjacent groups among R⁸¹, R⁸² and R⁸³ may jointly form methylenedioxy or ethylenedioxy ),
R⁴, R⁵, R⁶ and R⁷ may be the same or different and each represents hydrogen, halogen, hydroxy, amino, alkyl, haloalkyl, alkoxy, monoalkylamino, dialkylamino, arylalkyl, cyano or nitro, or the two adjacent groups among R⁴, R⁵, R⁶ and R⁷ may jointly form methylenedioxy or ethylenedioxy,
R³ represents the following group (1) or (2):
(1) cyclic amino [ the cyclic amino may be substituted by R⁹¹, R⁹² or R⁹³ on the same or different carbon atom and the benzene ring may be condensed, R⁹¹, R⁹² and R⁹³ may be the same or different and each represents alkyl, alkoxy, hydroxy, hydroxyalkyl, alkoxyalkyl, alkoxycarbonylalkyl, carboxy, carboxyalkyl, acyl, 2-hydroxybenzimidazole, aryl ( the aryl may be substituted by alkyl or alkoxy ) or an aromatic heterocyclic group ( the aromatic heterocyclic group may be substituted by alkyl, hydroxy, oxo or alkoxy ), or the two groups on the same carbon jointly represent -CONHCH₂N(Ph)- ( Ph represents phenyl ), the benzene ring condensed with the cyclic amino may be substituted with R⁹⁴ or R⁹⁵, and R⁹⁴ and R⁹⁵ may be the same or different and each represents alkyl, alkoxy, halogen, hydroxy, amino, monoalkylamino, dialkylamino, alkylsulfonylamino, acylamino, carbamoyl, monoalkylcarbamoyl, dialkylcarbamoyl, cyano, carboxy, alkoxycarbonyl, sulfamoyl, monoalkylaminosulfonyl, dialkylaminosulfonyl or nitro, or the two adjacent groups among R⁹⁴ and R⁹⁵ may jointly form methylenedioxy or ethylenedioxy ],
(2) a group represented by the following formula [5]: 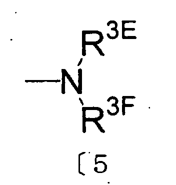
R^{3E} and R^{3F} may be the same or different and each represents any of the following groups (i) to (v):
(i) hydrogen,
(ii) alkyl { the alkyl may be substituted by 1 to 3 same or different members selected from the group consisting of (a) hydroxy, (b) aryl ( the aryl may be substituted with 1 to 3 same or different members selected from the group consisting of alkyl, haloalkyl, hydroxy, alkoxy, halogen, cyano and nitro ), (c) carboxy, (d) a saturated heterocyclic group [ the saturated heterocyclic group may be substituted by alkyl or arylalkyl ( the aryl moiety of the arylalkyl may be substituted with alkyl, alkoxy or halogen )], and (e) an aromatic heterocyclic group ( the aromatic heterocyclic group may be substituted by alkyl, haloalkyl, hydroxy, hydroxyalkyl, alkoxy, halogen, cyano or nitro )},
(iii) aryl (the aryl may be substituted by alkyl, haloalkyl, hydroxy, hydroxyalkyl, alkoxy, halogen, cyano or nitro ),
(iv) cycloalkyl ( the cycloalkyl may be substituted by alkoxy, alkoxycarbonyl or hydroxy and the benzene ring may be condensed ), and
(v) a saturated heterocyclic group ( the saturated heterocyclic group may be substituted by alkyl, haloalkyl, hydroxy, hydroxyalkyl, alkoxy, halogen, cyano or nitro ).

2. The amide derivative or pharmaceutically acceptable salt thereof according to claim 1, wherein R¹ and R² may be the same or different and each represents hydrogen, alkyl, alkoxyalkyl, aryl or an aromatic heterocyclic group ( the aryl and the aromatic heterocyclic group may be substituted by R⁸¹, R⁸² or R⁸³, and R⁸¹, R⁸² and R⁸³ may be the same or different and each represents halogen, alkyl or alkoxy ), R⁴, R⁵, R⁶ and R⁷ may be the same or different and each represents hydrogen, halogen or alkyl, or the two adjacent groups amongR⁴, R⁵, R⁶ and R⁷ may jointly form methylenedioxy in case (A).

3. The amide derivative or pharmaceutically acceptable salt thereof according to claim 1, wherein R¹ and R² may be the same or different and each represents hydrogen, alkyl, alkoxyalkyl, aryl or an aromatic heterocyclic group ( the aryl and the aromatic heterocyclic group may be substituted by R⁸¹, R⁸² or R⁸³, and R⁸¹, R⁸² and R⁸³ may be the same or different and each represents halogen, alkyl, alkoxy, alkoxycarbonyl or carboxy, or the two adjacent groups among R⁸¹, R⁸² and R⁸³ may jointly form methylenedioxy ), and R⁴, R⁵, R⁶ and R⁷ may be the same or different and each represents hydrogen, halogen, alkyl or alkoxy, or the two adjacent groups among R⁴, R⁵, R⁶ and R⁷ may jointly form methylenedioxy in case (C).

4. The amide derivative or pharmaceutically acceptable salt thereof according to claim 1 or 2, wherein R³ is a group represented by the following formula [2]: 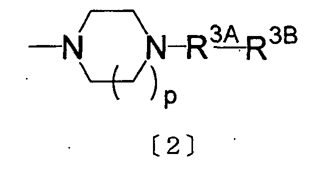 in which p represents 1,
R^{3A} represents a divalent group of pyridine or pyrazine,
R^{3B} represents tetrazolyl [ the tetrazolyl may be substituted by alkyl ( the alkyl may be substituted by hydroxy, dialkylamino or cycloalkyl )] or a group represented by the following formula [3] 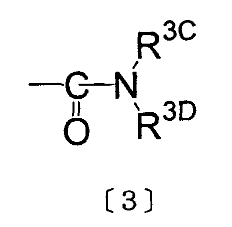 in which R^{3C} and R^{3D} may be the same or different and each represents any of the following groups (1) to (4):
(1) hydrogen,
(2) cycloalkyl ( the cycloalkyl may be substituted by hydroxy or alkoxycarbonyl ),
(3) alkyl [ the alkyl may be substituted by 1 to 5 same or different members selected from the group consisting of (i) a saturated heterocyclic group ( the saturated heterocyclic group may be substituted by alkyl), (ii) an aromatic heterocyclic group ( the aromatic heterocyclic group may be substituted by 1 to 3 same or different members selected from the group consisting of alkyl and amino), (iii) hydroxy, (iv) alkoxy, (v) aryl ( the aryl may be substituted by alkyl, hydroxy or alkoxy), (vi) aryloxy, (vii) dialkylamino, (viii) carboxy, (ix) alkoxycarbonyl and (x) halogen ], and
(4) an aromatic heterocyclic group ( the aromatic heterocyclic group may be substituted by alkoxycarbonylalkyl ),
or R^{3C}, R^{3D} and adjacent N jointly form cyclic amino ( the cyclic amino may be substituted by hydroxyalkyl, aminoalkyl or hydroxy ) in case (A).

5. The amide derivative or pharmaceutically acceptable salt thereof according to claim 1 or 3, wherein R³ is the following group (1) or (2):
(1) cyclic amino [ the cyclic amino may be substituted by R⁹¹, R⁹² or R⁹³ on the same or different carbon atom and the benzene ring may be condensed, R⁹¹, R⁹² and R⁹³ may be the same or different and each represents alkyl, hydroxy, hydroxyalkyl, alkoxycarbonylalkyl, carboxyalkyl, acyl, 2-hydroxybenzimidazole, aryl ( the aryl may be substituted by alkoxy ) or an aromatic heterocyclic group, or the two groups on the same carbon jointly represent -CONHCH₂N(Ph)- ( Ph represents phenyl ), the benzene ring condensed with the cyclic amino may be substituted by R⁹⁴ or R⁹⁵, and R⁹⁴ and R⁹⁵ may be the same or different and each represents alkoxy, halogen, hydroxy, amino, dialkylamino, carbamoyl, cyano, dialkylaminosulfonyl or nitro, or the two adjacent groups among R⁹⁴ and R⁹⁵ may jointly form methylenedioxy ],
(2) a group represented by the following formula [5]: 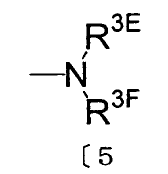 in which R^{3E} and R^{3F} may be the same or different and each represents any of the following groups (i) to (v):
(i) hydrogen,
(ii) alkyl { the alkyl may be substituted by 1 to 3 same or different members selected from the group consisting of (a) hydroxy, (b) aryl ( the aryl may be substituted by 1 to 3 same or different members selected from the group consisting of alkyl, haloalkyl, hydroxy, alkoxy and halogen), (c) carboxy, (d) a saturated heterocyclic group [ the saturated heterocyclic group may be substituted with arylalkyl ( the aryl moiety of the arylalkyl may be substituted by halogen )] and (e) an aromatic heterocyclic group ( the aromatic heterocyclic group may be substituted by alkyl, haloalkyl, hydroxy, hydroxyalkyl, alkoxy or halogen )},
(iii) aryl ( the aryl may be substituted by alkyl, haloalkyl, hydroxy, hydroxyalkyl, alkoxy or halogen ),
(iv) cycloalkyl (the cycloalkyl may be substituted by alkoxy, alkoxycarbonyl or hydroxy and the benzene ring may be condensed), and
(v) a saturated heterocyclic group ( the saturated heterocyclic group may be substituted by alkyl, haloalkyl, hydroxy, hydroxyalkyl, alkoxy or halogen ) in case (C).

6. The amide derivative or pharmaceutically acceptable salt thereof according to claim 1, 2 or 4, wherein X is CR⁴, Y is CR⁶, Z is CR⁷, R¹ and R² may be the same or different and each represents alkyl or aryl, and R⁴, R⁵, R⁶ and R⁷ may be the same or different and each represents hydrogen, halogen or alkyl in case (A).

7. The amide derivative or pharmaceutically acceptable salt thereof according to claim 1, 3 or 5, wherein R¹ and R² may be the same or different and each represents hydrogen, alkyl, alkoxyalkyl, aryl or an aromatic heterocyclic group ( the aryl and the aromatic heterocyclic group may be substituted by R⁸¹, R⁸² or R⁸³, R⁸¹, R⁸² and R⁸³ may be the same or different and each represents halogen, alkoxy, alkoxycarbonyl or carboxy, or the two adjacent groups among R⁸¹, R⁸² and R⁸³ may jointly form methylenedioxy ) , and R⁴, R⁵, R⁶ and R⁷ may be the same or different and each represents hydrogen or alkyl in case (C)..

8. The amide derivative or pharmaceutically acceptable salt thereof according to claim 1, 2, 4 or 6, wherein R³ is a group represented by the following formula [2]: 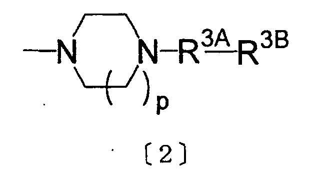 in which p represents 1,
R^{3A} represents a divalent group of pyrazine, and
R^{3B} represents a group represented by the following formula [3]: 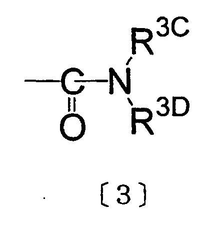
in which R^{3C} and R^{3D} may be the same or different and each represents any of the following groups (1) to (3):
(1) hydrogen,
(2) cycloalkyl ( the cycloalkyl may be substituted by alkoxycarbonyl ), and
(3) alkyl [ the alkyl may be substitiuted by 1 to 5 same or different members selected from the group consisting of (i) an aromatic heterocyclic group ( the aromatic heterocyclic group may be substituted by 1 to 3 same or different alkyl), (ii) aryl ( the aryl may be substituted by hydroxy ), (iii) carboxy and (iv) alkoxycarbonyl ] in case (A).

9. The amide derivative or pharmaceutically acceptable salt thereof according to claim 1, 3, 5 or 7, wherein R³ is the following group (1) or (2):
(1) cyclic amino [ the cyclic amino may be substituted by R⁹¹, R⁹² or R⁹³ on the same or different carbon atom and the benzene ring may be condensed, R⁹¹, R⁹² and R⁹³ may be the same or different and each represents alkoxycarbonylalkyl, carboxyalkyl, acyl or aryl, the benzene ring condensed with the cyclic amino may be substituted by R⁹⁴ or R⁹⁵, and R⁹⁴ and R⁹⁵ may be the same or different and each represents alkoxy ],
(2) a group represented by the following formula [5]: 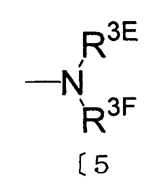
in which R^{3E} and R^{3F} may be the same or different and each represents any of the following groups (i) to (iv):
(i) hydrogen,
(ii) alkyl ( the alkyl may be substituted with 1 to 3 same or different members selected from the group consisting of carboxy and aromatic heterocyclic group ),
(iii) cycloalkyl ( the cycloalkyl may be substituted by alkoxycarbonyl ), and
(iv) a saturated heterocyclic group ( the saturated heterocyclic group may be substituted with alkyl ) in case (C).

10. The amide derivative or pharmaceutically acceptable salt thereof according to claim 1, 2, 4, 6 or 8, wherein X is CR⁴, Y is CR⁶, Z is CR⁷, R¹ and R² may be the same or different and each represents alkyl or aryl, R⁴, R⁵, R⁶ and R⁷ may be the same or different and each represents hydrogen, halogen or alkyl, and R³ is a group represented by the following formula [2]: 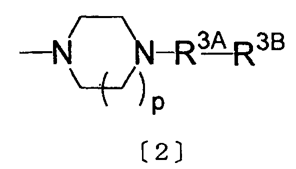 in which p represents 1,
R^{3A} represents a divalent group of pyrazine,
R^{3B} represents a group represented by the following formula [3]: 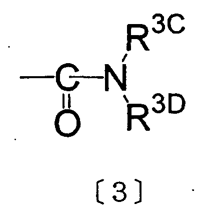
in which R^{3C} and R^{3D} may be the same or different and each represents any of the following groups (1) to (3):
(1) hydrogen,
(2) cycloalkyl ( the cycloalkyl may be substituted by alkoxycarbonyl ), and
(3) alkyl [ the alkyl may be substituted by 1 to 5 same or different members selected from the group consisting of (i) an aromatic heterocyclic group ( the aromatic heterocyclic group may be substituted by 1 to 3 same or different alkyl ) , (ii) aryl ( the aryl may be substituted by hydroxy ), (iii) carboxy and (iv) alkoxycarbonyl ] in case (A).

11. The amide derivative or pharmaceutically acceptable salt thereof according to claim 1, 3, 5, 7 or 9, wherein R¹ and R² may be the same or different and each represents hydrogen, alkyl, alkoxyalkyl, aryl or an aromatic heterocyclic group ( the aryl and the aromatic heterocyclic group may be substituted by R⁸¹, R⁸² or R⁸³, R⁸¹, R⁸² and R⁸³ may be the same or different and each represents halogen, alkoxy, alkoxycarbonyl or carboxy, or the two adjacent groups among R⁸¹, R⁸² and R⁸³ may jointly form methylenedioxy ), R⁴, R⁵, R⁶ and R⁷ may be the same or different and each represents hydrogen or alkyl, and R³ is the following group (1) or (2):
(1) cyclic amino [ the cyclic amino may be substituted by R⁹¹, R⁹² or R⁹³ on the same or different carbon atom and the benzene ring may be condensed, R⁹¹, R⁹² and R⁹³ may be the same or different and each represents alkoxycarbonylalkyl, carboxyalkyl, acyl or aryl, the benzene ring condensed with the cyclic amino may be substituted by R⁹⁴ or R⁹⁵, and R⁹⁴ and R⁹⁵ may be the same or different and each represents alkoxy ],
(2) a group represented by the following formula [5]: 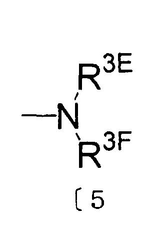
in which R^{3E} and R^{3F} may be the same or different and each represents any of the following groups (i) to (iv):
(i) hydrogen,
(ii) alkyl ( the alkyl may be substituted by 1 to 3 same or different members selected from the group consisting of carboxy and an aromatic heterocyclic group ),
(iii) cycloalkyl ( the cycloalkyl may be substituted by alkoxycarbonyl ), and
(iv) a saturated heterocyclic group ( the saturated heterocyclic group may be substituted by alkyl ) in case (C).

12. The amide derivative or pharmaceutically acceptable salt thereof according to any one of claims 1 to 11, which is a compound selected from the group consisting of the following compounds (1) to (36):
(1) 5-{4-[(1,5-dimethyl-2-phenyl-1H-indol-3-yl)carbonyl]piperazin-1-yl}-N-[2-(4-methyl-1,3-thiazol-5-yl)ethyl]pyrazine-2-carboxamide,
(2) 2-[(2E)-3-(1-methyl-2-phenyl-1H-pyrrolo[2,3-b]pyridin-3-yl)prop-2-enoyl]-6,7-dimethoxy-1,2,3,4-tetrahydroisoquinoline,
(3) (2E)-N-methyl-N-(1-methyl-piperidin-4-yl)-3-(1-methyl-2-phenyl-1H-pyrrolo[2,3-b]pyridin-3-yl)acrylamide,
(4) 2-[(2E)-3-(1,5-dimethyl-2-phenyl-1H-pyrrolo[2,3-b]pyridin-3-yl)prop-2-enoyl]-6,7-dimethoxy-1,2,3,4-tetrahydroisoquinoline,
(5) {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetic acid,
(6) ethyl N-benzyl-N-[(5-{4-[(1,5-dimethyl-2-phenyl-1H-indol-3-yl)carbonyl]piperazin-1-yl}pyrazin-2-yl)carbonyl]aminoacetate,
(7) ethyl 2-{N-[(2E)-3-(1-methyl-2-phenyl-1H-pyrrolo[2,3-b]pyridin-3-yl)prop-2-enoyl]amino}-1-cyclopentanecarboxylate,
(8) ethyl 2-{N-[(5-{4-[(1,5-dimethyl-2-phenyl-1H-indol-3-yl)carbonyl]piperazin-1-yl}pyrazin-2-yl)carbonyl]amino}-1-cyclohexanecarboxylate,
(9) 2-[(2E)-3-(5-methyl-6-phenyl-5H-pyrrolo[2,3-b]pyrazin-7-yl)prop-2-enoyl]-6,7-dimethoxy-1,2,3,4-tetrahydroisoquinoline,
(10) ethyl 2-{N-[(5-{4-[(1,5-dimethyl-2-phenyl-1H-indol-3-yl)carbonyl]piperazin-1-yl}pyrazin-2-yl)carbonyl]amino}-1-cyclopentanecarboxylate,
(11) 2-[(2E)-3-(1-methyl-2-phenyl-1H-pyrrolo[2,3-c]pyridin-3-yl)prop-2-enoyl]-6,7-dimethoxy-1,2,3,4-tetrahydroisoquinoline,
(12) 2-{(2E)-3-[1-methyl-2-(3,4-dimethoxyphenyl)-1H-pyrrolo[2,3-b]pyridin-3-yl]prop-2-enoyl}-6,7-dimethoxy-1,2,3,4-tetrahydroisoquinoline,
(13) N-methyl-N-[(5-{4-[(1,5-dimethyl-2-phenyl-1H-indol-3-yl)carbonyl]piperazin-1-yl}pyrazin-2-yl)carbonyl]-(S)-phenylalanine,
(14) methyl {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetate,
(15) {6,7-dimethoxy-2-[(2E)-3-(1-methyl-2-phenyl-1H-pyrrolo[2,3-b]pyridin-3-yl)prop-2-enoyl]-1,2,3,4-tetrahydroisoquinolin-1-yl}acetic acid,
(16) 2-{(2E)-3-[1-methyl-2-(4-pyridyl)-1H-pyrrolo[2,3-b]pyridin-3-yl]prop-2-enoyl}-6,7-dimethoxy-1,2,3,4-tetrahydroisoquinoline,
(17) 2-[(2E)-3-(1-methyl-2-phenyl-1H-pyrrolo[3,2-b]pyridin-3-yl)prop-2-enoyl]-6,7-dimethoxy-1,2,3,4-tetrahydroisoquinoline,
(18) N-[(2E)-3-(1-methyl-2-phenyl-1H-pyrrolo[2,3-b]pyridin-3-yl)prop-2-enoyl]-N-[2-(2-thienyl)ethyl]aminoacetic acid,
(19) 6,7-dimethoxy-2-[(2E)-3-(2-phenyl-1H-pyrrolo[2,3-b]pyridin-3-yl)prop-2-enoyl]-1,2,3,4-tetrahydroisoquinoline,
(20) 2-[(2E)-3-(1-methoxymethyl-2-phenyl-1H-pyrrolo[2,3-b]pyridin-3-yl)prop-2-enoyl]-6,7-dimethoxy-1,2,3,4-tetrahydroisoquinoline,
(21) 2-{(2E)-3-[1-methyl-2-(4-fluorophenyl)-1H-pyrrolo[2,3-b]pyridin-3-yl]prop-2-enoyl}-6,7-dimethoxy-1,2,3,4-tetrahydroisoquinoline,
(22) 4-acetyl-1-{(2E)-3-[1,5-dimethyl-2-(4-carbomethoxyphenyl)-1H-indol-3-yl]prop-2-enoyl}-4-phenylpiperidine,
(23) 2-{(2E)-3-[1,5-dimethyl-2-(4-carbomethoxyphenyl)-1H-indol-3-yl]prop-2-enoyl}-6,7-dimethoxy-1,2,3,4-tetrahydroisoquinoline,
(24) (2E)-N-methyl-N-(1-methylpiperidin-4-yl)-3-[1,5-dimethyl-2-(4-carbomethoxyphenyl)-1H-indol-3-yl]acrylamide,
(25)5-{4-[(5-bromo-1-methyl-2-phenyl-1H-indol-3-yl)carbonyl]piperazin-1-yl}-N-[2-(4-methyl-1,3-thiazol-5-yl)ethyl]pyrazine-2-carboxamide,
(26) 5-{4-[(1,5-dimethyl-2-phenyl-1H-indol-3-yl)carbonyl]piperazin-1-yl}-N-[2-(4-hydroxyphenyl)ethyl]pyrazine-2-carboxamide,
(27) 4-{3-[3-(4-acetyl-4-phenylpiperidin-1-yl)-3-oxo-1-propenyl]-1,5-dimethyl-1H-indol-2-yl}benzoic acid,
(28) 5-{4-[(5-fluoro-1-methyl-2-phenyl-1H-indol-3-yl)carbonyl]piperazin-1-yl}-N-[2-(4-methyl-1,3-thiazol-5-yl)ethyl]pyrazine-2-carboxamide,
(29) 5-{4-[(1,5-dimethyl-2-phenyl-1H-pyrrolo[2,3-b]pyridin-3-yl)carbonyl]piperazin-1-yl}-N-[2-(4-methyl-1,3-thiazol-5-yl)ethyl]pyrazine-2-carboxamide,
(30) 2-{(2E)-3-[1-methyl-2-(3,4-methylenedioxyphenyl)-1H-pyrrolo[2,3-b]pyridin-3-yl]prop-2-enoyl}-6,7-dimethoxy-1,2,3,4-tetrahydroisoquinoline hydrochloride,
(31) 5-{4-[(5-chloro-1-methyl-2-phenyl-1H-indol-3-yl)carbonyl]piperazin-1-yl}-N-[2-(4-methyl-1,3-thiazol-5-yl)ethyl]pyrazine-2-carboxamide dihydrochloride,
(32) N-{[5-{4-[(1,5-dimethyl-2-phenyl-1H-indol-3-yl)carbonyl]piperazin-1-yl}pyrazin-2-yl]carbonyl}-(S)-phenylalanine
(33) N-methyl-N-{[5-{4-[(1,5-dimethyl-2-phenyl-1H-indol-3-yl)carbonyl]piperazin-1-yl}pyrazin-2-yl]carbonyl}-(S)-phenylalaninemethyl ester,
(34) 4-{3-[3-(6,7-dimethoxy-1,2,3,4-tetrahydroisoquinolin-2-yl)-3-oxo-propenyl]-1-methyl-1H-pyrrolo[2,3-b]pyridin-2-yl}benzoic acid hydrochloride,
(35) methyl [6,7-dimethoxy-2-{(2E)-3-[1-methyl-2-(4-fluorophenyl)-1H-pyrrolo[2,3-b]pyridin-3-yl]prop-2-enoyl}-1,2,3,4-tetrahydroisoquinolin-1-yl]acetate hydrochloride, and
(36) [6,7-dimethoxy-2-{(2E)-3-[1-methyl-2-(4-fluorophenyl)-1H-pyrrolo[2,3-b]pyridin-3-yl]prop-2-enoyl}-1,2,3,4-tetrahydroisoquinolin-1-yl]acetic acid.

13. A pharmaceutical composition comprising the amide derivative or pharmacologically acceptable salt thereof of any one of claims 1 to 12 as an active ingredient.

14. A TGF-β inhibitor comprising the amide derivative or pharmacologically acceptable salt thereof of any one of claims 1 to 12 as an active ingredient.

15. A therapeutic agent for pulmonary fibrosis, scleroderma, pachyderma, nephritis, comprising the amide derivative or pharmacologically acceptable salt thereof of any one of claims 1 to 12 as an active ingredient.
